# EUROPEAN PATENT SPECIFICATION

(11) **EP 2 168 599 B1**
(45) Date of publication and mention of the grant of the patent: **10.08.2016**
(21) Application number: 08777680.3
(22) Date of filing: 27.06.2008
(51) Int. Cl.: A61K 39/395, A61P 35/00, A61P 35/04, G01N 33/574

(54) **CANCER REMEDY CONTAINING ANTIBODY AGAINST PEPTIDE ENCODED BY EXON-17 OF PERIOSTIN**
KREBSHEILMITTEL MIT EINEM ANTIKÖRPER GEGEN EIN VOM EXON-17-BEREICH VON PERIOSTIN KODIERTES PEPTID
REMÈDE ANTICANCÉREUX CONTENANT UN ANTICORPS DIRIGÉ CONTRE UN PEPTIDE CODÉ PAR L'EXON-17 DE LA PÉRIOSTINE

(30) Priority: 27.06.2007 JP 2007169494; 14.02.2008 JP 2008033827
(43) Date of publication of application: 31.03.2010
(73) Proprietor: Osaka University, Suita-shi Osaka 565-0871 (JP)
(72) Inventor: TANIYAMA, Yoshiaki, Toyonaka-shi Osaka 560-0012 (JP); MORISHITA, Ryuichi, Suita-shi Osaka 565-0851 (JP); KATSURAGI, Naruto, Ibaraki-shi Osaka 567-0021 (JP)
(74) Representative: Hoffmann Eitle
(86) International application number: PCT/JP2008/061768
(87) International publication number: WO 2009/001940

(56) References cited:
- EP-A1- 1 978 034
- WO-A1-02/20055
- WO-A1-2007/077934
- WO-A2-03/016471
- WO-A2-2005/019471
- JP-A- 2005 500 059
- KIM C-J ET AL: "Analysis of Correlation Between Alternative Splicing Variants of Periostatin Gene and Malignant Phenotypes", ANNUAL MEETING OF THE JAPAN CANCER ASSOCIATION KIJI, JAPAN CANCER ASSOCIATION, JAPAN, vol. 65, no. Suppl, 1 January 2006 (2006-01-01), XP008159057, ISSN: 0546-0476
- LITVIN J ET AL: "Periostin family of proteins: therapeutic targets for heart disease", THE ANATOMICAL RECORD PART A: DISCOVERIES IN MOLECULAR, CELLULAR, AND EVOLUTIONARY BIOLOGY, WILEY INTERSCIENCE, vol. 287, no. 2, 1 December 2005 (2005-12-01), pages 1205-1212, XP002573726, ISSN: 1552-4884, DOI: 10.1002/AR.A.20237 [retrieved on 2005-10-20]
- TAKESHITA S ET AL: "OSTEOBLAST-SPECIFIC FACTOR 2: CLONING OF A PUTATIVE BONE ADHESION PROTEIN WITH HOMOLOGY WITH THE INSECT PROTEIN FASCICLIN I", BIOCHEMICAL JOURNAL, THE BIOCHEMICAL SOCIETY, LONDON, GB, vol. 294, 1 January 1993 (1993-01-01), pages 271-278, XP002940428, ISSN: 0264-6021
- SASAKI HIDEFUMI ET AL: "Elevated serum periostin levels in patients with bone metastases from breast but not lung cancer", BREAST CANCER RESEARCH AND TREATMENT, SPRINGER, NEW YORK, NY, vol. 77, no. 3, 1 February 2003 (2003-02-01), pages 245-252, XP002541523, ISSN: 0167-6806, DOI: 10.1023/A:1021899904332
- HORIUCHI K ET AL: "Identification and characterization of a novel protein, periostin, with restricted expression to periosteum and periodontal ligament and increased expression by transforming growth factor beta", JOURNAL OF BONE AND MINERAL RESEARCH, AMERICAN SOCIETY FOR BONE AND MINERAL RESEARCH, NEW YORK, NY, US, vol. 14, no. 7, 1 January 1999 (1999-01-01), pages 1239-1249, XP003015313, ISSN: 0884-0431, DOI: 10.1359/JBMR.1999.14.7.1239
- Y KUDO ET AL: "Periostin: novel diagnostic and therapeutic target for cancer.", HISTOL HISTOPATHOL., vol. 10, 1 October 2007 (2007-10-01), pages 1167-1174, XP055048350,
- CHUL KIM ET AL: "Role of alternative splicing of periostin in human bladder carcinogenesis.", INTERNATIONAL JOURNAL OF ONCOLOGY, vol. 32, no. 1, 1 January 2008 (2008-01-01), pages 161-169, XP055048401, ISSN: 1019-6439
- KIM C.-J. ET AL.: 'Periostin no alternative splicing variants to Akuseika Oyobi Shijun.Ten-i tono Kanren' ANNUAL MEETING OF THE JAPAN CANCER ASSOCIATION KIJI vol. 65TH, 2006, page 302 + ABSTR. NO. P-572
- TAI I.T. ET AL.: 'Periostin induction in tumor cell line explants and inhibition of in vitro cell growth by anti-periostin antibodies' CARCINOGENESIS vol. 26, no. 5, 2005, pages 908 - 915, XP008127943

### SEQUENCE LISTING

<110> Asubio Pharma Co., Ltd.
   Å@Å@Å@ Osaka University
<120> A pharmaceutical composition for treating a cancer, which comprises an
   antibody against an peptide coded by Exon-17 in Periostin
<130> YCT-1386
<150> JP 2007-169494
   <151> 2007-06-27
<150> JP 2008-033827
   <151> 2008-02-14
<160> 36
<170> PatentIn version 3.1
<210> 1
   <211> 838
   <212> PRT
   <213> Rattus sp.
<400> 1
   Met Val Pro Leu Leu Pro Leu Ser Ala Leu Leu Leu Leu Phe Leu Cys
   1               5                   10                  15
   Asp Val Asp Pro Ala Asn Ala Asn Ser Tyr Tyr Asp Lys Val Leu Ala
               20                  25                  30
   His Ser Arg Ile Arg Gly Arg Asp Gln Gly Pro Asn Val Cys Ala Leu
           35                  40                  45
   Gln Gln Ile Leu Gly Thr Lys Lys Lys Tyr Phe Ser Ser Cys Lys Asn
       50                  55                  60
   Trp Tyr Gln Gly Ala Ile Cys Gly Lys Lys Thr Thr Val Leu Tyr Glu
   65                  70                  75                  80
   Cys Cys Pro Gly Tyr Met Arg Met Glu Gly Met Lys Gly Cys Pro Ala
                   85                  90                  95
   Val Met Pro Ile Asp His Val Tyr Gly Thr Leu Gly Ile Val Gly Ala
               100                 105                 110
   Thr Thr Thr Gln His Tyr Ser Asp Val Ser Lys Leu Arg Glu Glu Ile
           115                 120                 125
   Glu Gly Lys Gly Ser Tyr Thr Tyr Phe Ala Pro Ser Asn Glu Ala Trp
       130                 135                 140
   Asp Asn Leu Asp Ser Asp Ile Arg Arg Gly Leu Glu Asn Asn Val Asn
   145                 150                 155                 160
   Val Glu Leu Leu Asn Ala Leu His Ser His Met Val Asn Lys Arg Met
                   165                 170                 175
   Leu Thr Lys Asp Leu Lys His Gly Met Val Ile Pro Ser Met Tyr Asn
               180                 185                 190
   Asn Leu Gly Leu Phe Ile Asn His Tyr Pro Asn Gly Val Val Thr Val
           195                 200                 205
   Asn Cys Ala Arg Val Ile His Gly Asn Gln Ile Ala Thr Asn Gly Val
       210                 215                 220
   Val His Val Ile Asp Arg Val Leu Thr Gln Ile Gly Thr Ser Ile Gln
   225                 230                 235                 240
   Asp Phe Ile Glu Ala Glu Asp Glu Leu Ser Ser Phe Arg Ala Ala Ala
                   245                 250                 255
   Ile Thr Ser Asp Leu Leu Glu Ser Leu Gly Arg Asp Gly His Phe Thr
               260                 265                 270
   Leu Phe Ala Pro Thr Asn Glu Ala Phe Glu Lys Leu Pro Arg Gly Val
           275                 280                 285
   Leu Glu Arg Ile Met Gly Asp Lys Val Ala Ser Glu Ala Leu Met Lys
       290                 295                 300
   Tyr His Ile Leu Asn Thr Leu Gln Cys Ser Glu Ala Ile Thr Gly Gly
   305                 310                 315                 320
   Ala Val Phe Glu Thr Met Glu Gly Asn Thr Ile Glu Ile Gly Cys Glu
                   325                 330                 335
   Gly Asp Ser Ile Ser Ile Asn Gly Ile Lys Met Val Asn Lys Lys Asp
               340                 345                 350
   Ile Val Thr Lys Asn Gly Val Ile His Leu Ile Asp Glu Val Leu Ile
           355                 360                 365
   Pro Asp Ser Ala Lys Gln Val Ile Glu Leu Ala Gly Lys Gln Gln Thr
       370                 375                 380
   Thr Phe Thr Asp Leu Val Ala Gln Leu Gly Leu Ala Ser Ser Leu Lys
   385                 390                 395                 400
   Pro Asp Gly Glu Tyr Thr Leu Leu Ala Pro Val Asn Asn Ala Phe Ser
                   405                 410                 415
   Asp Asp Thr Leu Ser Met Asp Gln Arg Leu Leu Lys Leu Ile Leu Gln
               420                 425                 430
   Asn His Ile Leu Lys Val Lys Val Gly Leu Ser Asp Leu Tyr Asn Gly
           435                 440                 445
   Gln Ile Leu Glu Thr Ile Gly Gly Lys Gln Leu Arg Val Phe Val Tyr
       450                 455                 460
   Arg Thr Ala Ile Cys Ile Glu Asn Ser Cys Met Val Arg Gly Ser Lys
   465                 470                 475                 480
   Gln Gly Arg Asn Gly Ala Ile His Ile Phe Arg Glu Ile Ile Gln Pro
                   485                 490                 495
   Ala Glu Lys Ser Leu His Glu Lys Leu Arg Gln Asp Lys Arg Phe Ser
               500                 505                 510
   Ile Phe Leu Ser Leu Leu Glu Ala Ala Asp Leu Lys Asp Leu Leu Thr
           515                 520                 525
   Gln Pro Gly Asp Trp Thr Leu Phe Ala Pro Thr Asn Asp Ala Phe Lys
       530                 535                 540
   Gly Met Thr Asn Glu Glu Arg Glu Ile Leu Ile Gly Asp Lys Asn Ala
   545                 550                 555                 560
   Leu Gln Asn Ile Ile Leu Tyr His Leu Thr Pro Gly Val Tyr Ile Gly
                   565                 570                 575
   Lys Gly Phe Glu Pro Gly Val Thr Asn Ile Leu Lys Thr Thr Gln Gly
               580                 585                 590
   Ser Lys Ile Tyr Val Lys Gly Val Asn Glu Thr Leu Leu Val Asn Glu
           595                 600                 605
   Leu Lys Ser Lys Glu Ser Asp Ile Met Thr Thr Asn Gly Val Ile His
       610                 615                 620
   Val Val Asp Lys Leu Leu Tyr Pro Ala Asp Ile Pro Val Gly Asn Asp
   625                 630                 635                 640
   Gln Leu Leu Glu Leu Leu Asn Lys Leu Ile Lys Tyr Ile Gln Ile Lys
                   645                 650                 655
   Phe Val Arg Gly Ser Thr Phe Lys Glu Ile Pro Met Thr Val Tyr Thr
               660                 665                 670
   Thr Lys Ile Ile Thr Lys Leu Val Glu Pro Lys Ile Lys Val Ile Gln
           675                 680                 685
   Gly Ser Leu Gln Pro Ile Ile Lys Thr Glu Gly Pro Ala Met Thr Lys
       690                 695                 700
   Ile His Ile Glu Gly Glu Pro Asp Phe Arg Leu Ile Lys Glu Gly Glu
   705                 710                 715                 720
   Thr Val Thr Glu Val Ile His Gly Glu Pro Val Ile Lys Lys Tyr Thr
                   725                 730                 735
   Lys Ile Ile Asp Gly Val Pro Val Glu Ile Thr Glu Lys Glu Thr Arg
               740                 745                 750
   Glu Glu Arg Ile Ile Thr Gly Pro Glu Ile Lys Tyr Thr Arg Ile Ser
           755                 760                 765
   Thr Gly Gly Gly Glu Thr Glu Glu Thr Leu Gln Lys Phe Leu Gln Lys
       770                 775                 780
   Glu Val Ser Lys Val Thr Lys Phe Ile Glu Gly Gly Asp Gly His Leu
   785                 790                 795                 800
   Phe Glu Asp Glu Ala Ile Lys Arg Leu Leu Gln Gly Asp Thr Pro Ala
                   805                 810                 815
   Lys Lys Ile Gln Ala Asn Lys Arg Val Gln Gly Ser Arg Arg Arg Ser
               820                 825                 830
   Arg Glu Gly Arg Ser Gln
           835
<210> 2
   <211> 836
   <212> PRT
   <213> Homo sapiens
<400> 2
   Met Ile Pro Phe Leu Pro Met Phe Ser Leu Leu Leu Leu Leu Ile Val
   1               5                   10                  15
   Asn Pro Ile Asn Ala Asn Asn His Tyr Asp Lys Ile Leu Ala His Ser
               20                  25                  30
   Arg Ile Arg Gly Arg Asp Gln Gly Pro Asn Val Cys Ala Leu Gln Gln
           35                  40                  45
   Ile Leu Gly Thr Lys Lys Lys Tyr Phe Ser Thr Cys Lys Asn Trp Tyr
       50                  55                  60
   Lys Lys Ser Ile Cys Gly Gln Lys Thr Thr Val Leu Tyr Glu Cys Cys
   65                  70                  75                  80
   Pro Gly Tyr Met Arg Met Glu Gly Met Lys Gly Cys Pro Ala Val Leu
                   85                  90                  95
   Pro Ile Asp His Val Tyr Gly Thr Leu Gly Ile Val Gly Ala Thr Thr
               100                 105                 110
   Thr Gln Arg Tyr Ser Asp Ala Ser Lys Leu Arg Glu Glu Ile Glu Gly
           115                 120                 125
   Lys Gly Ser Phe Thr Tyr Phe Ala Pro Ser Asn Glu Ala Trp Asp Asn
       130                 135                 140
   Leu Asp Ser Asp Ile Arg Arg Gly Leu Glu Ser Asn Val Asn Val Glu
   145                 150                 155                 160
   Leu Leu Asn Ala Leu His Ser His Met Ile Asn Lys Arg Met Leu Thr
                   165                 170                 175
   Lys Asp Leu Lys Asn Gly Met Ile Ile Pro Ser Met Tyr Asn Asn Leu
               180                 185                 190
   Gly Leu Phe Ile Asn His Tyr Pro Asn Gly Val Val Thr Val Asn Cys
           195                 200                 205
   Ala Arg Ile Ile His Gly Asn Gln Ile Ala Thr Asn Gly Val Val His
       210                 215                 220
   Val Ile Asp Arg Val Leu Thr Gln Ile Gly Thr Ser Ile Gln Asp Phe
   225                 230                 235                 240
   Ile Glu Ala Glu Asp Asp Leu Ser Ser Phe Arg Ala Ala Ala Ile Thr
                   245                 250                 255
   Ser Asp Ile Leu Glu Ala Leu Gly Arg Asp Gly His Phe Thr Leu Phe
               260                 265                 270
   Ala Pro Thr Asn Glu Ala Phe Glu Lys Leu Pro Arg Gly Val Leu Glu
           275                 280                 285
   Arg Phe Met Gly Asp Lys Val Ala Ser Glu Ala Leu Met Lys Tyr His
       290                 295                 300
   Ile Leu Asn Thr Leu Gln Cys Ser Glu Ser Ile Met Gly Gly Ala Val
   305                 310                 315                 320
   Phe Glu Thr Leu Glu Gly Asn Thr Ile Glu Ile Gly Cys Asp Gly Asp
                   325                 330                 335
   Ser Ile Thr Val Asn Gly Ile Lys Met Val Asn Lys Lys Asp Ile Val
               340                 345                 350
   Thr Asn Asn Gly Val Ile His Leu Ile Asp Gln Val Leu Ile Pro Asp
           355                 360                 365
   Ser Ala Lys Gln Val Ile Glu Leu Ala Gly Lys Gln Gln Thr Thr Phe
       370                 375                 380
   Thr Asp Leu Val Ala Gln Leu Gly Leu Ala Ser Ala Leu Arg Pro Asp
   385                 390                 395                 400
   Gly Glu Tyr Thr Leu Leu Ala Pro Val Asn Asn Ala Phe Ser Asp Asp
                   405                 410                 415
   Thr Leu Ser Met Val Gln Arg Leu Leu Lys Leu Ile Leu Gln Asn His
               420                 425                 430
   Ile Leu Lys Val Lys Val Gly Leu Asn Glu Leu Tyr Asn Gly Gln Ile
           435                 440                 445
   Leu Glu Thr Ile Gly Gly Lys Gln Leu Arg Val Phe Val Tyr Arg Thr
       450                 455                 460
   Ala Val Cys Ile Glu Asn Ser Cys Met Glu Lys Gly Ser Lys Gln Gly
   465                 470                 475                 480
   Arg Asn Gly Ala Ile His Ile Phe Arg Glu Ile Ile Lys Pro Ala Glu
                   485                 490                 495
   Lys Ser Leu His Glu Lys Leu Lys Gln Asp Lys Arg Phe Ser Thr Phe
               500                 505                 510
   Leu Ser Leu Leu Glu Ala Ala Asp Leu Lys Glu Leu Leu Thr Gln Pro
           515                 520                 525
   Gly Asp Trp Thr Leu Phe Val Pro Thr Asn Asp Ala Phe Lys Gly Met
       530                 535                 540
   Thr Ser Glu Glu Lys Glu Ile Leu Ile Arg Asp Lys Asn Ala Leu Gln
   545                 550                 555                 560
   Asn Ile Ile Leu Tyr His Leu Thr Pro Gly Val Phe Ile Gly Lys Gly
                   565                 570                 575
   Phe Glu Pro Gly Val Thr Asn Ile Leu Lys Thr Thr Gln Gly Ser Lys
               580                 585                 590
   Ile Phe Leu Lys Glu Val Asn Asp Thr Leu Leu Val Asn Glu Leu Lys
           595                 600                 605
   Ser Lys Glu Ser Asp Ile Met Thr Thr Asn Gly Val Ile His Val Val
       610                 615                 620
   Asp Lys Leu Leu Tyr Pro Ala Asp Thr Pro Val Gly Asn Asp Gln Leu
   625                 630                 635                 640
   Leu Glu Ile Leu Asn Lys Leu Ile Lys Tyr Ile Gln Ile Lys Phe Val
                   645                 650                 655
   Arg Gly Ser Thr Phe Lys Glu Ile Pro Val Thr Val Tyr Thr Thr Lys
               660                 665                 670
   Ile Ile Thr Lys Val Val Glu Pro Lys Ile Lys Val Ile Glu Gly Ser
           675                 680                 685
   Leu Gln Pro Ile Ile Lys Thr Glu Gly Pro Thr Leu Thr Lys Val Lys
       690                 695                 700
   Ile Glu Gly Glu Pro Glu Phe Arg Leu Ile Lys Glu Gly Glu Thr Ile
   705                 710                 715                 720
   Thr Glu Val Ile His Gly Glu Pro Ile Ile Lys Lys Tyr Thr Lys Ile
                   725                 730                 735
   Ile Asp Gly Val Pro Val Glu Ile Thr Glu Lys Glu Thr Arg Glu Glu
               740                 745                 750
   Arg Ile Ile Thr Gly Pro Glu Ile Lys Tyr Thr Arg Ile Ser Thr Gly
           755                 760                 765
   Gly Gly Glu Thr Glu Glu Thr Leu Lys Lys Leu Leu Gln Glu Glu Val
       770                 775                 780
   Thr Lys Val Thr Lys Phe Ile Glu Gly Gly Asp Gly His Leu Phe Glu
   785                 790                 795                 800
   Asp Glu Glu Ile Lys Arg Leu Leu Gln Gly Asp Thr Pro Val Arg Lys
                   805                 810                 815
   Leu Gln Ala Asn Lys Lys Val Gln Gly Ser Arg Arg Arg Leu Arg Glu
               820                 825                 830
   Gly Arg Ser Gln
           835
<210> 3
   <211> 27
   <212> PRT
   <213> Rattus sp.
<400> 3
   Thr Thr Lys Ile Ile Thr Lys Leu Val Glu Pro Lys Ile Lys Val Ile
   1               5                   10                  15
   Gln Gly Ser Leu Gln Pro Ile Ile Lys Thr Glu
               20                  25
<210> 4
   <211> 27
   <212> PRT
   <213> Homo sapiens
<400> 4
   Thr Thr Lys Ile Ile Thr Lys Val Val Glu Pro Lys Ile Lys Val Ile
   1               5                   10                  15
   Glu Gly Ser Leu Gln Pro Ile Ile Lys Thr Glu
               20                  25
<210> 5
   <211> 811
   <212> PRT
   <213> Rattus sp.
<400> 5
   Met Val Pro Leu Leu Pro Leu Ser Ala Leu Leu Leu Leu Phe Leu Cys
   1               5                   10                  15
   Asp Val Asp Pro Ala Asn Ala Asn Ser Tyr Tyr Asp Lys Val Leu Ala
               20                  25                  30
   His Ser Arg Ile Arg Gly Arg Asp Gln Gly Pro Asn Val Cys Ala Leu
           35                  40                  45
   Gln Gln Ile Leu Gly Thr Lys Lys Lys Tyr Phe Ser Ser Cys Lys Asn
       50                  55                  60
   Trp Tyr Gln Gly Ala Ile Cys Gly Lys Lys Thr Thr Val Leu Tyr Glu
   65                  70                  75                  80
   Cys Cys Pro Gly Tyr Met Arg Met Glu Gly Met Lys Gly Cys Pro Ala
                   85                  90                  95
   Val Met Pro Ile Asp His Val Tyr Gly Thr Leu Gly Ile Val Gly Ala
               100                 105                 110
   Thr Thr Thr Gln His Tyr Ser Asp Val Ser Lys Leu Arg Glu Glu Ile
           115                 120                 125
   Glu Gly Lys Gly Ser Tyr Thr Tyr Phe Ala Pro Ser Asn Glu Ala Trp
       130                 135                 140
   Asp Asn Leu Asp Ser Asp Ile Arg Arg Gly Leu Glu Asn Asn Val Asn
   145                 150                 155                 160
   Val Glu Leu Leu Asn Ala Leu His Ser His Met Val Asn Lys Arg Met
                   165                 170                 175
   Leu Thr Lys Asp Leu Lys His Gly Met Val Ile Pro Ser Met Tyr Asn
               180                 185                 190
   Asn Leu Gly Leu Phe Ile Asn His Tyr Pro Asn Gly Val Val Thr Val
           195                 200                 205
   Asn Cys Ala Arg Val Ile His Gly Asn Gln Ile Ala Thr Asn Gly Val
       210                 215                 220
   Val His Val Ile Asp Arg Val Leu Thr Gln Ile Gly Thr Ser Ile Gln
   225                 230                 235                 240
   Asp Phe Ile Glu Ala Glu Asp Glu Leu Ser Ser Phe Arg Ala Ala Ala
                   245                 250                 255
   Ile Thr Ser Asp Leu Leu Glu Ser Leu Gly Arg Asp Gly His Phe Thr
               260                 265                 270
   Leu Phe Ala Pro Thr Asn Glu Ala Phe Glu Lys Leu Pro Arg Gly Val
           275                 280                 285
   Leu Glu Arg Ile Met Gly Asp Lys Val Ala Ser Glu Ala Leu Met Lys
       290                 295                 300
   Tyr His Ile Leu Asn Thr Leu Gln Cys Ser Glu Ala Ile Thr Gly Gly
   305                 310                 315                 320
   Ala Val Phe Glu Thr Met Glu Gly Asn Thr Ile Glu Ile Gly Cys Glu
                   325                 330                 335
   Gly Asp Ser Ile Ser Ile Asn Gly Ile Lys Met Val Asn Lys Lys Asp
               340                 345                 350
   Ile Val Thr Lys Asn Gly Val Ile His Leu Ile Asp Glu Val Leu Ile
           355                 360                 365
   Pro Asp Ser Ala Lys Gln Val Ile Glu Leu Ala Gly Lys Gln Gln Thr
       370                 375                 380
   Thr Phe Thr Asp Leu Val Ala Gln Leu Gly Leu Ala Ser Ser Leu Lys
   385                 390                 395                 400
   Pro Asp Gly Glu Tyr Thr Leu Leu Ala Pro Val Asn Asn Ala Phe Ser
                   405                 410                 415
   Asp Asp Thr Leu Ser Met Asp Gln Arg Leu Leu Lys Leu Ile Leu Gln
               420                 425                 430
   Asn His Ile Leu Lys Val Lys Val Gly Leu Ser Asp Leu Tyr Asn Gly
           435                 440                 445
   Gln Ile Leu Glu Thr Ile Gly Gly Lys Gln Leu Arg Val Phe Val Tyr
       450                 455                 460
   Arg Thr Ala Ile Cys Ile Glu Asn Ser Cys Met Val Arg Gly Ser Lys
   465                 470                 475                 480
   Gln Gly Arg Asn Gly Ala Ile His Ile Phe Arg Glu Ile Ile Gln Pro
                   485                 490                 495
   Ala Glu Lys Ser Leu His Glu Lys Leu Arg Gln Asp Lys Arg Phe Ser
               500                 505                 510
   Ile Phe Leu Ser Leu Leu Glu Ala Ala Asp Leu Lys Asp Leu Leu Thr
           515                 520                 525
   Gln Pro Gly Asp Trp Thr Leu Phe Ala Pro Thr Asn Asp Ala Phe Lys
       530                 535                 540
   Gly Met Thr Asn Glu Glu Arg Glu Ile Leu Ile Gly Asp Lys Asn Ala
   545                 550                 555                 560
   Leu Gln Asn Ile Ile Leu Tyr His Leu Thr Pro Gly Val Tyr Ile Gly
                   565                 570                 575
   Lys Gly Phe Glu Pro Gly Val Thr Asn Ile Leu Lys Thr Thr Gln Gly
               580                 585                 590
   Ser Lys Ile Tyr Val Lys Gly Val Asn Glu Thr Leu Leu Val Asn Glu
           595                 600                 605
   Leu Lys Ser Lys Glu Ser Asp Ile Met Thr Thr Asn Gly Val Ile His
       610                 615                 620
   Val Val Asp Lys Leu Leu Tyr Pro Ala Asp Ile Pro Val Gly Asn Asp
   625                 630                 635                 640
   Gln Leu Leu Glu Leu Leu Asn Lys Leu Ile Lys Tyr Ile Gln Ile Lys
                   645                 650                 655
   Phe Val Arg Gly Ser Thr Phe Lys Glu Ile Pro Met Thr Val Tyr Arg
               660                 665                 670
   Pro Ala Met Thr Lys Ile His Ile Glu Gly Glu Pro Asp Phe Arg Leu
           675                 680                 685
   Ile Lys Glu Gly Glu Thr Val Thr Glu Val Ile His Gly Glu Pro Val
       690                 695                 700
   Ile Lys Lys Tyr Thr Lys Ile Ile Asp Gly Val Pro Val Glu Ile Thr
   705                 710                 715                 720
   Glu Lys Glu Thr Arg Glu Glu Arg Ile Ile Thr Gly Pro Glu Ile Lys
                   725                 730                 735
   Tyr Thr Arg Ile Ser Thr Gly Gly Gly Glu Thr Glu Glu Thr Leu Gln
               740                 745                 750
   Lys Phe Leu Gln Lys Glu Val Ser Lys Val Thr Lys Phe Ile Glu Gly
           755                 760                 765
   Gly Asp Gly His Leu Phe Glu Asp Glu Ala Ile Lys Arg Leu Leu Gln
       770                 775                 780
   Gly Asp Thr Pro Ala Lys Lys Ile Gln Ala Asn Lys Arg Val Gln Gly
   785                 790                 795                 800
   Ser Arg Arg Arg Ser Arg Glu Gly Arg Ser Gln
                   805                 810
<210> 6
   <211> 2517
   <212> DNA
   <213> Rattus sp.
<400> 6
   atggttcctc tcctgccctt atctgctctg ctgctgctgt tcctgtgtga cgttgacccc      60
   gcaaatgcca acagttacta tgacaaggtc ctagctcaca gccgcatcag gggtcgggat     120
   cagggcccaa atgtctgtgc cctccagcag attctgggca ccaaaaagaa atacttcagc     180
   tcctgtaaga actggtatca aggtgctatc tgcgggaaga aaaccactgt gctatatgaa     240
   tgctgccccg gctatatgag aatggaaggg atgaaaggct gcccagcagt gatgcccatt     300
   gaccatgttt atggcacgct gggcatcgtg ggagccacga ccactcaaca ctattctgat     360
   gtctcgaagc tcagggaaga gattgaagga aaagggtcct acacatactt cgcgccgagt     420
   aacgaagctt gggacaacct ggattccgac atccgcagag gactagagaa caatgtcaat     480
   gttgagttac tgaacgcttt acacagccac atggttaata agagaatgct aaccaaggac     540
   ctgaaacacg gcatggttat tccttcaatg tacaacaatc tggggctttt tatcaatcat     600
   tatcccaatg gggttgtcac tgtgaactgt gctcgagtaa tccacgggaa ccagattgcc     660
   acaaatggtg ttgtccatgt catcgaccgt gtcctgacac aaattggcac ctccatccaa     720
   gacttcattg aagcagaaga tgagctttca tcattcagag cggctgccat cacttctgac     780
   cttttggagt cccttggaag agacggtcac ttcacactct ttgctcccac caatgaggct     840
   ttcgagaaac tcccacgagg agtcctagaa aggatcatgg gagacaaagt ggcttctgaa     900
   gctctcatga agtaccacat cctgaatacc ctccagtgct ctgaggctat cacaggagga     960
   gcggtgtttg agaccatgga aggaaacact attgaaatag ggtgtgaggg agacagcatc    1020
   tccattaacg gaatcaagat ggtgaacaag aaagacattg tgacgaagaa tggtgtcatc    1080
   cacctgattg atgaagtcct cattcctgat tctgctaaac aagttattga gctggctgga    1140
   aaacagcaaa ccactttcac ggacctggta gcccagttag ggttggcgtc ttctctgaag    1200
   ccggatggag agtacacgct gttagcgcct gtgaacaatg cgttctctga tgacactctg    1260
   agcatggacc agcgccttct taagctaatt ctgcaaaatc acatattgaa agtaaaagtc    1320
   ggccttagtg atctctacaa tggacagatt ctggagacca ttggaggcaa acaactccgt    1380
   gtcttcgtgt atcggacggc tatctgcata gaaaactcat gcatggtgag aggaagcaag    1440
   caggggagga acggtgccat tcacatattc cgagagatca tccaaccggc ggagaagtcc    1500
   ctgcacgaaa aactgcgcca agataagcgc ttcagcatct tcctcagcct cctcgaagct    1560
   gcagatctga aagatcttct gacacagccc ggagattgga ccttgtttgc accaaccaat    1620
   gatgccttca agggaatgac taatgaagaa agggagattc tgattgggga taaaaatgct    1680
   ctccaaaaca tcattcttta ccacctgacc ccaggggttt atattggaaa gggatttgaa    1740
   cccggagtca ccaacatcct gaagaccaca cagggaagca aaatctatgt gaaaggagtc    1800
   aatgagacgc ttttggtgaa tgagttgaag tccaaagaat ctgacatcat gacaacaaac    1860
   ggcgtcattc acgttgtgga caaactcctc tatccagcag acattccggt tggaaatgat    1920
   cagctcttgg aattactgaa caaactgata aaatacatcc aaattaagtt cgttcgtggc    1980
   agcaccttca aagaaatccc catgactgtc tatacaacta aaattataac caaactcgtg    2040
   gaaccaaaaa ttaaagtcat tcaaggcagt cttcagccta ttatcaaaac agaaggacct    2100
   gcaatgacga agatccacat tgaaggcgag cctgacttca ggctgattaa agaaggtgaa    2160
   acagtgacag aagtgatcca cggagaacca gtcattaaaa agtacaccaa aatcatagac    2220
   ggggttcctg ttgaaataac tgaaaaagag acccgggaag aacgcatcat cacaggtcct    2280
   gagataaaat acactaggat ttccacagga ggtggggaaa cagaagagac cctgcagaaa    2340
   ttcttgcaaa aagaggtctc caaggtcaca aagttcattg aaggtggcga tggtcactta    2400
   tttgaagatg aggcgattaa aagactgctt cagggagaca cacctgcaaa gaagatacaa    2460
   gccaacaaaa gggttcaagg gtctagaagg cgatcaagag aaggccgttc tcagtga       2517
<210> 7
   <211> 2436
   <212> DNA
   <213> Rattus sp.
<400> 7
   atggttcctc tcctgccctt atctgctctg ctgctgctgt tcctgtgtga cgttgacccc 60
   gcaaatgcca acagttacta tgacaaggtc ctagctcaca gccgcatcag gggtcgggat 120
   cagggcccaa atgtctgtgc cctccagcag attctgggca ccaaaaagaa atacttcagc 180
   tcctgtaaga actggtatca aggtgctatc tgcgggaaga aaaccactgt gctatatgaa 240
   tgctgccccg gctatatgag aatggaaggg atgaaaggct gcccagcagt gatgcccatt 300
   gaccatgttt atggcacgct gggcatcgtg ggagccacga ccactcaaca ctattctgat 360
   gtctcgaagc tcagggaaga gattgaagga aaagggtcct acacatactt cgcgccgagt 420
   aacgaagctt gggacaacct ggattccgac atccgcagag gactagagaa caatgtcaat 480
   gttgagttac tgaacgcttt acacagccac atggttaata agagaatgct aaccaaggac 540
   ctgaaacacg gcatggttat tccttcaatg tacaacaatc tggggctttt tatcaatcat 600
   tatcccaatg gggttgtcac tgtgaactgt gctcgagtaa tccacgggaa ccagattgcc 660
   acaaatggtg ttgtccatgt catcgaccgt gtcctgacac aaattggcac ctccatccaa 720
   gacttcattg aagcagaaga tgagctttca tcattcagag cggctgccat cacttctgac 780
   cttttggagt cccttggaag agacggtcac ttcacactct ttgctcccac caatgaggct 840
   ttcgagaaac tcccacgagg agtcctagaa aggatcatgg gagacaaagt ggcttctgaa 900
   gctctcatga agtaccacat cctgaatacc ctccagtgct ctgaggctat cacaggagga 960
   gcggtgtttg agaccatgga aggaaacact attgaaatag ggtgtgaggg agacagcatc 1020
   tccattaacg gaatcaagat ggtgaacaag aaagacattg tgacgaagaa tggtgtcatc 1080
   cacctgattg atgaagtcct cattcctgat tctgctaaac aagttattga gctggctgga 1140
   aaacagcaaa ccactttcac ggacctggta gcccagttag ggttggcgtc ttctctgaag 1200
   ccggatggag agtacacgct gttagcgcct gtgaacaatg cgttctctga tgacactctg 1260
   agcatggacc agcgccttct taagctaatt ctgcaaaatc acatattgaa agtaaaagtc 1320
   ggccttagtg atctctacaa tggacagatt ctggagacca ttggaggcaa acaactccgt 1380
   gtcttcgtgt atcggacggc tatctgcata gaaaactcat gcatggtgag aggaagcaag 1440
   caggggagga acggtgccat tcacatattc cgagagatca tccaaccggc ggagaagtcc 1500
   ctgcacgaaa aactgcgcca agataagcgc ttcagcatct tcctcagcct cctcgaagct 1560
   gcagatctga aagatcttct gacacagccc ggagattgga ccttgtttgc accaaccaat 1620
   gatgccttca agggaatgac taatgaagaa agggagattc tgattgggga taaaaatgct 1680
   ctccaaaaca tcattcttta ccacctgacc ccaggggttt atattggaaa gggatttgaa 1740
   cccggagtca ccaacatcct gaagaccaca cagggaagca aaatctatgt gaaaggagtc   1800
   aatgagacgc ttttggtgaa tgagttgaag tccaaagaat ctgacatcat gacaacaaac   1860
   ggcgtcattc acgttgtgga caaactcctc tatccagcag acattccggt tggaaatgat   1920
   cagctcttgg aattactgaa caaactgata aaatacatcc aaattaagtt cgttcgtggc   1980
   agcaccttca aagaaatccc catgactgtc tatagacctg caatgacgaa gatccacatt   2040
   gaaggcgagc ctgacttcag gctgattaaa gaaggtgaaa cagtgacaga agtgatccac   2100
   ggagaaccag tcattaaaaa gtacaccaaa atcatagacg gggttcctgt tgaaataact   2160
   gaaaaagaga cccgggaaga acgcatcatc acaggtcctg agataaaata cactaggatt   2220
   tccacaggag gtggggaaac agaagagacc ctgcagaaat tcttgcaaaa agaggtctcc   2280
   aaggtcacaa agttcattga aggtggcgat ggtcacttat ttgaagatga ggcgattaaa   2340
   agactgcttc agggagacac acctgcaaag aagatacaag ccaacaaaag ggttcaaggg   2400
   tctagaaggc gatcaagaga aggccgttct cagtga                             2436
<210> 8
   <211> 838
   <212> PRT
   <213> Mus musculus
<400> 8
   Met Val Pro Leu Leu Pro Leu Tyr Ala Leu Leu Leu Leu Phe Leu Cys
   1               5                   10                  15
   Asp Ile Asn Pro Ala Asn Ala Asn Ser Tyr Tyr Asp Lys Val Leu Ala
               20                  25                  30
   His Ser Arg Ile Arg Gly Arg Asp Gln Gly Pro Asn Val Cys Ala Leu
           35                  40                  45
   Gln Gln Ile Leu Gly Thr Lys Lys Lys Tyr Phe Ser Ser Cys Lys Asn
       50                  55                  60
   Trp Tyr Gln Gly Ala Ile Cys Gly Lys Lys Thr Thr Val Leu Tyr Glu
   65                  70                  75                  80
   Cys Cys Pro Gly Tyr Met Arg Met Glu Gly Met Lys Gly Cys Pro Ala
                   85                  90                  95
   Val Met Pro Ile Asp His Val Tyr Gly Thr Leu Gly Ile Val Gly Ala
               100                 105                 110
   Thr Thr Thr Gln His Tyr Ser Asp Val Ser Lys Leu Arg Glu Glu Ile
   115 120 125
   Glu Gly Lys Gly Ser Tyr Thr Tyr Phe Ala Pro Ser Asn Glu Ala Trp
   130 135 140
   Glu Asn Leu Asp Ser Asp Ile Arg Arg Gly Leu Glu Asn Asn Val Asn
   145 150 155 160
   Val Glu Leu Leu Asn Ala Leu His Ser His Met Val Asn Lys Arg Met
   165 170 175
   Leu Thr Lys Asp Leu Lys His Gly Met Val Ile Pro Ser Met Tyr Asn
   180 185 190
   Asn Leu Gly Leu Phe Ile Asn His Tyr Pro Asn Gly Val Val Thr Val
   195 200 205
   Asn Cys Ala Arg Val Ile His Gly Asn Gln Ile Ala Thr Asn Gly Val
   210 215 220
   Val His Val Ile Asp Arg Val Leu Thr Gln Ile Gly Thr Ser Ile Gln
   225 230 235 240
   Asp Phe Leu Glu Ala Glu Asp Asp Leu Ser Ser Phe Arg Ala Ala Ala
   245 250 255
   Ile Thr Ser Asp Leu Leu Glu Ser Leu Gly Arg Asp Gly His Phe Thr
   260 265 270
   Leu Phe Ala Pro Thr Asn Glu Ala Phe Glu Lys Leu Pro Arg Gly Val
   275 280 285
   Leu Glu Arg Ile Met Gly Asp Lys Val Ala Ser Glu Ala Leu Met Lys
   290 295 300
   Tyr His Ile Leu Asn Thr Leu Gln Cys Ser Glu Ala Ile Thr Gly Gly
   305 310 315 320
   Ala Val Phe Glu Thr Met Glu Gly Asn Thr Ile Glu Ile Gly Cys Glu
   325 330 335
   Gly Asp Ser Ile Ser Ile Asn Gly Ile Lys Met Val Asn Lys Lys Asp
   340 345 350
   Ile Val Thr Lys Asn Gly Val Ile His Leu Ile Asp Glu Val Leu Ile
   355 360 365
   Pro Asp Ser Ala Lys Gln Val Ile Glu Leu Ala Gly Lys Gln Gln Thr
       370                 375                 380
   Thr Phe Thr Asp Leu Val Ala Gln Leu Gly Leu Ala Ser Ser Leu Lys
   385                 390                 395                 400
   Pro Asp Gly Glu Tyr Thr Leu Leu Ala Pro Val Asn Asn Ala Phe Ser
                   405                 410                 415
   Asp Asp Thr Leu Ser Met Asp Gln Arg Leu Leu Lys Leu Ile Leu Gln
               420                 425                 430
   Asn His Ile Leu Lys Val Lys Val Gly Leu Ser Asp Leu Tyr Asn Gly
           435                 440                 445
   Gln Ile Leu Glu Thr Ile Gly Gly Lys Gln Leu Arg Val Phe Val Tyr
       450                 455                 460
   Arg Thr Ala Ile Cys Ile Glu Asn Ser Cys Met Val Arg Gly Ser Lys
   465                 470                 475                 480
   Gln Gly Arg Asn Gly Ala Ile His Ile Phe Arg Glu Ile Ile Gln Pro
                   485                 490                 495
   Ala Glu Lys Ser Leu His Asp Lys Leu Arg Gln Asp Lys Arg Phe Ser
               500                 505                 510
   Ile Phe Leu Ser Leu Leu Glu Ala Ala Asp Leu Lys Asp Leu Leu Thr
           515                 520                 525
   Gln Pro Gly Asp Trp Thr Leu Phe Ala Pro Thr Asn Asp Ala Phe Lys
       530                 535                 540
   Gly Met Thr Ser Glu Glu Arg Glu Leu Leu Ile Gly Asp Lys Asn Ala
   545                 550                 555                 560
   Leu Gln Asn Ile Ile Leu Tyr His Leu Thr Pro Gly Val Tyr Ile Gly
                   565                 570                 575
   Lys Gly Phe Glu Pro Gly Val Thr Asn Ile Leu Lys Thr Thr Gln Gly
               580                 585                 590
   Ser Lys Ile Tyr Leu Lys Gly Val Asn Glu Thr Leu Leu Val Asn Glu
           595                 600                 605
   Leu Lys Ser Lys Glu Ser Asp Ile Met Thr Thr Asn Gly Val Ile His
       610                 615                 620
   Val Val Asp Lys Leu Leu Tyr Pro Ala Asp Ile Pro Val Gly Asn Asp
   625                 630                 635                 640
   Gln Leu Leu Glu Leu Leu Asn Lys Leu Ile Lys Tyr Ile Gln Ile Lys
                   645                 650                 655
   Phe Val Arg Gly Ser Thr Phe Lys Glu Ile Pro Met Thr Val Tyr Thr
               660                 665                 670
   Thr Lys Ile Ile Thr Lys Val Val Glu Pro Lys Ile Lys Val Ile Gln
           675                 680                 685
   Gly Ser Leu Gln Pro Ile Ile Lys Thr Glu Gly Pro Ala Met Thr Lys
       690                 695                 700
   Ile Gln Ile Glu Gly Asp Pro Asp Phe Arg Leu Ile Lys Glu Gly Glu
   705                 710                 715                 720
   Thr Val Thr Glu Val Ile His Gly Glu Pro Val Ile Lys Lys Tyr Thr
                   725                 730                 735
   Lys Ile Ile Asp Gly Val Pro Val Glu Ile Thr Glu Lys Gln Thr Arg
               740                 745                 750
   Glu Glu Arg Ile Ile Thr Gly Pro Glu Ile Lys Tyr Thr Arg Ile Ser
           755                 760                 765
   Thr Gly Gly Gly Glu Thr Gly Glu Thr Leu Gln Lys Phe Leu Gln Lys
       770                 775                 780
   Glu Val Ser Lys Val Thr Lys Phe Ile Glu Gly Gly Asp Gly His Leu
   785                 790                 795                 800
   Phe Glu Asp Glu Glu Ile Lys Arg Leu Leu Gln Gly Asp Thr Pro Ala
                   805                 810                 815
   Lys Lys Ile Pro Ala Asn Lys Arg Val Gln Gly Pro Arg Arg Arg Ser
               820                 825                 830
   Arg Glu Gly Arg Ser Gln
           835
<210> 9
   <211> 2517
   <212> DNA
   <213> Mus musculus
<400> 9
   atggttcctc tcctgccctt atatgctctg ctgctgctgt tcctgtgtga tattaaccct     60
   gcaaatgcca acagttacta tgacaaggtc ctggctcaca gccgcatcag gggtcgggat    120
   cagggcccaa acgtctgtgc cctccagcaa attctgggca ccaaaaagaa atacttcagc    180
   tcctgtaaga actggtatca aggtgctatc tgcgggaaga aaaccactgt gctatatgaa    240
   tgctgccctg gctatatgag aatggaaggg atgaaaggct gccccgcagt gatgcctatt    300
   gaccatgttt atggcacgct gggcattgtg ggagccacta ccactcagca ctactccgat    360
   gtctcgaagc tgagagaaga gattgaagga aaagggtcat acacgtactt cgcgccgagt    420
   aacgaggctt gggagaacct ggattctgac attcgcagag gactggagaa caatgtcaat    480
   gttgagctac tgaatgcctt acacagccac atggttaata agagaatgtt aaccaaggac    540
   ctgaaacacg gcatggttat tccttcaatg tacaacaatc tggggctttt tattaaccat    600
   tatcccaatg gggttgtcac tgtgaactgt gctcgagtca tccatgggaa ccagattgcc    660
   acaaatggtg tcgtccatgt cattgaccgt gtcctgacac aaattggtac ctccatccaa    720
   gacttccttg aagcagaaga cgacctttca tcatttagag cagccgccat cacctctgac    780
   ctcttggagt cccttggaag agatggtcac ttcacgctct ttgctcccac caatgaagct    840
   ttcgagaaac tgccacgagg tgtcctagaa aggatcatgg gagacaaagt ggcttctgaa    900
   gctctcatga agtaccacat cctaaatacc ctccagtgct ctgaggccat cactggagga    960
   gccgtgtttg agaccatgga aggaaacact attgagatag ggtgcgaagg ggacagtatc   1020
   tccattaacg gaatcaagat ggtgaacaag aaagacattg tgactaagaa tggtgtcatc   1080
   cacctgattg atgaagtcct cattcctgat tctgccaaac aagttattga gctggctgga   1140
   aaacagcaaa ccactttcac cgacctggta gcccaattag gcttggcatc ctctctgaag   1200
   ccagatggag agtacacctt attagcacct gtgaacaatg cgttctctga tgacactctg   1260
   agcatggacc aacgccttct taagctaatt ctgcaaaatc acatattgaa agtaaaagtt   1320
   ggccttagcg acctctacaa tggacagata ctggaaacca ttggaggcaa acaactccga   1380
   gtctttgtgt atcggacggc tatctgcata gaaaactcat gcatggtgag aggaagcaag   1440
   cagggaagga atggtgccat tcacatattc cgagaaatca tccaaccagc agagaaatcc   1500
   ctgcacgaca agctgcggca agacaagcgc tttagcatct tcctcagcct ccttgaagct   1560
   gcagatttga aagatctcct gacacagccc ggagattgga ccttgtttgc accaaccaat   1620
   gatgccttca agggaatgac tagcgaagaa agggagcttc tgattgggga taaaaatgct   1680
   ctccaaaaca tcattcttta tcacctgacc ccaggggttt atattggaaa gggattcgaa 1740
   cccggagtca ctaatatcct gaagaccaca cagggaagca aaatctatct gaaaggagta 1800
   aacgaaacgc ttctagtgaa tgagttgaag tccaaagaat ctgacatcat gacgacaaat 1860
   ggtgtcatcc acgtcgtgga caaactcctc tatccagcag atattccagt tggaaatgat 1920
   cagctcttgg aattactgaa caaactgata aaatacatcc aaatcaagtt tgttcgtggc 1980
   agcaccttca aagaaatccc catgactgtc tatacaacta aaattataac caaagtcgtg 2040
   gaaccaaaaa ttaaagtcat tcaaggcagt cttcagccta ttatcaaaac ggaaggacct 2100
   gcaatgacga agatccaaat tgaaggtgat cccgacttca ggctgattaa agaaggcgaa 2160
   acggtgacag aagtgatcca cggagagcca gtcattaaaa agtacaccaa aatcatagat 2220
   ggagttcctg ttgaaataac tgaaaaacag actcgggaag aacgaatcat tacaggtcct 2280
   gagataaaat ataccaggat ttccacagga ggtggagaaa caggagagac cttgcagaaa 2340
   ttcttgcaaa aagaggtctc caaggtcaca aagttcattg aaggtggcga tggtcactta 2400
   tttgaagatg aggagattaa aagactgctt cagggagaca cacctgcaaa gaagatacca 2460
   gccaacaaaa gggttcaagg gcctagaaga cgatcaagag aaggccgttc tcagtga 2517
<210> 10
   <211> 811
   <212> PRT
   <213> Mus musculus
<400> 10
   Met Val Pro Leu Leu Pro Leu Tyr Ala Leu Leu Leu Leu Phe Leu Cys
   1 5 10 15
   Asp Ile Asn Pro Ala Asn Ala Asn Ser Tyr Tyr Asp Lys Val Leu Ala
   20 25 30
   His Ser Arg Ile Arg Gly Arg Asp Gln Gly Pro Asn Val Cys Ala Leu
   35 40 45
   Gln Gln Ile Leu Gly Thr Lys Lys Lys Tyr Phe Ser Ser Cys Lys Asn
   50 55 60
   Trp Tyr Gln Gly Ala Ile Cys Gly Lys Lys Thr Thr Val Leu Tyr Glu
   65 70 75 80
   Cys Cys Pro Gly Tyr Met Arg Met Glu Gly Met Lys Gly Cys Pro Ala
   85 90 95
   Val Met Pro Ile Asp His Val Tyr Gly Thr Leu Gly Ile Val Gly Ala
                   100                 105                 110
   Thr Thr Thr Gln His Tyr Ser Asp Val Ser Lys Leu Arg Glu Glu Ile
               115                 120                 125
   Glu Gly Lys Gly Ser Tyr Thr Tyr Phe Ala Pro Ser Asn Glu Ala Trp
       130                 135                 140
   Glu Asn Leu Asp Ser Asp Ile Arg Arg Gly Leu Glu Asn Asn Val Asn
   145                 150                 155                 160
   Val Glu Leu Leu Asn Ala Leu His Ser His Met Val Asn Lys Arg Met
                       165                 170                 175
   Leu Thr Lys Asp Leu Lys His Gly Met Val Ile Pro Ser Met Tyr Asn
                   180                 185                 190
   Asn Leu Gly Leu Phe Ile Asn His Tyr Pro Asn Gly Val Val Thr Val
               195                 200                 205
   Asn Cys Ala Arg Val Ile His Gly Asn Gln Ile Ala Thr Asn Gly Val
       210                 215                 220
   Val His Val Ile Asp Arg Val Leu Thr Gln Ile Gly Thr Ser Ile Gln
   225                 230                 235                 240
   Asp Phe Leu Glu Ala Glu Asp Asp Leu Ser Ser Phe Arg Ala Ala Ala
                       245                 250                 255
   Ile Thr Ser Asp Leu Leu Glu Ser Leu Gly Arg Asp Gly His Phe Thr
                   260                 265                 270
   Leu Phe Ala Pro Thr Asn Glu Ala Phe Glu Lys Leu Pro Arg Gly Val
               275                 280                 285
   Leu Glu Arg Ile Met Gly Asp Lys Val Ala Ser Glu Ala Leu Met Lys
       290                 295                 300
   Tyr His Ile Leu Asn Thr Leu Gln Cys Ser Glu Ala Ile Thr Gly Gly
   305                 310                 315                 320
   Ala Val Phe Glu Thr Met Glu Gly Asn Thr Ile Glu Ile Gly Cys Glu
                       325                 330                 335
   Gly Asp Ser Ile Ser Ile Asn Gly Ile Lys Met Val Asn Lys Lys Asp
               340                 345                 350
   Ile Val Thr Lys Asn Gly Val Ile His Leu Ile Asp Glu Val Leu Ile
           355                 360                 365
   Pro Asp Ser Ala Lys Gln Val Ile Glu Leu Ala Gly Lys Gln Gln Thr
       370                 375                 380
   Thr Phe Thr Asp Leu Val Ala Gln Leu Gly Leu Ala Ser Ser Leu Lys
   385                 390                 395                 400
   Pro Asp Gly Glu Tyr Thr Leu Leu Ala Pro Val Asn Asn Ala Phe Ser
                   405                 410                 415
   Asp Asp Thr Leu Ser Met Asp Gln Arg Leu Leu Lys Leu Ile Leu Gln
               420                 425                 430
   Asn His Ile Leu Lys Val Lys Val Gly Leu Ser Asp Leu Tyr Asn Gly
           435                 440                 445
   Gln Ile Leu Glu Thr Ile Gly Gly Lys Gln Leu Arg Val Phe Val Tyr
       450                 455                 460
   Arg Thr Ala Ile Cys Ile Glu Asn Ser Cys Met Val Arg Gly Ser Lys
   465                 470                 475                 480
   Gln Gly Arg Asn Gly Ala Ile His Ile Phe Arg Glu Ile Ile Gln Pro
                   485                 490                 495
   Ala Glu Lys Ser Leu His Asp Lys Leu Arg Gln Asp Lys Arg Phe Ser
               500                 505                 510
   Ile Phe Leu Ser Leu Leu Glu Ala Ala Asp Leu Lys Asp Leu Leu Thr
           515                 520                 525
   Gln Pro Gly Asp Trp Thr Leu Phe Ala Pro Thr Asn Asp Ala Phe Lys
       530                 535                 540
   Gly Met Thr Ser Glu Glu Arg Glu Leu Leu Ile Gly Asp Lys Asn Ala
   545                 550                 555                 560
   Leu Gln Asn Ile Ile Leu Tyr His Leu Thr Pro Gly Val Tyr Ile Gly
                   565                 570                 575
   Lys Gly Phe Glu Pro Gly Val Thr Asn Ile Leu Lys Thr Thr Gln Gly
               580                 585                 590
   Ser Lys Ile Tyr Leu Lys Gly Val Asn Glu Thr Leu Leu Val Asn Glu
           595                 600                 605
   Leu Lys Ser Lys Glu Ser Asp Ile Met Thr Thr Asn Gly Val Ile His
       610                 615                 620
   Val Val Asp Lys Leu Leu Tyr Pro Ala Asp Ile Pro Val Gly Asn Asp
   625                 630                 635                 640
   Gln Leu Leu Glu Leu Leu Asn Lys Leu Ile Lys Tyr Ile Gln Ile Lys
                   645                 650                 655
   Phe Val Arg Gly Ser Thr Phe Lys Glu Ile Pro Met Thr Val Tyr Arg
               660                 665                 670
   Pro Ala Met Thr Lys Ile Gln Ile Glu Gly Asp Pro Asp Phe Arg Leu
           675                 680                 685
   Ile Lys Glu Gly Glu Thr Val Thr Glu Val Ile His Gly Glu Pro Val
       690                 695                 700
   Ile Lys Lys Tyr Thr Lys Ile Ile Asp Gly Val Pro Val Glu Ile Thr
   705                 710                 715                 720
   Glu Lys Gln Thr Arg Glu Glu Arg Ile Ile Thr Gly Pro Glu Ile Lys
                   725                 730                 735
   Tyr Thr Arg Ile Ser Thr Gly Gly Gly Glu Thr Gly Glu Thr Leu Gln
               740                 745                 750
   Lys Phe Leu Gln Lys Glu Val Ser Lys Val Thr Lys Phe Ile Glu Gly
           755                 760                 765
   Gly Asp Gly His Leu Phe Glu Asp Glu Glu Ile Lys Arg Leu Leu Gln
       770                 775                 780
   Gly Asp Thr Pro Ala Lys Lys Ile Pro Ala Asn Lys Arg Val Gln Gly
   785                 790                 795                 800
   Pro Arg Arg Arg Ser Arg Glu Gly Arg Ser Gln
                   805                 810
<210> 11
   <211> 2436
   <212> DNA
   <213> Mus musculus
<400> 11
   atggttcctc tcctgccctt atatgctctg ctgctgctgt tcctgtgtga tattaaccct      60
   gcaaatgcca acagttacta tgacaaggtc ctggctcaca gccgcatcag gggtcgggat     120
   cagggcccaa acgtctgtgc cctccagcaa attctgggca ccaaaaagaa atacttcagc     180
   tcctgtaaga actggtatca aggtgctatc tgcgggaaga aaaccactgt gctatatgaa     240
   tgctgccctg gctatatgag aatggaaggg atgaaaggct gccccgcagt gatgcctatt     300
   gaccatgttt atggcacgct gggcattgtg ggagccacta ccactcagca ctactccgat     360
   gtctcgaagc tgagagaaga gattgaagga aaagggtcat acacgtactt cgcgccgagt     420
   aacgaggctt gggagaacct ggattctgac attcgcagag gactggagaa caatgtcaat     480
   gttgagctac tgaatgcctt acacagccac atggttaata agagaatgtt aaccaaggac     540
   ctgaaacacg gcatggttat tccttcaatg tacaacaatc tggggctttt tattaaccat     600
   tatcccaatg gggttgtcac tgtgaactgt gctcgagtca tccatgggaa ccagattgcc     660
   acaaatggtg tcgtccatgt cattgaccgt gtcctgacac aaattggtac ctccatccaa     720
   gacttccttg aagcagaaga cgacctttca tcatttagag cagccgccat cacctctgac     780
   ctcttggagt cccttggaag agatggtcac ttcacgctct ttgctcccac caatgaagct     840
   ttcgagaaac tgccacgagg tgtcctagaa aggatcatgg gagacaaagt ggcttctgaa     900
   gctctcatga agtaccacat cctaaatacc ctccagtgct ctgaggccat cactggagga     960
   gccgtgtttg agaccatgga aggaaacact attgagatag ggtgcgaagg ggacagtatc    1020
   tccattaacg gaatcaagat ggtgaacaag aaagacattg tgactaagaa tggtgtcatc    1080
   cacctgattg atgaagtcct cattcctgat tctgccaaac aagttattga gctggctgga    1140
   aaacagcaaa ccactttcac cgacctggta gcccaattag gcttggcatc ctctctgaag    1200
   ccagatggag agtacacctt attagcacct gtgaacaatg cgttctctga tgacactctg    1260
   agcatggacc aacgccttct taagctaatt ctgcaaaatc acatattgaa agtaaaagtt    1320
   ggccttagcg acctctacaa tggacagata ctggaaacca ttggaggcaa acaactccga    1380
   gtctttgtgt atcggacggc tatctgcata gaaaactcat gcatggtgag aggaagcaag    1440
   cagggaagga atggtgccat tcacatattc cgagaaatca tccaaccagc agagaaatcc    1500
   ctgcacgaca agctgcggca agacaagcgc tttagcatct tcctcagcct ccttgaagct    1560
   gcagatttga aagatctcct gacacagccc ggagattgga ccttgtttgc accaaccaat    1620
   gatgccttca agggaatgac tagcgaagaa agggagcttc tgattgggga taaaaatgct    1680
   ctccaaaaca tcattcttta tcacctgacc ccaggggttt atattggaaa gggattcgaa    1740
   cccggagtca ctaatatcct gaagaccaca cagggaagca aaatctatct gaaaggagta   1800
   aacgaaacgc ttctagtgaa tgagttgaag tccaaagaat ctgacatcat gacgacaaat   1860
   ggtgtcatcc acgtcgtgga caaactcctc tatccagcag atattccagt tggaaatgat   1920
   cagctcttgg aattactgaa caaactgata aaatacatcc aaatcaagtt tgttcgtggc   1980
   agcaccttca aagaaatccc catgactgtc tatagacctg caatgacgaa gatccaaatt   2040
   gaaggtgatc ccgacttcag gctgattaaa gaaggcgaaa cggtgacaga agtgatccac   2100
   ggagagccag tcattaaaaa gtacaccaaa atcatagatg gagttcctgt tgaaataact   2160
   gaaaaacaga ctcgggaaga acgaatcatt acaggtcctg agataaaata taccaggatt   2220
   tccacaggag gtggagaaac aggagagacc ttgcagaaat tcttgcaaaa agaggtctcc   2280
   aaggtcacaa agttcattga aggtggcgat ggtcacttat ttgaagatga ggagattaaa   2340
   agactgcttc agggagacac acctgcaaag aagataccag ccaacaaaag ggttcaaggg   2400
   cctagaagac gatcaagaga aggccgttct cagtga                             2436
<210> 12
   <211> 2511
   <212> DNA
   <213> Homo sapiens
<400> 12
   atgattccct ttttacccat gttttctcta ctattgctgc ttattgttaa ccctataaac     60
   gccaacaatc attatgacaa gatcttggct catagtcgta tcaggggtcg ggaccaaggc    120
   ccaaatgtct gtgcccttca acagattttg ggcaccaaaa agaaatactt cagcacttgt    180
   aagaactggt ataaaaagtc catctgtgga cagaaaacga ctgttttata tgaatgttgc    240
   cctggttata tgagaatgga aggaatgaaa ggctgcccag cagttttgcc cattgaccat    300
   gtttatggca ctctgggcat cgtgggagcc accacaacgc agcgctattc tgacgcctca    360
   aaactgaggg aggagatcga gggaaaggga tccttcactt actttgcacc gagtaatgag    420
   gcttgggaca acttggattc tgatatccgt agaggtttgg agagcaacgt gaatgttgaa    480
   ttactgaatg ctttacatag tcacatgatt aataagagaa tgttgaccaa ggacttaaaa    540
   aatggcatga ttattccttc aatgtataac aatttggggc ttttcattaa ccattatcct    600
   aatggggttg tcactgttaa ttgtgctcga atcatccatg ggaaccagat tgcaacaaat    660
   ggtgttgtcc atgtcattga ccgtgtgctt acacaaattg gtacctcaat tcaagacttc    720
   attgaagcag aagatgacct ttcatctttt agagcagctg ccatcacatc ggacatattg    780
   gaggcccttg gaagagacgg tcacttcaca ctctttgctc ccaccaatga ggcttttgag    840
   aaacttccac gaggtgtcct agaaaggttc atgggagaca aagtggcttc cgaagctctt    900
   atgaagtacc acatcttaaa tactctccag tgttctgagt ctattatggg aggagcagtc    960
   tttgagacgc tggaaggaaa tacaattgag ataggatgtg acggtgacag tataacagta   1020
   aatggaatca aaatggtgaa caaaaaggat attgtgacaa ataatggtgt gatccatttg   1080
   attgatcagg tcctaattcc tgattctgcc aaacaagtta ttgagctggc tggaaaacag   1140
   caaaccacct tcacggatct tgtggcccaa ttaggcttgg catctgctct gaggccagat   1200
   ggagaataca ctttgctggc acctgtgaat aatgcatttt ctgatgatac tctcagcatg   1260
   gttcagcgcc tccttaaatt aattctgcag aatcacatat tgaaagtaaa agttggcctt   1320
   aatgagcttt acaacgggca aatactggaa accatcggag gcaaacagct cagagtcttc   1380
   gtatatcgta cagctgtctg cattgaaaat tcatgcatgg agaaagggag taagcaaggg   1440
   agaaacggtg cgattcacat attccgcgag atcatcaagc cagcagagaa atccctccat   1500
   gaaaagttaa aacaagataa gcgctttagc accttcctca gcctacttga agctgcagac   1560
   ttgaaagagc tcctgacaca acctggagac tggacattat ttgtgccaac caatgatgct   1620
   tttaagggaa tgactagtga agaaaaagaa attctgatac gggacaaaaa tgctcttcaa   1680
   aacatcattc tttatcacct gacaccagga gttttcattg gaaaaggatt tgaacctggt   1740
   gttactaaca ttttaaagac cacacaagga agcaaaatct ttctgaaaga agtaaatgat   1800
   acacttctgg tgaatgaatt gaaatcaaaa gaatctgaca tcatgacaac aaatggtgta   1860
   attcatgttg tagataaact cctctatcca gcagacacac ctgttggaaa tgatcaactg   1920
   ctggaaatac ttaataaatt aatcaaatac atccaaatta agtttgttcg tggtagcacc   1980
   ttcaaagaaa tccccgtgac tgtctataca actaaaatta taaccaaagt tgtggaacca   2040
   aaaattaaag tgattgaagg cagtcttcag cctattatca aaactgaagg acccacacta   2100
   acaaaagtca aaattgaagg tgaacctgaa ttcagactga ttaaagaagg tgaaacaata   2160
   actgaagtga tccatggaga gccaattatt aaaaaataca ccaaaatcat tgatggagtg   2220
   cctgtggaaa taactgaaaa agagacacga gaagaacgaa tcattacagg tcctgaaata   2280
   aaatacacta ggatttctac tggaggtgga gaaacagaag aaactctgaa gaaattgtta   2340
   caagaagagg tcaccaaggt caccaaattc attgaaggtg gtgatggtca tttatttgaa   2400
   gatgaagaaa ttaaaagact gcttcaggga gacacacccg tgaggaagtt gcaagccaac   2460
   aaaaaagttc aaggttctag aagacgatta agggaaggtc gttctcagtg a            2511
<210> 13
   <211> 809
   <212> PRT
   <213> Homo sapiens
<400> 13
   Met Ile Pro Phe Leu Pro Met Phe Ser Leu Leu Leu Leu Leu Ile Val
   1               5                   10                  15
   Asn Pro Ile Asn Ala Asn Asn His Tyr Asp Lys Ile Leu Ala His Ser
               20                  25                  30
   Arg Ile Arg Gly Arg Asp Gln Gly Pro Asn Val Cys Ala Leu Gln Gln
           35                  40                  45
   Ile Leu Gly Thr Lys Lys Lys Tyr Phe Ser Thr Cys Lys Asn Trp Tyr
       50                  55                  60
   Lys Lys Ser Ile Cys Gly Gln Lys Thr Thr Val Leu Tyr Glu Cys Cys
   65                  70                  75                  80
   Pro Gly Tyr Met Arg Met Glu Gly Met Lys Gly Cys Pro Ala Val Leu
                   85                  90                  95
   Pro Ile Asp His Val Tyr Gly Thr Leu Gly Ile Val Gly Ala Thr Thr
               100                 105                 110
   Thr Gln Arg Tyr Ser Asp Ala Ser Lys Leu Arg Glu Glu Ile Glu Gly
           115                 120                 125
   Lys Gly Ser Phe Thr Tyr Phe Ala Pro Ser Asn Glu Ala Trp Asp Asn
       130                 135                 140
   Leu Asp Ser Asp Ile Arg Arg Gly Leu Glu Ser Asn Val Asn Val Glu
   145                 150                 155                 160
   Leu Leu Asn Ala Leu His Ser His Met Ile Asn Lys Arg Met Leu Thr
                   165                 170                 175
   Lys Asp Leu Lys Asn Gly Met Ile Ile Pro Ser Met Tyr Asn Asn Leu
               180                 185                 190
   Gly Leu Phe Ile Asn His Tyr Pro Asn Gly Val Val Thr Val Asn Cys
           195                 200                 205
   Ala Arg Ile Ile His Gly Asn Gln Ile Ala Thr Asn Gly Val Val His
       210                 215                 220
   Val Ile Asp Arg Val Leu Thr Gln Ile Gly Thr Ser Ile Gln Asp Phe
   225                 230                 235                 240
   Ile Glu Ala Glu Asp Asp Leu Ser Ser Phe Arg Ala Ala Ala Ile Thr
                   245                 250                 255
   Ser Asp Ile Leu Glu Ala Leu Gly Arg Asp Gly His Phe Thr Leu Phe
               260                 265                 270
   Ala Pro Thr Asn Glu Ala Phe Glu Lys Leu Pro Arg Gly Val Leu Glu
           275                 280                 285
   Arg Phe Met Gly Asp Lys Val Ala Ser Glu Ala Leu Met Lys Tyr His
       290                 295                 300
   Ile Leu Asn Thr Leu Gln Cys Ser Glu Ser Ile Met Gly Gly Ala Val
   305                 310                 315                 320
   Phe Glu Thr Leu Glu Gly Asn Thr Ile Glu Ile Gly Cys Asp Gly Asp
                   325                 330                 335
   Ser Ile Thr Val Asn Gly Ile Lys Met Val Asn Lys Lys Asp Ile Val
               340                 345                 350
   Thr Asn Asn Gly Val Ile His Leu Ile Asp Gln Val Leu Ile Pro Asp
           355                 360                 365
   Ser Ala Lys Gln Val Ile Glu Leu Ala Gly Lys Gln Gln Thr Thr Phe
       370                 375                 380
   Thr Asp Leu Val Ala Gln Leu Gly Leu Ala Ser Ala Leu Arg Pro Asp
   385                 390                 395                 400
   Gly Glu Tyr Thr Leu Leu Ala Pro Val Asn Asn Ala Phe Ser Asp Asp
                   405                 410                 415
   Thr Leu Ser Met Val Gln Arg Leu Leu Lys Leu Ile Leu Gln Asn His
               420                 425                 430
   Ile Leu Lys Val Lys Val Gly Leu Asn Glu Leu Tyr Asn Gly Gln Ile
           435                 440                 445
   Leu Glu Thr Ile Gly Gly Lys Gln Leu Arg Val Phe Val Tyr Arg Thr
       450                 455                 460
   Ala Val Cys Ile Glu Asn Ser Cys Met Glu Lys Gly Ser Lys Gln Gly
   465                 470                 475                 480
   Arg Asn Gly Ala Ile His Ile Phe Arg Glu Ile Ile Lys Pro Ala Glu
                   485                 490                 495
   Lys Ser Leu His Glu Lys Leu Lys Gln Asp Lys Arg Phe Ser Thr Phe
               500                 505                 510
   Leu Ser Leu Leu Glu Ala Ala Asp Leu Lys Glu Leu Leu Thr Gln Pro
           515                 520                 525
   Gly Asp Trp Thr Leu Phe Val Pro Thr Asn Asp Ala Phe Lys Gly Met
       530                 535                 540
   Thr Ser Glu Glu Lys Glu Ile Leu Ile Arg Asp Lys Asn Ala Leu Gln
   545                 550                 555                 560
   Asn Ile Ile Leu Tyr His Leu Thr Pro Gly Val Phe Ile Gly Lys Gly
                   565                 570                 575
   Phe Glu Pro Gly Val Thr Asn Ile Leu Lys Thr Thr Gln Gly Ser Lys
               580                 585                 590
   Ile Phe Leu Lys Glu Val Asn Asp Thr Leu Leu Val Asn Glu Leu Lys
           595                 600                 605
   Ser Lys Glu Ser Asp Ile Met Thr Thr Asn Gly Val Ile His Val Val
       610                 615                 620
   Asp Lys Leu Leu Tyr Pro Ala Asp Thr Pro Val Gly Asn Asp Gln Leu
   625                 630                 635                 640
   Leu Glu Ile Leu Asn Lys Leu Ile Lys Tyr Ile Gln Ile Lys Phe Val
                   645                 650                 655
   Arg Gly Ser Thr Phe Lys Glu Ile Pro Val Thr Val Tyr Arg Pro Thr
               660                 665                 670
   Leu Thr Lys Val Lys Ile Glu Gly Glu Pro Glu Phe Arg Leu Ile Lys
           675                 680                 685
   Glu Gly Glu Thr Ile Thr Glu Val Ile His Gly Glu Pro Ile Ile Lys
       690                 695                 700
   Lys Tyr Thr Lys Ile Ile Asp Gly Val Pro Val Glu Ile Thr Glu Lys
   705                 710                 715                 720
   Glu Thr Arg Glu Glu Arg Ile Ile Thr Gly Pro Glu Ile Lys Tyr Thr
                   725                 730                 735
   Arg Ile Ser Thr Gly Gly Gly Glu Thr Glu Glu Thr Leu Lys Lys Leu
               740                 745                 750
   Leu Gln Glu Glu Val Thr Lys Val Thr Lys Phe Ile Glu Gly Gly Asp
           755                 760                 765
   Gly His Leu Phe Glu Asp Glu Glu Ile Lys Arg Leu Leu Gln Gly Asp
       770                 775                 780
   Thr Pro Val Arg Lys Leu Gln Ala Asn Lys Lys Val Gln Gly Ser Arg
   785                 790                 795                 800
   Arg Arg Leu Arg Glu Gly Arg Ser Gln
                   805
<210> 14
   <211> 2430
   <212> DNA
   <213> Homo sapiens
<400> 14
   atgattccct ttttacccat gttttctcta ctattgctgc ttattgttaa ccctataaac      60
   gccaacaatc attatgacaa gatcttggct catagtcgta tcaggggtcg ggaccaaggc     120
   ccaaatgtct gtgcccttca acagattttg ggcaccaaaa agaaatactt cagcacttgt     180
   aagaactggt ataaaaagtc catctgtgga cagaaaacga ctgttttata tgaatgttgc     240
   cctggttata tgagaatgga aggaatgaaa ggctgcccag cagttttgcc cattgaccat     300
   gtttatggca ctctgggcat cgtgggagcc accacaacgc agcgctattc tgacgcctca     360
   aaactgaggg aggagatcga gggaaaggga tccttcactt actttgcacc gagtaatgag     420
   gcttgggaca acttggattc tgatatccgt agaggtttgg agagcaacgt gaatgttgaa     480
   ttactgaatg ctttacatag tcacatgatt aataagagaa tgttgaccaa ggacttaaaa     540
   aatggcatga ttattccttc aatgtataac aatttggggc ttttcattaa ccattatcct     600
   aatggggttg tcactgttaa ttgtgctcga atcatccatg ggaaccagat tgcaacaaat     660
   ggtgttgtcc atgtcattga ccgtgtgctt acacaaattg gtacctcaat tcaagacttc     720
   attgaagcag aagatgacct ttcatctttt agagcagctg ccatcacatc ggacatattg     780
   gaggcccttg gaagagacgg tcacttcaca ctctttgctc ccaccaatga ggcttttgag     840
   aaacttccac gaggtgtcct agaaaggttc atgggagaca aagtggcttc cgaagctctt     900
   atgaagtacc acatcttaaa tactctccag tgttctgagt ctattatggg aggagcagtc     960
   tttgagacgc tggaaggaaa tacaattgag ataggatgtg acggtgacag tataacagta    1020
   aatggaatca aaatggtgaa caaaaaggat attgtgacaa ataatggtgt gatccatttg   1080
   attgatcagg tcctaattcc tgattctgcc aaacaagtta ttgagctggc tggaaaacag   1140
   caaaccacct tcacggatct tgtggcccaa ttaggcttgg catctgctct gaggccagat   1200
   ggagaataca ctttgctggc acctgtgaat aatgcatttt ctgatgatac tctcagcatg   1260
   gttcagcgcc tccttaaatt aattctgcag aatcacatat tgaaagtaaa agttggcctt   1320
   aatgagcttt acaacgggca aatactggaa accatcggag gcaaacagct cagagtcttc   1380
   gtatatcgta cagctgtctg cattgaaaat tcatgcatgg agaaagggag taagcaaggg   1440
   agaaacggtg cgattcacat attccgcgag atcatcaagc cagcagagaa atccctccat   1500
   gaaaagttaa aacaagataa gcgctttagc accttcctca gcctacttga agctgcagac   1560
   ttgaaagagc tcctgacaca acctggagac tggacattat ttgtgccaac caatgatgct   1620
   tttaagggaa tgactagtga agaaaaagaa attctgatac gggacaaaaa tgctcttcaa   1680
   aacatcattc tttatcacct gacaccagga gttttcattg gaaaaggatt tgaacctggt   1740
   gttactaaca ttttaaagac cacacaagga agcaaaatct ttctgaaaga agtaaatgat   1800
   acacttctgg tgaatgaatt gaaatcaaaa gaatctgaca tcatgacaac aaatggtgta   1860
   attcatgttg tagataaact cctctatcca gcagacacac ctgttggaaa tgatcaactg   1920
   ctggaaatac ttaataaatt aatcaaatac atccaaatta agtttgttcg tggtagcacc   1980
   ttcaaagaaa tccccgtgac tgtctataga cccacactaa caaaagtcaa aattgaaggt   2040
   gaacctgaat tcagactgat taaagaaggt gaaacaataa ctgaagtgat ccatggagag   2100
   ccaattatta aaaaatacac caaaatcatt gatggagtgc ctgtggaaat aactgaaaaa   2160
   gagacacgag aagaacgaat cattacaggt cctgaaataa aatacactag gatttctact   2220
   ggaggtggag aaacagaaga aactctgaag aaattgttac aagaagaggt caccaaggtc   2280
   accaaattca ttgaaggtgg tgatggtcat ttatttgaag atgaagaaat taaaagactg   2340
   cttcagggag acacacccgt gaggaagttg caagccaaca aaaaagttca aggttctaga   2400
   agacgattaa gggaaggtcg ttctcagtga                                    2430
<210> 15
   <211> 23
   <212> DNA
   <213> Artificial
<220>
   <223> Primer No.1
<400> 15
   gttcattgaa ggtggcgatg gtc 23
<210> 16
   <211> 24
   <212> DNA
   <213> Artificial
<220>
   <223> Primer No.2
<400> 16
   gagataaaat ccctgcatgg tcct 24
<210> 17
   <211> 25
   <212> DNA
   <213> Artificial
<220>
   <223> Primer No. 3
<400> 17
   cacggtcgat gacatggaca acacc 25
<210> 18
   <211> 22
   <212> DNA
   <213> Artificial
<220>
   <223> Primer No. 4
<400> 18
   acggagctca gggctgaaga tg 22
<210> 19
   <211> 22
   <212> DNA
   <213> Artificial
<220>
   <223> Primer No. 5
<400> 19
   gacccgggaa gaacgcatca tc 22
<210> 20
   <211> 32
   <212> DNA
   <213> Artificial
<220>
   <223> Primer No. 6
<400> 20
   tgggtgaccc tgagaacggc cttctcttga tc 32
<210> 21
   <211> 27
   <212> PRT
   <213> Mus musculus
<400> 21
   Thr Thr Lys Ile Ile Thr Lys Val Val Glu Pro Lys Ile Lys Val Ile
   1               5                   10                  15
   Glu Gly Ser Leu Gln Pro Ile Ile Lys Thr Glu
               20                  25
<210> 22
   <211> 9
   <212> PRT
   <213> Homo sapiens
<400> 22
   Thr Thr Lys Ile Ile Thr Lys Val Val
   1               5
<210> 23
   <211> 9
   <212> PRT
   <213> Rattus sp.
<400> 23
   Thr Thr Lys Ile Ile Thr Lys Leu Val
   1               5
<210> 24
   <211> 9
   <212> PRT
   <213> Mus musculus
<400> 24
   Thr Thr Lys Ile Ile Thr Lys Val Val
   1               5
<210> 25
   <211> 28
   <212> PRT
   <213> Homo sapiens
<400> 25
   Cys Thr Thr Lys Ile Ile Thr Lys Val Val Glu Pro Lys Ile Lys Val Ile
   1               5                   10                  15
   Glu Gly Ser Leu Gln Pro Ile Ile Lys Thr Glu
           20                  25
<210> 26
   <211> 10
   <212> PRT
   <213> Homo sapiens
<400> 26
   Tyr Thr Thr Lys Ile Ile Thr Lys Val Val
   1               5                   10
<210> 27
   <211> 32
   <212> DNA
   <213> Artificial
<220>
   <223> Primer No.7
<400> 15
   aagctagcca ccatgattcc ctttttaccc at 32
<210> 28
   <211> 24
   <212> DNA
   <213> Artificial
<220>
   <223> Primer No.8
<400> 20
   aactccacaa tttccctcat 20
<210> 29
   <211> 21
   <212> DNA
   <213> Artificial
<220>
   <223> Primer No. 9
<400> 29
   taaccaaagt tgtggaacca a 21
<210> 30
   <211> 20
   <212> DNA
   <213> Artificial
<220>
   <223> Primer No. 10
<400> 30
   tgtgtctccc tgaagcagtc 20
<210> 31
   <211> 21
   <212> DNA
   <213> Artificial
<220>
   <223> Primer No. 11
<400> 31
   catcaccatc accatcacta a 21
<210> 32
   <211> 56
   <212> DNA
   <213> Artificial
<220>
   <223> Primer No. 12
<400> 32
   ctagaagacg attaagggaa ggtcgttctc agctggaagt tctgttccag gggccc 56
<210> 33
   <211> 52
   <212> DNA
   <213> Artificial
<220>
   <223> Primer No. 13
<400> 33
   gggcccctgg aacagaactt ccagctgaga acgaccttcc cttaatcgtc tt 52
<210> 34
   <211> 6
   <212> PRT
   <213> Homo sapiens
<400> 34
   Thr Thr Lys Ile Ile Thr
   1               5
<210> 35
   <211> 27
   <212> DNA
   <213> Artificial
<220>
   <223> Primer
<400> 35
   taaaattata accaaagttg tggaacc 27
<210> 36
   <211> 23
   <212> DNA
   <213> Artificial
<220>
   <223> Primer
<400> 36
   agtgtgggtc cttcagtttt gat 23

## Description

### TECHNICAL FIELD

The present invention relates to a pharmaceutical composition for cancer treatment, which comprises an antibody against a peptide region encoded by Exon-17 of periostin. More specifically, the present invention relates to a pharmaceutical composition for cancer treatment, which comprises an anti-periostin antibody recognizing a splice variant of periostin having anti-cell adhesive properties specifically expressed in interstitial tissue during tissue restructuring such as cancer tissues, or a method and reagent for cancer diagnosis using the above anti-periostin antibody.

### BACKGROUND ART

In recent years, the healing rate of cancer has been rising steadily with advances in cancer therapy. In particular, the improved success rate of primary carcinoma removal by surgical operation, radiation therapy or chemotherapy contributes to the advance of cancer therapy. However, on a worldwide basis, the cancer mortality rate continues to increase for reasons such as aging population, and cancer remains the primary cause of death. This is because not a few patients will die of cancer metastasis even when primary carcinoma removal is completely achieved, and there is a limit to surgical operation, radiation therapy or chemotherapy for completely blocking cancer metastasis, so that the distant metastasis of cancer is still directly or indirectly related to the cause of cancer death. Cancer metastasis is mediated by processes such as invasion of cancer cells released from their primary tumor into blood vessels or lymph vessels, selective migration of cancer cells to metastatic organs, invasion of cancer cells from blood vessels into metastatic organs, growth of cancer cells supported by the microenvironment where metastasis occurred, and angiogenesis-associated growth of tumors whose diameter exceeds several millimeters (Non-patent Document 1, Non-patent Document 2). Among these complex processes for metastasis establishment, invasion and metastasis induced by the enhanced motility of cancer cells are very important stages (Non-patent Document 3). Until now, it has been reported that highly metastatic cancer cells produce an autocrine motility factor by themselves to enhance their own motion (Non-patent Document 4). Inhibitory substances against this malignant factor are expected as metastasis inhibitors, but no specific inhibitor has been found at present.

Periostin is an extracellular matrix protein and consists of a polypeptide having a molecular weight of about 90000. Each polypeptide chain has a signal sequence, a cysteine-rich domain, a fourfold repeated domain, and a C-terminal domain.

Periostin was first called osteoblast-specific factor-2 (OSF-2) and was isolated and identified as a gene specifically expressed in the mouse osteoblast cell line MC3T3-E1 (Patent Document 1, Non-patent Document 5), and later came to be known as periostin and was reported to have adhesion-promoting activity in osteoblast cells (Non-patent Document 6).

In early studies, periostin was thought to be an extracellular matrix specifically expressed in bone tissue. However, it is currently known to be expressed not only in bone tissue but also very highly at the onset of heart failure (Non-patent Document 7, Non-patent Document 8), aneurysms (Non-patent Document 9), highly metastatic cancers (Non-patent Document 10, Non-patent Document 11, Non-patent Document 12), preeclampsia (Non-patent Document 13) as well as very slightly in normal tissue. Moreover, some periostin splice variants were shown to be expressed in osteoblasts (Non-patent Document 5, Non-patent Document 6, Non-patent Document 14, Patent Document 2).

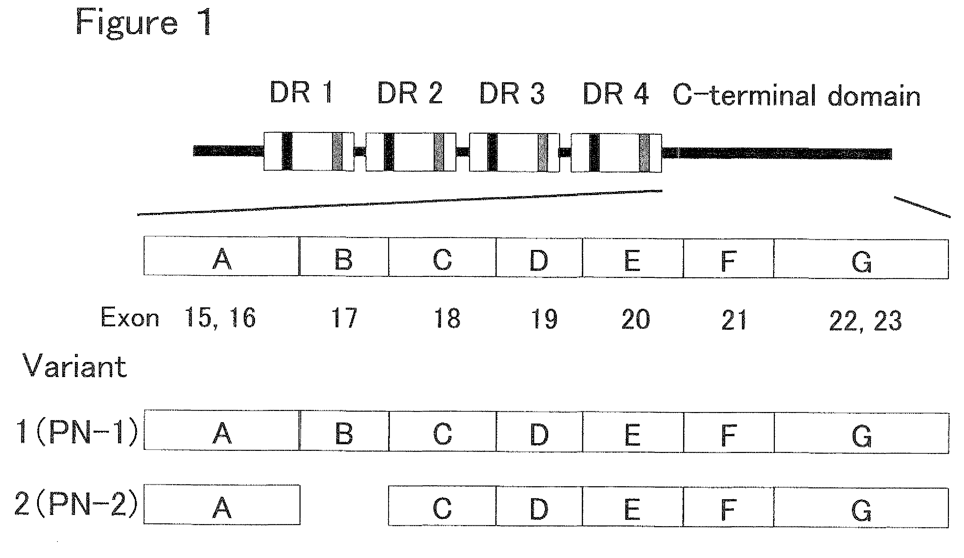

As to functions of periostin, a periostin splice variant consisting of 811 amino acids (corresponding to PN-2 in Figure 1) (Non-patent Document 6) and a periostin splice variant consisting of 782 amino acids (Non-patent Document 15) were reported to have cell adhesive properties. In contrast, it has been reported that a periostin splice variant consisting of 838 amino acids (corresponding to PN-1 in Figure 1) prevents heart fibroblasts from adhering to a plate coated with the periostin splice variant, i.e., has no cell adhesive activity; the gene expression of the periostin splice variant consisting of 838 amino acids (corresponding to PN-1 in Figure 1) is significantly increased in heart failure model rats as compared with normal rats; this variant is an aggravating factor inducing heart dilation; and that the survival rate was significantly increased by inhibition of the expression of this protein (Non-patent Document 7). Further, there is a report of a prophylactic or therapeutic agent for heart failure, in which an antisense nucleotide against the periostin splice variant consisting of 838 amino acids (corresponding to PN-1 in Figure 1) is used to suppress expression of the periostin splice variant (Patent Document 3). In addition, the inventors of the present invention have reported a prophylactic or therapeutic agent for heart failure, which is based on the following findings: the periostin splice variant consisting of 811 amino acids (corresponding to PN-2 in Figure 1) is involved in cell adhesion whereas the periostin splice variant consisting of 838 amino acids (corresponding to PN-1 in Figure 1) has cell- detaching activity; an antibody against an antigen composed of the Exon-17 sequence inhibits the cell-detaching activity; and improved heart function was observed in acute myocardial infarction model animals (Japanese Patent Application No. 2005-380009).

As to cancers, various reports have been issued on high level expression of periostin in highly metastatic cancers [Non-patent Document 16 (pancreatic cancer), Non-patent Document 17 (oral cancer), Non-patent Document 18 (pancreatic cancer), Non-patent Document 19 (breast cancer), Non-patent Document 20 (head and neck cancer), Non-patent Document 21 (colon cancer), Non-patent Document 10 (breast cancer), Non-patent Document 12 (breast cancer), Non-patent Document 22 (thymic cancer), Non-patent Document 23 (non-small cell lung cancer), Non-patent Document 11 (head and neck squamous cell carcinoma)]. However, none of these documents focuses on splice variants of periostin. Although some reports use RT-PCR analysis to confirm periostin expression (Non-patent Documents 6, 11, 13, 14, 17, 18, 20, 26, 28 and 29), the primers used were prepared at different sites than the Exon-17 region, and none of these documents specifically analyzes variants having the Exon-17 region. Also, highly metastatic cancers are reported to express the transcription factor Twist at high level (Non-patent Document 24, Non-patent Document 25) and receive attention, but there is a report showing that Twist is also located in the promoter region of periostin (Non-patent Document 26). In addition, it has been reported that the human fetal kidney epithelial cell line 293T, which is carcinogenic and non-metastatic, enhances its invasion ability when introduced with the periostin gene (Non-patent Document 27). It has also been reported that a mouse homolog of rat periostin was less expressed in various cancer cells, introduction of the periostin gene into bladder cancer cells inhibited invasion of the bladder cancer cells, and introduction of the periostin gene into mouse melanoma B16-F10 cells inhibited their metastasis to lung (Non-patent Document 28).

As shown above, it has been suggested that expression of the periostin gene is related not only to the pathology of heart failure, but also to the pathology of cancer. However, it is unknown what function each splice variant has on the progress of cancer condition.

As to antibodies against periostin, there is a report of an anti-periostin antibody which inhibits periostin overexpression-enhanced migration of mesenchymal cells (Non-patent Document 29). This is a polyclonal antibody against an antigen composed of the N-terminal 123-141 amino acid sequence (KLREEIEGKGSYTYFAPSN) of mouse or rat periostin protein. There is also another report of an anti-periostin antibody which inhibits periostin-induced proliferation and cell differentiation in colorectal cancer (Non-patent Document 30). However, this document contains no information about periostin variants, and it is unclear to which variant the purified protein used as an antigen corresponds and it is also unclear which site the antibody recognizes.
Patent Document 1: JPA No. HEI-5-268982
Patent Document 2: International Publication WO2005/019471
Patent Document 3: Republication WO02/020055
Non-patent Document 1: Folkman J. Semin. Cancer Biol, 3, 65-71 (1992)
Non-patent Document 2: Hanahan D. et al., Cell, 86, 353-364 (1996)
Non-patent Document 3: Liotta LA. et al., Cell, 64, 327-336 (1991)
Non-patent Document 4: Liotta LA. et al., Proc. Natl. Acad. Sci., 83, 3302-3306 (1986)
Non-patent Document 5: Takeshita S. et al., Biochem J (1993) 294, 271-8
Non-patent Document 6: Horiuchi K. et al., J. Bone Miner. Res. (1999) 14, 1239-49
Non-patent Document 7: Katsuragi N. et al., Circulation (2004) 110, 1806-13
Non-patent Document 8: Wang D. et al., Hypertension (2003) 42, 88-95
Non-patent Document 9: Peters DG. et al., Stroke (2001) 32, 1036-42
Non-patent Document 10: Shao R. et al., Mol Cell Biol. (2004) 24, 3992-4003
Non-patent Document 11: Gonzalez HE. et al., Arch Otolaryngol Head Neck Surg. (2003) 129, 754-9
Non-patent Document 12: Sasaki H. et al., Breast Cancer Res Treat. (2003) 77, 245-52
Non-patent Document 13: Sasaki H. et al., Am J Obstet Gynecol. (2002) 186, 103-8
Non-patent Document 14: Litvin J. et al., J Cell Biochem. (2004) 92, 1044-61
Non-patent Document 15: Gillan L. et al., Cancer Res. (2002) 62, 5358-64
Non-patent Document 16: Erkan M. et al., Gastroenterology, 132(4), 1447-64 (2007)
Non-patent Document 17: Siriwardena BS. et al., Br J Cancer, 95(10), 1396-403 (2006)
Non-patent Document 18: Baril P. et al., Oncogene, 26(14), 2082-94 (2007)
Non-patent Document 19: Grigoriadis A. et al. Breast Cancer Res, 8(5), R56 (2006)
Non-patent Document 20: Kudo Y. et al., Cancer Res, 66(14), 6928-35 (2006)
Non-patent Document 21: Bao S. et al., Cancer Cell. 5(4), 329-39 (2004)
Non-patent Document 22: Sasaki H. et al., Cancer Lett., 72(1), 37-42 (2001)
Non-patent Document 23: Sasaki H. et al., Cancer, 92(4), 843-8 (2001)
Non-patent Document 24: Thiery JP. et al., Nat Med. 10(8), 777-8 (2004)
Non-patent Document 25: Yang J, et al., Cell. 117(7), 927-39 (2004)
Non-patent Document 26: Oshima A, et al., J Cell Biochem, 86(4), 792-804 (2002)
Non-patent Document 27: Yan W. et al., J. Biol. Chem., 281(28), 19700-8 (2006)
Non-patent Document 28: Kim CJ, et al., Int J Cancer, 117(1), 51-8 (2005)
Non-patent Document 29: Lindner V. et al., Arterioscler Thromb Vasc Biol. (2005) 25, 77-83
Non-patent Document 30: Tai IT. et al., Carcinogenesis (2005) 26, 908-15
Non-patent Document 31: Kim CJ, et al., Annual meeting of the Japan Cancer Association Kiji, Japan Cancer Association, Japan (2006) 65, ISSN: 0546-0476

### DISCLOSURE OF THE INVENTION

### PROBLEMS TO BE SOLVED BY THE INVENTION

The present invention aims to provide a novel cancer therapeutic agent which enables an improvement in the quality of life and an improvement in long-term vital prognosis and whose mechanism is different from that of existing agents. The present invention further aims to provide a method and reagent for cancer diagnosis.

### MEANS FOR SOLVING THE PROBLEMS

The inventors of the present invention have clarified that a periostin splice variant having no cell adhesive activity (PN-1) has anti-cell adhesive activity, i.e., the activity of detaching adhered cells. On the other hand, the inventors have also confirmed that periostin having cell adhesive activity (PN-2) has no anti-cell adhesive activity, i.e., does not detach adhered cells. Moreover, the inventors noted a difference in structure and cell adhesive activity between periostin splice variant having anti-cell adhesive activity (PN-1) and periostin showing no anti-cell adhesive activity (PN-2), and they have already demonstrated that diseases related to periostin isoforms having anti-cell adhesive activity could be prevented or treated by inhibiting a region specifically present in the periostin splice variants having anti-cell adhesive activity, i.e., the Exon-17 region (Japanese Patent Application No. 2005-380009).

Based on these findings, the inventors of the present invention deduced that the cell- detaching effect (i.e., cell-releasing effect) of PN-1 protein is related to the release of cancer cells from their primary tumor during the cancer metastasis processes mentioned above, and thereby facilitates cancer metastasis. Thus, they attempted to clarify the relationship between PN-1 protein and the pathology of cancer.

The C-terminal domains in which periostin splice variants are formed consist of exons 15 to 23; specifically rats have the following variants (1) to (4):
(1) a variant retaining all the exons (called PN-1; consisting of 838 amino acids shown as SEQ ID NO: 1; the cDNA sequence shown as SEQ ID NO: 6),
(2) a variant lacking Exon-17 (called PN-2; consisting of 811 amino acids shown as SEQ ID NO: 5; 27 amino acids (Exon-17) shown in SEQ ID NO: 3 are deleted from PN-1; the cDNA sequence shown as SEQ ID NO: 7),
(3) a variant lacking Exon-21 (called PN-3; consisting of 810 amino acids), and
(4) a variant lacking Exon-17 and Exon-21 (called PN-4; consisting of 783 amino acids).

In addition to rats, mouse and human PN-1 and PN-2 were also found (mouse PN-1: SEQ ID NO: 8 (amino acid sequence), SEQ ID NO: 9 (cDNA sequence); mouse PN-2: SEQ ID NO: 10 (amino acid sequence), SEQ ID NO: 11 (cDNA sequence); human PN-1: SEQ ID NO: 12 (cDNA sequence); human PN-2: SEQ ID NO: 13 (amino acid sequence), SEQ ID NO: 14 (cDNA sequence)).

Further, analysis of periostin splice variants highly expressed during the pathology of cancer revealed that PN-1 having the functions of inhibiting cell adhesion and detaching adhered cells is expressed at very high level in tumor tissues of model mice for lung metastasis of mouse melanoma B16-F10 cells or mouse 4T1 breast cancer cells when compared to normal tissues, thus showing that PN-1 is expressed at a very high level during the pathology of cancer (Examples 12 and 13 described later).

Likewise, in various human cancer tissues (esophageal cancer, lung cancer, thymic cancer, pancreatic cancer, thyroid cancer, gastric cancer, small intestinal cancer, colon (large bowel) cancer, rectal cancer, liver cancer, bladder cancer, kidney cancer, breast cancer, breast ductal cancer, mammary gland cancer, uterine cancer, uterine cervical cancer, ovarian cancer, testicular cancer, lymphoma, adrenal cancer, prostate cancer, osteogenic sarcoma, malignant melanoma, synovioma, leukemia), a variant having the Exon-17 region was also confirmed to be expressed (Example 16 described later).

Then, the inventors of the present invention considered that a site structurally different between PN-1 having the function of inhibiting cell adhesion and PN-2 having the function of allowing cell adhesion, i.e., the Exon-17 site that is found only in PN-1 is a region related to the inhibition of cell adhesion, and further considered that when inhibiting this Exon-17 site, it is possible to suppress the PN-1's inhibitory function on cell adhesion and further inhibit cancer metastasis. Thus, the inventors contemplated preparing an antibody specifically recognizing a peptide part consisting of an amino acid sequence encoded by Exon-17 as an inhibitor against that site, which site is found in PN-1.

In order to prepare an antibody, the material used as an immunogen must be hydrophilic, and if an antibody is to be prepared using a part of a large polypeptide such as protein, the part used as an immunogen must be exposed on the surface of the protein to form an epitope part. Thus, in order to examine the possibility of using the Exon-17 peptide chain as an antigen, an epitope search was initially performed using Accelrys software Mac Vector 7.2 that is widely used in the field of bioinformatics. It was judged from "Hydrophilicity", "Surface Probability" and "Antigenicity" that TTKIITKLVEPKIKVIQGSLQPIIKTE (SEQ ID NO: 3) of the Exon-17 region is mostly hydrophobic, suggesting that this region is very unlikely to be exposed on the surface of the protein molecule and has no immunogenicity, so it cannot be used to prepare an antibody, and therefore, it was presumed that it would be difficult to use to practically prepare an antibody.

However, when a peptide consisting of 27 amino acids constituting a peptide encoded by the Exon-17 region was synthesized and used to immunize rabbits, and the resulting serum was purified to give an IgG fraction, whereby an anti-Exon-17 peptide polyclonal antibody was prepared, it was surprisingly possible to prepare an antibody against a peptide region encoded by Exon-17 specifically found in PN-1 (hereinafter referred to as "anti-Exon-17 polyclonal antibody") although the peptide was presumed to be hydrophobic and non-immunogenic (Example 1 described later).

Next, when mouse melanoma B16-F10 cells or mouse 4T1 breast cancer cells were added to PN-1 protein-coated plates, cell adhesion did not occur, thus showing that rat PN-1 protein has the effect of preventing cells from adhering (i.e., non-cell adhesive properties) (Example 2 described later). Moreover, when PN-1 protein was added to an 80% confluent culture of mouse melanoma B16-F10 cells or mouse 4T1 breast cancer cells, nearly 100% cell elimination (i.e., anti-cell adhesive activity) was observed (Example 3 described later). Administration of the anti-Exon-17 polyclonal antibody to this experimental system inhibited the cell elimination mediated by PN-1 protein, showing that the anti-Exon-17 polyclonal antibody is an antibody having a neutralizing activity against PN-1 protein (Example 4 described later). Moreover, addition of the anti-Exon-17 polyclonal antibody to mouse melanoma B16-F10 cells or mouse 4T1 cells inhibited cell proliferation, showing that the anti-Exon-17 polyclonal antibody also has an inhibitory effect on proliferation of mouse melanoma B16-F10 cells or mouse 4T1 breast cancer cells (Example 5 described later).

When the anti-Exon-17 polyclonal antibody was then continued to be administered once a week to model mice which will develop lung metastasis when inoculated with mouse melanoma B16-F10 cells or mouse 4T1 breast cancer cells into their paws, this antibody significantly inhibited not only primary tumor growth, but also the metastasis rate and number of lung metastases, within 3 to 5 weeks after model preparation (Examples 14 and 15 described later). Moreover, in mouse 4T1 breast cancer cells, this antibody significantly inhibited bone invasion of the breast cancer cells from primary tumor and bone destruction caused thereby (Example 15 described later). This indicated that the anti-Exon-17 polyclonal antibody is an antibody having the activity of inhibiting primary tumor growth associated with the progress of cancer condition, the activity of inhibiting bone invasion, the activity of inhibiting bone destruction caused by cancer bone invasion, and the activity of inhibiting metastasis from primary tumor to lung. This suggested that a neutralizing antibody against PN-1 suppresses the adhesion-inhibiting effect of PN-1 and is useful as a novel therapeutic agent to inhibit the growth and lung metastasis of malignant melanoma or breast cancer cells, as well as bone invasion of breast cancer cells and bone destruction caused thereby. These findings showed that PN-1 has the function of causing the growth of primary tumor and metastasis from primary tumor during the pathology of cancer, leading to a finding that the progress of these cancer conditions can be inhibited by such an antibody against a peptide region encoded by Exon-17 specifically found in PN-1.

Furthermore, the inventors of the present invention prepared a monoclonal antibody against the human periostin Exon-17 peptide chain (hereinafter referred to as "anti-Exon-17 monoclonal antibody") (Example 6 described later). In experiments using model mice for lung metastasis of mouse melanoma B16-F10 cells, administration of the anti-Exon-17 monoclonal antibody showed cancer growth inhibition and inhibitory effects on the metastasis rate and the number of lung metastases (Example 14 described later).

These findings indicated that a periostin isoform having the Exon-17 region (PN-1), whose expression is increased in various cancer tissues, has the function of causing the growth of primary tumor and metastasis from primary tumor, and that administration of the antibody against a peptide region encoded by Exon-17 specifically found in PN-1 (i.e., anti-Exon-17 polyclonal antibody or anti-Exon-17 monoclonal antibody) inhibits the growth of primary tumor and metastasis from primary tumor, and thereby allows inhibition of the progress of cancer condition, leading to accomplishment of the present invention.

Namely, the present invention as claimed provides a pharmaceutical composition for use in a method of treatment of cancer, which comprises an antibody against periostin, particularly an antibody against a peptide region encoded by Exon-17.

Namely, the present invention includes the following aspects.
(1) A pharmaceutical composition for use in a method of treatment of cancer, which comprises an antibody against a peptide consisting of any one amino acid sequence selected from the group consisting of SEQ ID NO: 3, SEQ ID NO: 4 and SEQ ID NO: 21.
(2) The pharmaceutical composition for cancer treatment as defined in (1), wherein the antibody specifically binds to a site consisting of an amino acid sequence of SEQ ID NO: 26.
(3) The pharmaceutical composition for cancer treatment as defined in (1) or (2), wherein the antibody specifically recognizes a site consisting of an amino acid sequence of SEQ ID NO: 34.
(4) The pharmaceutical composition for cancer treatment as defined in any one of (1) to (3), wherein the cancer treatment is to inhibit cancer metastasis.
(5) The pharmaceutical composition for cancer treatment as defined in any one of (1) to (3), wherein the cancer treatment is to inhibit the growth of primary tumor.
(6) The pharmaceutical composition for cancer treatment as defined in any one of (1) to (3), wherein the cancer treatment is to inhibit cancer bone invasion and bone destruction caused thereby.
(7) The pharmaceutical composition for cancer treatment as defined in any one of (1) to (6), wherein the cancer is esophageal cancer, lung cancer, thymic cancer, pancreatic cancer, thyroid cancer, gastric cancer, small intestinal cancer, colon (large bowel) cancer, rectal cancer, liver cancer, bladder cancer, kidney cancer, breast cancer, breast ductal cancer, mammary gland cancer, uterine cancer, uterine cervical cancer, ovarian cancer, testicular cancer, lymphoma, adrenal cancer, prostate cancer, osteogenic sarcoma, malignant melanoma, synovioma, or leukemia.
(8) The pharmaceutical composition for cancer treatment as defined in any one of (1) to (6), wherein the cancer is malignant melanoma or breast cancer.
(9) An ex vivo method for cancer diagnosis, which comprises the step of measuring the amount of a periostin isoform having a peptide region encoded by Exon-17 in a biological sample by using an antibody against a peptide consisting of any one amino acid sequence selected from the group consisting of SEQ ID NO: 3, SEQ ID NO: 4 and SEQ ID NO: 21.
(10) A reagent for use in an in vivo method of diagnosis of cancer, which comprises an antibody against a peptide consisting of any one amino acid sequence selected from the group consisting of SEQ ID NO: 3, SEQ ID NO: 4 and SEQ ID NO: 21.
(11) An ex vivo method for cancer diagnosis, which comprises the step of measuring the amount of a periostin isoform having a peptide region encoded by Exon-17 in a biological sample by using PCR primers consisting of a nucleotide having sequence of SEQ ID NO: 35 and a nucleotide having sequence of SEQ ID NO: 36.
(12) A reagent for use in an in vivo method of diagnosis of cancer, which comprises PCR primers consisting of a nucleotide having sequence of SEQ ID NO: 35 and a nucleotide having sequence of SEQ ID NO: 36.

### BRIEF DESCRIPTION OF THE DRAWINGS

Figure 1 is a schematic diagram showing mouse periostin splice variants.
Figure 2A is a diagram showing assay results of the non-cell adhesive properties of rat PN-1 protein in mouse melanoma B16-F10 cells (Example 2).
Figure 2B is a diagram showing assay results of the non-cell adhesive properties of rat PN-1 protein in mouse 4T1 breast cancer cells (Example 2).
Figure 3A is a diagram showing assay results of the anti-cell adhesive properties of rat PN-1 protein in mouse melanoma B16-F10 cells (Example 3).
Figure 3B is a diagram showing assay results of the anti-cell adhesive properties of rat PN-1 protein in mouse 4T1 breast cancer cells (Example 3).
Figure 4A is a diagram showing assay results of the inhibition of rat PN-1 protein activity by anti-rat Exon-17 polyclonal antibody in mouse melanoma B16-F10 cells (Example 4).
Figure 4B is a diagram showing assay results of the inhibition of rat PN-1 protein activity by anti-rat Exon-17 polyclonal antibody in mouse 4T1 breast cancer cells (Example 4).
Figure 5A is a diagram showing assay results of the inhibition of cell proliferation in mouse melanoma B16-F10 cells by anti-rat Exon-17 polyclonal antibody (Example 5).
Figure 5B is a diagram showing assay results of the inhibition of cell proliferation in mouse 4T1 breast cancer cells by anti-rat Exon-17 polyclonal antibody (Example 5).
Figure 6A is a diagram showing the expression of a periostin isoform having a peptide region encoded by Exon-17 in primary tumor inoculated with mouse melanoma B16-F10 cells and in normal tissue (Example 12).
Figure 6B is a diagram showing the expression of a periostin isoform having a peptide region encoded by Exon-17 in primary tumor inoculated with mouse 4T1 breast cancer cells and in normal tissue (Example 13).
Figure 7 is a diagram showing assay results of the effect of anti-human Exon-17 monoclonal antibodies (No. 1 and No. 3) by using model mice for lung metastasis of mouse melanoma B16-F10 cells (Example 14).
Figure 8 is a diagram showing assay results of the effect of anti-rat Exon-17 polyclonal antibody by using model mice for lung metastasis of mouse melanoma B16-F10 cells (Example 14: % increase in primary tumor size, lung metastasis rate, number of lung metastases).
Figure 9 is a diagram showing assay results of the effect of anti-human Exon-17 monoclonal antibody (No. 3) by using model mice for lung metastasis of mouse melanoma B16-F10 cells (Example 14: % increase in primary tumor size, lung metastasis rate, number of lung metastases).
Figure 10 is a diagram showing assay results of the effect of anti-rat Exon-17 polyclonal antibody by using model mice for lung metastasis of mouse 4T1 breast cancer cells (Example 15: body weight change).
Figure 11 is a diagram showing assay results of the effect of anti-rat Exon-17 polyclonal antibody by using model mice for lung metastasis of mouse 4T1 breast cancer cells (Example 15: tumor volume change in primary tumor).
Figure 12 is a diagram showing assay results of the effect of anti-rat Exon-17 polyclonal antibody by using model mice for lung metastasis of mouse 4T1 breast cancer cells (Example 15: comparison of bone area in bone destruction caused by bone invasion).
Figure 13 is a diagram showing assay results of the effect of anti-rat Exon-17 polyclonal antibody by using model mice for lung metastasis of mouse 4T1 breast cancer cells (Example 15: comparison of osteoclast counts in bone destruction caused by bone invasion).
Figure 14 is a diagram showing assay results of the effect of anti-rat Exon-17 polyclonal antibody by using model mice for lung metastasis of mouse 4T1 breast cancer cells (Example 15: number of lung metastases at 3 weeks after inoculation of mouse 4T1 breast cancer cells).
Figure 15 is a diagram showing the expression of a variant having the Exon-17 region, confirmed in human cancer cell lines purchased from ATCC and ECACC.
Figure 16 is a diagram showing the expression of a variant having the Exon-17 region, confirmed in human cancer cell line total RNAs purchased from Ambion.
Figure 17 is a diagram showing the expression of a variant having the Exon-17 region, confirmed in human cancer tissue-derived total RNAs purchased from Ambion.
Figure 18 is a diagram showing the expression of a variant having the Exon-17 region, confirmed in human cancer tissue-derived total RNAs purchased from Biochain.
Figure 19 is a diagram showing the expression of a variant having the Exon-17 region, confirmed in human normal tissue-derived total RNAs purchased from CLONTECH and COSMO BIO.

### BEST MODE FOR CARRYING OUT THE INVENTION

### 1. Pharmaceutical composition for cancer treatment

The present invention as claimed is directed to a pharmaceutical composition for cancer treatment, which comprises an anti-periostin antibody recognizing a periostin splice variant having anti-cell adhesive properties (PN-1 protein).

The pharmaceutical composition of the present invention can be used for cancer treatment or diagnosis. Cancers to be treated or diagnosed by the present invention include, but not limited to, malignant melanoma, as well as cancers of breast, large bowel, lung, bone, pancreas, stomach, skin, uterus, ovary, rectum, colon, fallopian tube, esophagus, small intestine, thyroid, parathyroid, adrenal gland, prostate, bladder, kidney, thymus, pancreas, liver, breast duct, mammary gland, uterine cervix, ovary and testis, as well as lymphoma, osteogenic sarcoma, synovioma and leukemia. The pharmaceutical composition of the present invention can be particularly applied to highly metastatic cancers such as malignant melanoma, breast cancer, large bowel cancer or lung cancer.

The pharmaceutical composition of the present invention has inhibitory effects on the growth of primary tumor, on bone invasion of cancer cells and bone destruction caused thereby, and on cancer metastasis. Thus, cancer treatment can be achieved by inhibiting the growth of primary tumor or by inhibiting bone invasion of cancer cells or bone destruction caused thereby or cancer metastasis.

The pharmaceutical composition of the present invention may be prepared using carriers and/or excipients or other additives, which are used in standard formulation techniques.

The antibody serving as an active ingredient in the pharmaceutical composition of the present invention is preferably administered in admixture with known pharmacologically acceptable carriers, excipients, diluents or the like by any administration mode commonly used for pharmaceutical preparations, especially antibody drugs, for example, via the intravenous, subcutaneous, intracutaneous, intramuscular, intraperitoneal or oral route.

The pharmaceutical composition of the present invention can be prepared by appropriately mixing the active ingredient with physiologically acceptable carriers, flavors, excipients, stabilizers, diluents, emulsifiers, solutions, suspensions, syrups or the like, and can be used in the form of tablets, powders, granules, solutions or the like. Additives which can be incorporated into tablets or the like include, for example, binders such as gelatin and lubricants such as corn starch. Tablets may also be coated with a sugar coating or a gastric or enteric film. In the dosage form of capsules, the above composition can further comprise liquid carriers. Injectable sterile compositions can also be prepared by applying standard formulae. Injectable aqueous vehicles include isotonic solutions containing glucose and the like, which may be used in combination with appropriate solubilizers such as polyethylene glycol, etc. The compositions may also be incorporated with buffers, stabilizers, preservatives, antioxidants, soothing agents and the like. For oral administration, when the active ingredient is likely to be decomposed in the digestive tract, the composition may be administered orally as a formulation that is resistant to decomposition in the digestive tract, for example, as microcapsules encapsulating the active ingredient within liposomes. It is also possible to use other administration modes intended for absorption through mucous membranes other than the digestive tract, including rectal, intranasal, sublingual and transpulmonary routes. In this case, the composition can be administered in the form of suppositories, nose drops, sublingual tablets, transpulmonary agents or the like.

When the pharmaceutical composition of the present invention is used for therapeutic purposes, its dosage is set at a therapeutically effective dosage, which varies depending on, e.g., an age and a body weight of a subject to which the composition is to be administered, a severity of symptoms and a route of administration, and thus administration is determined on an individual basis. In general, the daily adult dosage for oral administration is about 0.1 to 1000 mg, given as a single dose or in divided doses. For continuous intravenous administration, the composition can be administered in the range of 0.01 µg/kg/min to 1.0 µg/kg/min, desirably 0.025 µg/kg/min to 0.1 µg/kg/min.

### 2. Antibody

Antibodies for use in the present disclosure are those against a region specifically found in PN-1 protein.

In the specification, PN-1 protein includes periostin isoforms consisting of an amino acid sequence of SEQ ID NO: 1 (rat periostin PN-1, 838 amino acids), SEQ ID NO: 2 (human periostin PN-1, 836 amino acids having an N-terminal signal sequence shorter by 2 amino acids than that of rat periostin), or SEQ ID NO: 8 (mouse periostin PN-1).

Examples of such a region specifically found in PN-1 protein include the splice site of Exon-17, especially a peptide region encoded by Exon-17. Specific examples include a peptide part shown in SEQ ID NO: 3 of a periostin isoform having an amino acid sequence shown in SEQ ID NO: 1 (672-698 amino acids of SEQ ID NO: 1), a peptide part shown in SEQ ID NO: 4 of a periostin isoform having an amino acid sequence shown in SEQ ID NO: 2 (670-696 amino acids of SEQ ID NO: 2), and a peptide part shown in SEQ ID NO: 21 of a periostin isoform having an amino acid sequence shown in SEQ ID NO: 8 (672-698 amino acids of SEQ ID NO: 8).

In a preferred embodiment of the present invention, antibodies against the peptide encoded by Exon-17 include those specifically binding to a peptide consisting of an amino acid sequence covering from the -1st tyrosine to the 9th valine (SEQ ID NO: 26) from the N-terminus of the amino acid sequence of the peptide encoded by Exon-17. Moreover, preferred are those specifically recognizing a peptide consisting of an amino acid sequence covering from the 1st threonine to the 6th threonine (SEQ ID NO: 34) from the N-terminus of the amino acid sequence of the peptide encoded by Exon-17.

In an embodiment, antibodies for use in the present invention are monoclonal and polyclonal antibodies which are prepared using the above peptide regions encoded by Exon-17 as antigens. The "monoclonal antibodies" here refer to any monoclonal antibody showing reactivity against the antigens described above, and the "monoclonal antibodies" include natural antibodies obtained by immunizing mammals such as mice, rats, hamsters, guinea pigs or rabbits with the antigens, chimeric monoclonal antibodies (chimeric antibodies) and humanized monoclonal antibodies (humanized antibodies; CDR-grafted antibodies) that can be prepared by using genetic recombination techniques, as well as human monoclonal antibodies (human antibodies) that can be prepared by using human antibody-producing transgenic animals or the like. Antibodies for use in the present invention include monoclonal antibodies having any isotype such as IgG (IgG1, IgG2, IgG3, IgG4), IgM, IgA, IgD or IgE, preferably IgG (IgG1, IgG2, IgG3, IgG4) or IgM.

### 3. Preparation of antibodies

An antibody for use in the present invention can be prepared against an antigen composed of a chemically synthesized peptide consisting of an amino acid sequence encoded by the Exon-17 region of the C-terminal domain at which a splice variant specific to cancer cells during the pathology of cancer is formed, though such a peptide can also be obtained from any source by enzymatic digestion of periostin protein or by genetic engineering techniques.

When the peptides described above are to be used as antigens, they can be used alone as antigens but also can be used for immunization by adsorption to a macromolecular material such as polyvinyl pyrrolidone, latex or polymethyl methacrylate, or coupling to a carrier protein such as KLH (Keyhole Limpet Hemocyanin) or BSA (bovine serum albumin), and any method can be used. Generally, the peptides may preferably be coupled to a carrier protein by known methods (e.g., "New series of Development of Drugs, Vol. 14, Hirokawa Publishing Co., 1991"). The peptides are coupled to a carrier protein via cysteine residues introduced into the C- or N-terminus of the peptides so that the peptides have directionality. Crosslinkers commonly used in the field of the art can be used so long as they are suitable for this purpose. Suitable crosslinkers include succinimidyl-4-(N-maleimidomethyl)cyclohexane-1-carboxylate (hereinafter abbreviated as "SMCC") or 3-maleimidobenzoic acid-N-hydroxysuccinimide ester (MBS). Monoclonal antibodies are prepared by culturing hybridomas prepared by the cell fusion method of Kohler and Milstein (G. Kohler et al Nature (1975) 256, 495-7) to secrete the antibodies and isolating them from the cultures. That is, a mammal is immunized with a peptide having an amino acid sequence encoded by Exon-17 or the like and then antibody-producing cells of this animal are fused to myeloma cells to give hybridomas. Search for hybridomas producing antibodies binding to Exon-17 is performed by e.g., an enzyme immunoassay (hereinafter abbreviated as "ELISA") for hybridoma supernatants using a microplate on which the antigen has been immobilized. Animals to be immunized are not specifically limited, but include various mammals such as mice, rats, guinea pigs, rabbits, sheep, goats, cats, dogs, etc. Among the animals listed above, Balb/c mice are generally used for preparation of monoclonal antibodies because of ease of handling or for other reasons, but other strains of mice can also be used. The concentration of the antigen used for immunization here is selected to form sufficient amounts of antigenically stimulated lymphocytes. Preferably, 1-100 µg of an antigen is diluted to an appropriate concentration with physiological saline or the like and suspended in Freund's complete adjuvant or Freund's incomplete adjuvant or the like, and the suspension is administered to an animal by intraperitoneal or subcutaneous inoculation or other means. Administration is performed once to several times every 2-4 weeks. The final immunization is normally performed by administering a solution of 1-100 µg of the antigen in physiological saline by intravenous or subcutaneous inoculation or other means. Several days after the final immunization, antibody-producing cells such as lymphocytes, preferably spleen cells or lymph node cells, are removed from the immunized animal for cell fusion. Cell fusion using spleen cells as antibody-producing cells is explained below, though antibody-producing cells other than spleen cells can also be used for cell fusion. Spleen cells prepared from the spleen aseptically removed 3-4 days after the final immunization are fused to appropriate myeloma cells in the presence of a fusion promoter. The myeloma cells used for fusion may be derived from mammals, but generally those derived from the same species as the animal used for immunization. Various cell lines are already known, for example, SP2/0-Ag14(SP2) [Nature, 276, 269(1978)], NS-1-Ag4/1(NS-1), P3-X63Ag8U.1(P3U1) [Curr. Top. Microbiol. Immunol. 81, 1-7(1978): available from ATCC under ATCC No. CRL-1597], P3-NS1-1-Ag4-1, P3-X63Ag8(P3), FO, X63Ag8.653(X63.653), 210.RCY3.Ag1.2.3, S194/5XXO.BU1, SKO-007, GM15006TG-A12 and the like are preferably used for mice, and Y3.Ag1.2.3 and the like are preferably used for rats. Preferred fusion promoters include polyethylene glycol (PEG) having a molecular weight of 1000-6000 and Sendai virus. Generally, the ratio of spleen cells and myeloma cells during fusion is preferably 10:1-2:1.

Hybridomas can be separated from fused cells by culturing of a mixture of unfused spleen cells, unfused myeloma cells and fused cells in a selective medium inhibiting the survival of unfused myeloma cells for an appropriate period until unfused cells die (about 1 week). The selective medium may be, e.g., HAT medium (medium containing hypoxanthine, aminopterin and thymidine). In this selective medium, unfused myeloma cells die, and unfused spleen cells die after a certain period of time (after about 1 week) because they are non-tumorous cells, so that hybridomas can be obtained by selecting viable cells. Hybridomas producing desired antibodies can be obtained by searching of strains producing the desired antibodies and cloning of the strains to prepare monoclonal antibodies by standard limiting dilution. Thus obtained hybridomas producing monoclonal antibodies can grow in media suitable for their growth and can be readily stored in deep freezers or liquid nitrogen. Thus obtained hybridomas can produce antibodies by growing them in nutrient media or in the abdominal cavity of a mammal, and the produced antibodies can be purified from culture supernatants or the ascites fluid or serum of the mammal. As an example of the hybridomas, a hybridoma can be used that was deposited under FERM BP-10718 on November 1, 2006 with the International Patent Organism Depositary, the National Institute of Advanced Industrial Science and Technology. Purification of the antibodies can be performed by standard isolation/purification methods such as centrifugation, dialysis, salting out with ammonium sulfate or the like, ion exchange chromatography using a DEAE column or the like, gel filtration, affinity chromatography, etc. The isotypes and subclasses of thus obtained monoclonal antibodies can be determined using an identification method such as Ouchterlony assay, ELISA, or RIA. Ouchterlony assay is convenient but requires a concentration operation if the monoclonal antibody concentration is low. When ELISA or RIA is used, however, the isotypes and subclasses of the monoclonal antibodies can be identified by direct reaction of the culture supernatant with an antigen-adsorbed solid phase and by use of antibodies corresponding to various immunoglobulin isotypes and subclasses as secondary antibodies. More convenient methods employ commercially available identification kits (e.g., Mouse Typer kit; Bio-Rad) or the like. The quantification of protein can be performed by the Folin-Lowry method and calculation from the absorbance at 280 nm [1.4 (OD280) = 1 mg/ml immunoglobulin]. Thus obtained monoclonal antibodies specifically recognize a periostin isoform consisting of an amino acid sequence shown in SEQ ID NO: 1, SEQ ID NO: 2 or SEQ ID NO: 8 (PN-1 protein) or a peptide encoded by Exon-17 consisting of an amino acid sequence shown in SEQ ID NO: 3, SEQ ID NO: 4 or SEQ ID NO: 21. Preferably, the monoclonal antibodies for use in the present invention can specifically recognize and bind to a peptide consisting of the amino acid sequence YTTKIITKVV (SEQ ID NO: 26), i.e., a peptide consisting of an amino acid sequence covering from the -1st tyrosine to the 9th valine from the N-terminus of the amino acid sequence of the human periostin Exon-17 peptide chain (SEQ ID NO: 4), and a peptide consisting of an amino acid sequence covering from the 669th tyrosine to the 679th valine from the N-terminus of the amino acid sequence of human periostin PN-1 (SEQ ID NO: 2). Namely, the monoclonal antibodies for use in the present invention can specifically recognize an amino acid sequence site (TTKIITKVV; SEQ ID NO: 22), or a part thereof, which covers from the N-terminal threonine to the 9th valine of the amino acid sequence of the human periostin Exon-17 peptide chain (SEQ ID NO: 4). More preferably, the monoclonal antibodies for use in the present invention can recognize and bind to a peptide comprising alanine substitutions at the 1st and the 8-10th amino acids from the N-terminus of a peptide consisting of an amino acid sequence (YTTKIITKVV; SEQ ID NO: 26) covering from the - 1st tyrosine to the 9th valine from the N-terminus of the human periostin Exon-17 peptide chain (SEQ ID NO: 4). Namely, the monoclonal antibodies for use in the present invention can specifically recognize at least an amino acid sequence site (SEQ ID NO: 34), or a part thereof, which covers from the 1st threonine to the 6th threonine from the N-terminus of the amino acid sequence of the human periostin Exon-17 peptide chain (SEQ ID NO: 4) or the amino acid sequence of the rat periostin Exon-17 peptide chain (SEQ ID NO: 3). Moreover, the monoclonal antibodies for use in the present invention have the activity of suppressing or inhibiting the PN-1 protein's effects of inhibiting cancer cell adhesion and detaching adhered cells, i.e., the activity of neutralizing the PN-1 protein's effects of inhibiting cancer cell adhesion and detaching adhered cells. The monoclonal antibodies for use in the present invention further have inhibitory activity on the growth of primary tumor, inhibitory activity on bone invasion of cancer cells, inhibitory activity on bone destruction caused by bone invasion of cancer cells, and inhibitory activity on the metastasis of cancer cells.

When polyclonal antibodies are used as antibodies, the polyclonal antibodies can be obtained by standard methods such as the method described in "New Lecture on Biochemical Experiments, 12, edited by the Japanese Biochemical Society, Tokyo Kagaku Dozin, 1992". Animals to be immunized are not specifically limited, but include horses, goats, sheep, rabbits, guinea pigs, mice, chickens, etc. When a rabbit is to be immunized, an antigen is diluted to an appropriate concentration with physiological saline or the like and suspended in Freund's complete adjuvant, Freund's incomplete adjuvant or aluminum hydroxide adjuvant or the like, and the suspension is injected at a dose of 10-1000 µg per animal followed by 1-3 booster inoculations after 2-4 weeks to give antisera. Multi-site subcutaneous inoculation is preferred. Preparation of polyclonal antibodies from antisera can be performed by the method as described for the purification of monoclonal antibodies. Thus obtained polyclonal antibodies specifically recognize a periostin isoform consisting of an amino acid sequence shown in SEQ ID NO: 1, SEQ ID NO: 2 or SEQ ID NO: 8 (PN-1 protein) or a peptide encoded by Exon-17 consisting of an amino acid sequence shown in SEQ ID NO: 3, SEQ ID NO: 4 or SEQ ID NO: 21. Preferably, the polyclonal antibodies for use in the present invention can specifically recognize and bind to a peptide consisting of the amino acid sequence YTTKIITKVV (SEQ ID NO: 26), i.e., a peptide consisting of an amino acid sequence covering from the -1st tyrosine to the 9th valine from the N-terminus of the amino acid sequence of the human periostin Exon-17 peptide chain (SEQ ID NO: 4), and a peptide consisting of an amino acid sequence covering from the 669th tyrosine to the 679th valine from the N-terminus of the amino acid sequence of human periostin PN-1 (SEQ ID NO: 2). Namely, the polyclonal antibodies for use in the present invention can specifically recognize an amino acid sequence site (TTKIITKVV; SEQ ID NO: 22), or a part thereof, which covers from the N-terminal threonine to the 9th valine of the amino acid sequence of the human periostin Exon-17 peptide chain (SEQ ID NO: 4). More preferably, the polyclonal antibodies for use in the present invention can recognize and bind to a peptide comprising alanine substitutions at the 1st and the 8-10th amino acids from the N-terminus of a peptide consisting of an amino acid sequence (YTTKIITKVV; SEQ ID NO: 26) covering from the - 1st tyrosine to the 9th valine from the N-terminus of the human periostin Exon-17 peptide chain (SEQ ID NO: 4). Namely, the polyclonal antibodies for use in the present invention can specifically recognize at least an amino acid sequence site (SEQ ID NO: 34), or a part thereof, which covers from the 1st threonine to the 6th threonine from the N-terminus of the amino acid sequence of the human periostin Exon-17 peptide chain (SEQ ID NO: 4) or the amino acid sequence of the rat periostin Exon-17 peptide chain (SEQ ID NO: 3). Moreover, the polyclonal antibodies for use in the present invention have the activity of suppressing or inhibiting the PN-1 protein's effects of inhibiting cancer cell adhesion and detaching adhered cells, i.e., the activity of neutralizing the PN-1 protein's effects of inhibiting cancer cell adhesion and detaching adhered cells. The polyclonal antibodies for use in the present invention further have inhibitory activity on the growth of primary tumor, inhibitory activity on bone invasion of cancer cells, inhibitory activity on bone destruction caused by bone invasion of cancer cells, and inhibitory activity on the metastasis of cancer cells.

### 4. Preparation of humanized antibodies

Immunoglobulin G (hereinafter simply referred to as "IgG") consists of two light polypeptide chains having a molecular weight of about 23000 (hereinafter referred to as "light chain") and two heavy polypeptide chains having a molecular weight of about 50000 (hereinafter referred to as "heavy chain"). The heavy and light chains both have a repeating structure of conserved amino acid sequence regions consisting of about 110 residues, which constitute a basic unit of three-dimensional structure of IgG (hereinafter referred to as "domain"). The heavy and light chains consist of 4 and 2 successive domains, respectively. In both of the heavy and light chains, the amino acid sequence of the amino terminal domain is more variable between antibody molecules than that of the other domains, and this domain is called variable domain (hereinafter referred to as "V domain"). At the amino terminus of IgG, the V domains of the heavy and light chains are complementarily associated to form a variable region. In contrast, the remaining domains collectively form a constant region. The constant region has a sequence characteristic of each animal species, e.g., the constant region of mouse IgG differs from the constant region of human IgG so that mouse IgG is recognized as foreign matter by the human immune system, resulting in a Human Anti Mouse Antibody (hereinafter referred to as "HAMA") response (Schroff RW. et al., Cancer Res. (1985) 45,879-85). Thus, mouse antibodies cannot be repeatedly administered to humans. In order to administer such antibodies to humans, the antibody molecules must be modified to prevent HAMA response while maintaining the specificity of the antibodies. According to the results of X-ray crystallography, such a domain is generally in the form of an elliptic cylindrical structure formed of two antiparallel beta sheets consisting of 3 to 5 beta-chains. In the variable region, three loops for each of the V domains of the heavy and light chains are assembled to form an antigen-binding site. These loops are called complementarity determining regions (hereinafter referred to as "CDRs"), which are most variable in amino acid sequence. The remaining parts of the variable region other than the CDRs serve to maintain the structures of the CDRs and are called "framework". Kabatt et al. collected a number of primary sequences of heavy and light chain variable regions and prepared a table classifying the primary sequences into CDRs and frameworks on the basis of sequence conservation (Kabatt et al., SEQUENCES OF IMMUNOLOGICAL INTEREST, 5th edition, NIH publication, No. 91-3242, E.A.). The frameworks were further classified into a plurality of subgroups having common amino acid sequence patterns. The presence of a consensus framework between human and mouse sequences was also found. Such studies on structural features of IgG led to the development of the processes for preparing humanized antibodies described below. At an early stage of the studies, chimeric antibodies having a variable region from a mouse antibody fused to a constant region from a human antibody were proposed (Morrison SL. et al., Proc Natl Acad Sci USA. (1984)81, 6851-5). However, such chimeric antibodies may induce a HAMA response, especially when they are administered for a long term, because they still contain many non-human amino acid residues (Begent et al., Br. J. Cancer, (1990)62, 487).

A method for further reducing amino acid residues derived from a non-human mammal that may induce a HAMA response to humans by transferring only the CDRs into a human antibody was proposed (Peter T et al., Nature, (1986) 321, 522-5), but grafting of only the CDRs was normally insufficient to maintain immunoglobulin activity against the antigen. On the other hand, Chothia et al. used X-ray crystallographic data in 1987 to find that (a) the amino acid sequences of the CDRs contain a site directly binding to the antigen and a site maintaining the structures of the CDRs, and possible three-dimensional structures of the CDRs are classified into multiple typical patterns (canonical structures), and that (b) the classes of the canonical structures are determined by not only the CDRs but also the types of amino acids at specific locations on the framework (Chothia C. et al., J. Mol. Biol. (1987) 196, 901-17). Based on this finding, a document suggested that when CDR grafting is used, amino acid residues on a part of the framework should also be grafted into a human antibody in addition to the CDR sequences (JPA No. HEI-4-502408). Generally, an antibody derived from a non-human mammal having CDRs to be grafted is defined as "donor", and a human antibody into which the CDRs are grafted is defined as "acceptor", and considerations in CDR grafting are to conserve the structures of the CDRs to the extent possible to maintain the activity of the immunoglobulin molecule. To achieve this object, two points should be kept in mind, i.e., (a) which subgroup of acceptor should be selected, and (b) which amino acid residue should be selected from the framework of the donor.

Queen et al. proposed methods for designing immunoglobulins wherein an amino acid residue in the framework of a donor is grafted into an acceptor in addition to the CDR sequences when at least one of the following criteria is satisfied (JPA No. HEI-4-502408):
(a) the amino acid in the framework region of the acceptor is rare for that position and the corresponding amino acid in the donor is common for that position;
(b) the amino acid is immediately adjacent to one of the CDRs; or
(c) the amino acid is predicted to have a side chain atom within about 3 angstroms of the CDRs in a three-dimensional immunoglobulin model and to be capable of interacting with the antigen or with the CDRs of the humanized antibody.

The DNA encoding the heavy or light chain of an anti-Exon-17 monoclonal antibody for use in the present invention can be obtained by preparation of mRNA from hybridoma cells producing the anti-Exon-17 monoclonal antibody, conversion of the mRNA into cDNA by reverse transcriptase and then isolation of the DNA encoding the heavy or light chain of the antibody.

### 5. Preparation of human antibodies

As used herein, the "human antibody" or "human immunoglobulin" means an immunoglobulin in which all the regions constituting the immunoglobulin including heavy chain variable regions (VH) and heavy chain constant regions (CH) as well as light chain variable regions (VL) and light chain constant regions (CL) are derived from genes encoding a human immunoglobulin. In other words, it means an antibody in which the heavy chain is derived from a human immunoglobulin heavy chain gene and the light chain is derived from a human immunoglobulin light chain gene. Human antibodies can be prepared by standard methods, e.g., by immunization of a transgenic animal prepared by integration of at least a human immunoglobulin gene into the locus of a non-human mammal such as a mouse with an antigen, in the same manner as described above for the preparation of monoclonal antibodies. For example, transgenic mice producing human antibodies can be prepared by the methods described in prior documents (Mendez MJ et al., Nature Genetics (1997)15, 146-56, Green LL et al., Nature Genetics (1994)7, 13-21, JPA No. HEI-4-504365; International Publication No. WO94/25585; Nikkei Science, June, pp. 40-50, 1995; Nils Lonberg et al., Nature (1994) 368, 856-9, and JPA No. HEI-6-500233).

Antibodies for use in the present invention are not limited to whole antibody molecules and may be antibody fragments or derivatives as long as they can inhibit (suppress) the activity of a periostin isoform having anti-cell adhesive activity.

### 6. Antibody fragments

Antibody fragments include, for example, Fab, F(ab')₂, Fv, single chain antibody (scFv), disulfide-stabilized antibody (dsFv), a CDR-containing peptide, etc.

Among antibody fragments for use in the present invention, Fab, F(ab')₂ and the like can be obtained by treating an antibody against a peptide encoded by Exon-17 of PN-1 with a proteolytic enzyme such as papain or pepsin, or alternatively, can be prepared by constructing a gene encoding the resulting antibody fragment and introducing this construct into an expression vector, followed by expression in an appropriate host cell.

Among antibody fragments for use in the present invention, scFv can be prepared by linking together an H chain V region and an L chain V region from an antibody against a peptide encoded by Exon-17 of PN-1 by using an appropriate peptide linker or the like. Alternatively, scFv can be prepared by constructing a DNA segment encoding the entire sequences or desired amino acid sequences of a gene encoding an H chain or H chain V region from the above antibody and a gene encoding an L chain or L chain V region from the antibody, and introducing this construct into an expression vector, followed by expression in an appropriate host cell.

Among antibody fragments for use in the present invention, dsFv is an antibody fragment in which polypeptides modified to replace one amino acid residue by a cysteine residue in both H and L chain V regions from an antibody against a peptide encoded by Exon-17 of PN-1 are linked together between these cysteine residues via a disulfide linkage. An amino acid residue to be replaced by a cysteine residue can be selected by stereostructural estimation of the antibody. dsFv can be prepared by constructing a DNA segment encoding the entire sequence or a desired amino acid sequence of a gene encoding the antibody fragment, and introducing this construct into an expression vector, followed by expression in an appropriate host cell.

Among antibody fragments for use in the present invention, a CDR-containing peptide comprises at least one or more CDR regions selected from CDR regions in H or L chains of an antibody inhibiting the anti-cell adhesive activity of periostin. Also, multiple CDR regions may be linked together by techniques using an appropriate peptide linker or the like. The CDR-containing peptide may also be prepared by constructing a DNA segment encoding the entire sequence or a desired amino acid sequence of a gene encoding the peptide, and introducing this construct into an expression vector, followed by expression in an appropriate host cell. Alternatively, the CDR-containing peptide can also be prepared by chemical synthesis such as Fmoc or tBoc method.

### 7. Diagnostic reagent

The present invention also provides a reagent for cancer diagnosis prepared by labeling of an antibody as described above with a marker. Markers that can be used here include enzymes, radioisotopes, fluorescent dyes, etc. The enzymes used here are not specifically limited so long as they satisfy criteria such as high turnover number, stability even after conjugation, and the ability to specifically react with their substrates to develop color, etc., and enzymes used in standard enzyme immunoassays (EIA) can be used. Examples of preferred enzymes include peroxidases, β-galactosidases, alkaline phosphatases, glucose oxidase, acetylcholine esterase, glucose-6-phosphate dehydrogenase, malate dehydrogenase, etc. Enzyme inhibitors and coenzymes and the like can also be used.

Conjugation of these enzymes and antibodies can be performed by known methods using crosslinkers such as maleimide compounds. Substrates that can be used are known materials, selected depending on the enzymes used. For example, when the enzyme used is a peroxidase, 3,3',5,5'-tetramethylbenzidine can be used, or when the enzyme used is an alkaline phosphatase, paranitrophenol or the like can be used. Radioisotopes that can be used as markers include those used in the standard radioimmunoassay (RIA) such as 1251 and 3H. Fluorescent dyes that can be used are those used in the standard fluoroimmunoassay such as fluorescence isothiocyanate (FITC) and tetramethyl rhodamine isothiocyanate (TRITC). The present diagnostic reagent can also be used as immunohistological staining capable of specifically staining cancer interstitial tissue. When it is labeled with a radioisotope, it can also be used to image the lesion during the pathology of cancer by internal administration.

### 8. Diagnostic method

The present invention is also directed to a method for cancer diagnosis, which comprises measuring the amount of a periostin isoform having a peptide region encoded by Exon-17 in a biological sample, for example, a serum sample prepared from human or animal blood, by using an antibody for use in the present invention. In the present method, a periostin isoform having a peptide region encoded by Exon-17 can be detected by a so-called sandwich ELISA (Enzyme-linked immunosorbent assay). When a diagnostic kit is used, a sample is initially contacted with a plate on which a primary antibody has been immobilized to form a complex, and a secondary antibody labeled with a marker is bound to this complex, and then the signal intensity of the marker in this ternary complex is measured, whereby a periostin isoform having a peptide region encoded by Exon-17 can be detected or quantified. In particular, since a periostin isoform having a peptide region encoded by Exon-17 is a splice variant which is highly expressed during the pathology of cancer or the like and is involved in primary tumor growth and cancer metastasis, the pathology in cancer or the like can be diagnosed by monitoring its production.

In this way, an antibody for use in the present invention can be used here as the secondary antibody by labeling of the antibody.

The present invention is also directed to a method for cancer diagnosis, which comprises measuring the amount of a periostin isoform having the Exon-17 region in a biological sample, for example, an RNA sample prepared from human or animal tissue, by using primers specific to the Exon-17 region (sense strand: 5'-TAAAATTATAACCAAAGTTGTGGAACC-3' (SEQ ID NO: 35) and antisense strand: 5'-AGTGTGGGTCCTTCAGTTTTGAT-3' (SEQ ID NO: 36)). In the present method, the presence or absence of a periostin isoform having the Exon-17 region can be detected and quantified by so-called RT-PCR or PCR with SYBR^{®} Green I. In particular, since a periostin isoform having the Exon-17 region is a splice variant which is highly expressed during the pathology of cancer and is involved in primary tumor growth and cancer metastasis, the pathology in cancer can be diagnosed by monitoring its production.

The following examples further illustrate the present invention in detail and specifically.

### EXAMPLES

### [Preparation example 1] Search for periostin by subtraction

### 1-1 Preparation of pathologic model rats of heart failure and collection of left ventricular samples

Male Dahl salt-sensitive rats (Dahl-S) (Shimizu Laboratory Supplies) were raised on an 8% high salt diet from 6 weeks of age, and the left ventricle was collected from three animals each at cardiac hypertrophy stage (11 weeks of age) and heart failure stage (14 weeks of age).

### 1-2 Preparation of mRNA

Total RNA was prepared from about 500 mg of the left ventricle using ISOGEN (Nippon Gene) as instructed by the manufacturer. Then, mRNA was purified from about 400 µg of the combined total RNA from three animals each at cardiac hypertrophy stage and heart failure stage using Fast Track 2.0 Kit (Invitrogen) as instructed by the manufacturer to recover about 3 µg of mRNA at each stage.

### 1-3 cDNA subtraction

cDNA subtraction was performed using PCR-Select cDNA subtraction kit (Clontech) as instructed by the manufacturer. That is, cDNA was synthesized from 2 µg of each mRNA obtained in 1-2 above and digested with restriction enzyme RsaI. Then, subtraction hybridization was performed using the cDNA synthesized from the animals at 14 weeks of age as tester cDNA and the cDNA synthesized from the animals at 11 weeks of age as driver cDNA after 2 adapters included in the kit had been separately linked to the tester cDNA. Then, a cDNA fragment with altered expression level was specifically amplified by PCR using primers complementary to the adapters to give amplification product 1.

A similar subtraction operation was performed using the cDNA synthesized from the animals at 11 weeks of age as tester cDNA and the cDNA synthesized from the animals at 14 weeks of age as driver cDNA to give amplification product 2.

### 1-4 Dot blot screening

### A. Preparation of dot blots

Amplification product 1 was TA cloned into a PCR II vector (Invitrogen) and clones having the insert fragment were selected. The insert fragment of each clone was amplified by PCR reaction, and then 1 µl each of the reaction solution was heat-treated and then dot-blotted on 2 nylon membrane filters (Boehringer) and fixed with a UV crosslinker (Stratagene).

### B. Preparation of cDNA probes

Amplification product 1 was digested with restriction enzymes RsaI and EaeI, SmaI to remove the adapters and subjected to random prime labeling with DIG-dUTP using DIG High Prime DNA labeling/detection kit II (Boehringer) as instructed by the manufacturer to prepare cDNA probe 1. Similarly, cDNA probe 2 was prepared from amplification product 2.

### C. Screening

One of the dot blot membranes prepared in A above was hybridized with cDNA probe 1 and the other with cDNA probe 2. Specifically, hybridization was performed in DIG Easy Hyb solution at 42 °C overnight using DIG High Prime DNA labeling/detection kit II (Boehringer) as instructed by the manufacturer. The membranes were washed twice with 2 x SSC, 0.1% SDS at room temperature for 5 minutes and twice with 0.1 x SSC, 0.1% SDS at 68 °C for 15 minutes, and then reacted with alkaline phosphatase-labeled anti-DIG antibodies in the blocking buffer included in the kit, and then CSPD ready-to use was added to advance chemiluminescence and X-ray film was exposed. Clones showing a stronger signal in cDNA probe 1 than cDNA probe 2 were selected as positive clones and sequenced.

### 1-5 Sequencing

The nucleotide sequences were determined by analysis on an automatic DNA sequencer model 373A (PE Applied Biosystems) using THERMO Sequenase^{™} II dye terminator cycle sequencing kit (Amersham Pharmacia). Thus obtained gene sequences were compared with sequences in the GenBank databank to reveal that one of the clones (SF014) was a gene having an 86% homology to mouse periostin (GenBank Accession No. D13664).

### [Preparation example 2] Cloning of rat periostin cDNA

Rat periostin cDNA was isolated by screening 10 phage subpools of about 4000 clones (a total of about 40,000 clones) prepared from a rat aorta cDNA library (Clontech) inserted into λgtII vector by PCR using primers (1) 5'-GTTCATTGAAGGTGGCGATGGTC-3' (SEQ ID NO: 15), and (2) 5'-GAGATAAAATCCCTGCATGGTCCT-3' (SEQ ID NO: 16) designed on the basis of the nucleotide sequence of SF014 to give 3 positive subpools. One of the subpools was screened by hybridization using the fragment amplified by PCR as a probe labeled with alkaline phosphatase using AlkPhos Direct^{™} (Amersham Pharmacia) to give one positive clone rat periostin #1. Its insert fragment was subcloned into the EcoRI site of pBluescript II (Stratagene) and the total nucleotide sequence was determined according to the method of Preparation example 1-5.

The resulting clone had a length of about 3 kb corresponding to nucleotide 292 to the 3' end of mouse periostin (GenBank Accession No. D13664), suggesting that it was a 5'-truncated clone.

Thus, SMART^{™} RACE cDNA Amplification Kit (Clontech) was used as instructed by the manufacturer to perform 5'-RACE reaction using rat aorta cDNA as a template and the primer (2) 5'-GAGATAAAATCCCTGCATGGTCCT-3' (SEQ ID NO: 16) and a primer (3) 5'-CACGGTCGATGACATGGACAACACC-3' (SEQ ID NO: 17) designed on the basis of the nucleotide sequence of rat periostin #1. The resulting PCR product was TA cloned into PCR II vector of Invitrogen to give a clone designated as rat periostin 5'RACE #1. The nucleotide sequence was determined according to the method of Preparation example 1-5.

The results showed that rat periostin 5'RACE #1 was a clone longer by about 300 bp than the initially obtained rat periostin #1 in the 5' direction with the 5' end being longer by 15 bp than the 5' end of mouse periostin (GenBank Accession No. D13664). Ten phage subpools of about 40,000 clones (a total of about 400,000 clones) prepared from the rat aorta cDNA library were screened by PCR using a primer (4) 5'-ACGGAGCTCAGGGCTGAAGATG-3' (SEQ ID NO: 18) designed on the basis of the nucleotide sequence of rat periostin 5'RACE #1 and the primer (3) 5'-CACGGTCGATGACATGGACAACACC-3' (SEQ ID NO: 17) to give 2 positive subpools. One of the subpools was screened by hybridization using the fragment amplified by PCR as a probe to give one positive clone designated as rat periostin #2. The insert fragment was subcloned into the EcoRI site of pBluescript II (Stratagene) and the nucleotide sequence was determined according to the method of Preparation example 1-5.

The resulting clone had a length of about 2.6 kb with the 5' end being the same as that of the clone obtained with 5'-RACE and the 3' end corresponding to up to nucleotide 2410 of mouse periostin (GenBank Accession No. D13664). The nucleotide sequence of rat periostin 5'RACE #1 previously obtained was exactly the same as the nucleotide sequence of the relevant region of rat periostin #2. The full length of rat periostin cDNA was completed by rat periostin #1 and rat periostin #2. The nucleotide sequence of this full-length cDNA and the amino acid sequence translated from this nucleotide sequence are shown as SEQ ID NOs: 6 and 1.

### [Preparation example 3] Construction of an Myc-His-rat periostin fusion protein expression vector

An expression vector having an Myc epitope and 6 histidine tags at the carboxyl terminus of the protein translated from the coding region of the rat periostin gene obtained in Preparation example 2, and having a CMV promoter, was prepared.

Initially, a fragment of about 500 bp obtained by digestion of rat periostin 5'RACE #1 obtained in Preparation example 2 with restriction enzymes EcoRI and HindIII and a fragment of about 2780 bp obtained by digestion of rat periostin #1 obtained in Preparation example 2 with restriction enzymes HindIII and HpaI were ligated to a vector fragment obtained by digesting pTracer-CMV2 vector (Invitrogen) with restriction enzymes EcoRI and EcoRV using a ligation kit (Takara Bio Inc.) to give a plasmid designated as pTracer-CMV2/rat periostin. Thus prepared pTracer-CMV2/rat periostin was digested with restriction enzymes EcoRI and SmaI to give a fragment of about 2330 bp containing the coding region of the rat periostin gene, and PCR was performed using rat periostin #1 obtained in Preparation example 2 as a template and primer (5) 5'-GACCCGGGAAGAACGCATCATC-3' (SEQ ID NO: 19) designed on the basis of the sequence of the template and primer (6) 5'-TGGGTGACCCTGAGAACGGCCTTCTCTTGATC-3' (SEQ ID NO: 20) designed to insert a BstEII site immediately before the stop codon of rat periostin and the amplification product was purified and then digested with restriction enzymes SmaI and BstEII to give a fragment of about 270 bp. These two fragments were ligated to a vector fragment obtained by digestion of an expression vector constructing plasmid pcDNA4/Myc-His/type C (Invitrogen) with restriction enzymes EcoRI and BstEII using a ligation kit (Takara Bio Inc.) to give a plasmid designated as pcDNA4/Myc-His/rat periostin. The total nucleotide sequence of the insert was confirmed by the method described in Preparation example 1-5.

### [Preparation example 4] Construction of a baculovirus expression vector

The plasmid pcDNA4/Myc-His/rat periostin obtained in Preparation example 3 was digested with restriction enzymes SacI and PmeI to excise a peptide fragment rat PN-1/Myc-His. This fragment was ligated to a vector fragment obtained by digesting pFastBacHTc (Invitrogen) with restriction enzymes SacI and Kpnl (blunting) using a ligation kit (Takara Bio Inc.) to give an expression vector designated as pFastBac/rat periostin-1/Myc-His. The nucleotide sequence of the insert was confirmed by the method described in Preparation example 1-5.

### [Preparation example 5] Preparation and cultivation of a recombinant baculovirus

DH10BAC cells of Escherichia coli were transformed with pFastBac/rat periostin-1/Myc-His obtained in Preparation example 5 to prepare a recombinant baculovirus. Electrophoresis and PCR confirmed that the resulting baculovirus contains the desired insert.

Insect Sf9 cells (2 x 10⁶ cells/mL) infected with this recombinant baculovirus at MOI=0.1 were cultured in a serum-free medium (containing 50 µg/mL gentamicin in 2000 mL of Sf-900IISFM (Invitrogen)) at 28°C for 4-5 days and then the culture supernatant was harvested.

### [Preparation example 6] Purification of rat periostin protein

To an SP Sepharose Fast Flow column (10 mL bed volume) equilibrated with an equilibration buffer (50 mM sodium acetate buffer, pH 6.0, 0.1 M sodium chloride) was applied 2000 mL of the culture supernatant obtained in Preparation example 5, and the resulting flow-through fraction was pooled as an SP Sepharose flow-through fraction.

The column was washed with the equilibration buffer until the absorbance at 280 nm approached 0 (about 100 mL) to give an SP Sepharose wash fraction.

The column was eluted with 100 mL of an elution buffer (50 mM sodium dihydrogen phosphate (pH 8.0), 0.5M sodium chloride, 5 mM imidazole) to give an SP Sepharose eluate fraction.

Then, 100 mL of the SP Sepharose eluate fraction was applied to an Ni-NTAagarose column (5 mL bed volume) equilibrated with 50 mM sodium phosphate buffer, pH 8.0, 0.5M sodium chloride and 5 mM imidazole, and the resulting flow-through fraction was pooled as an Ni-NTA agarose flow-through fraction.

The column was washed with about 50 mL of a washing buffer (50 mL sodium dihydrogen phosphate, pH 8.0, 0.5 M sodium chloride, 5 mM imidazole) to give an Ni-NTA agarose wash fraction.

The column was eluted with about 25 mL each of elution buffers (1) 50 mM sodium dihydrogen phosphate, 0.5 M sodium chloride, 20 mM imidazole, followed by similar compositions except that the imidazole concentrations were (2) 30 mM, (3) 40 mM, (4) 50 mM and (5) 60 mM to give Ni-NTA agarose eluate fractions (1)-(6).

Fractions shown to contain the desired protein by Western blotting were concentrated to 1 mL or less.

Then, the concentrated samples were applied to a gel filtration column (Sephacryl S-200HR φ11 mm x 95 cm; 90 bed volume) equilibrated with degassed PBS(-) (137 mM NaCl, 8.1 mM Na₂HPO₄, 2.68 mM KCl, 1.47 mM KH₂PO₄) and eluted with PBS(-) and the eluate was lyophilized to give a purified rat periostin protein.

### <Example 1> Synthesis of rat Exon-17 peptide chain and preparation of polyclonal antibody against it

A structure specific to rat PN-1 protein was identified as Exon-17 sequence by sequence comparison since heart dilation was induced by increased expression of the PN-1 gene in the heart of normal SD rats and the survival rate was improved by administration of an antisense oligonucleotide against rat periostin to the heart of Dahl heart failure model rats and rat PN-1 protein was shown to have no cell adhesive properties in contrast to previously reported PN-2 (see Figure 1). A peptide having a Cys residue added to the N-terminus of the amino acid sequence encoded by this Exon-17 was chemically synthesized in 10 mg yield at a purity of 80% or more. Rabbits (Kbl:JW) were immunized with the peptide coupled to 6 mg of a carrier protein KLH. FCA (Freund's complete adjuvant) was used in the primary immunization, and FIA (Freund's incomplete adjuvant) was used in the secondary and subsequent immunizations. Administration was performed at 20 dorsal subcutaneous sites at weeks 0, 2, 4 and 6 using a peptide dose of 800 µg/animal in the primary immunization, and 400 µg/animal in the secondary and subsequent immunizations. The antibody titer was determined by ELISA and total sera were collected at week 7. Then, an affinity column was prepared by use of a synthetic peptide, and only the antibody specifically reacting to the Exon-17 peptide was collected. The polyclonal antibody against the peptide encoded by Exon-17 of rat periostin is hereinafter referred to as anti-rat Exon-17 polyclonal antibody.

### <Example 2> In vitro study of the presence or absence of non-cell adhesive activity of rat PN-1 protein

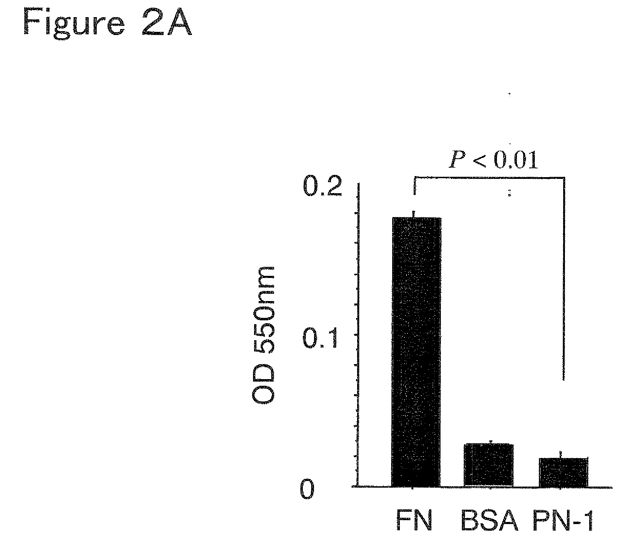

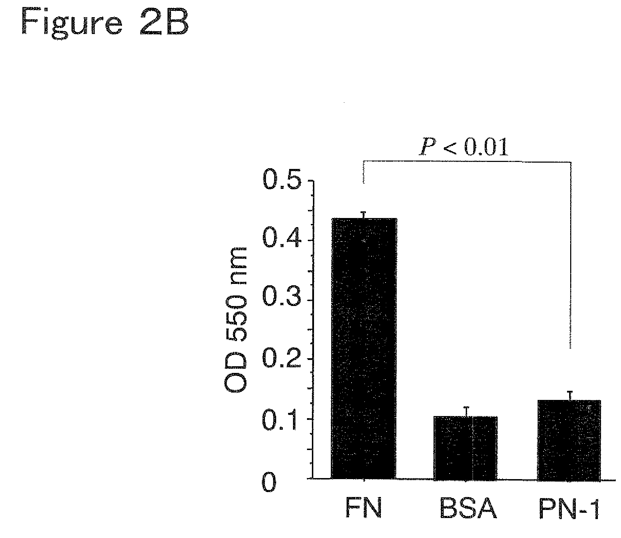

To a 96-well cell culture multiwell plate, 10 µg/ml fibronectin, 100 µg/ml BSA or 10 µg/ml PN-1 protein was added and coated overnight at 4°C. After removal of the protein solution from the wells, mouse melanoma B16-F10 cells (ATCC No. CRL-6475) or mouse 4T1 breast cancer cells (ATCC No. CRL-2539) suspended in DMEM (10% BSA, PC/SM) were added at 10⁴ cells/well and cultured in a 37°C incubator for 3 hours. The level of cell adhesion was measured as follows. After removal of the culture supernatant, the cells were fixed in 2.5% glutaraldehyde for 30 minutes, stained with 0.02% crystal violet and then measured for their absorbance at OD 550 nm with a plate reader (BIO-RAD, Model 680 MICRO PLATE READER). Untreated wells were stained as background samples and used to correct the absorbance values for comparison purposes. Data analysis was made by the Fisher's PLSD test (Figures 2A and 2B). As a result, the positive control fibronectin showed cell adhesion, whereas the negative control BSA showed no cell adhesion. The group treated with rat PN-1 protein showed no cell adhesion, indicating that rat PN-1 protein has the effect of preventing cells from adhering, i.e., non-cell adhesive properties.

### <Example 3> In vitro study of the presence or absence of anti-cell adhesive activity of rat PN-1 protein

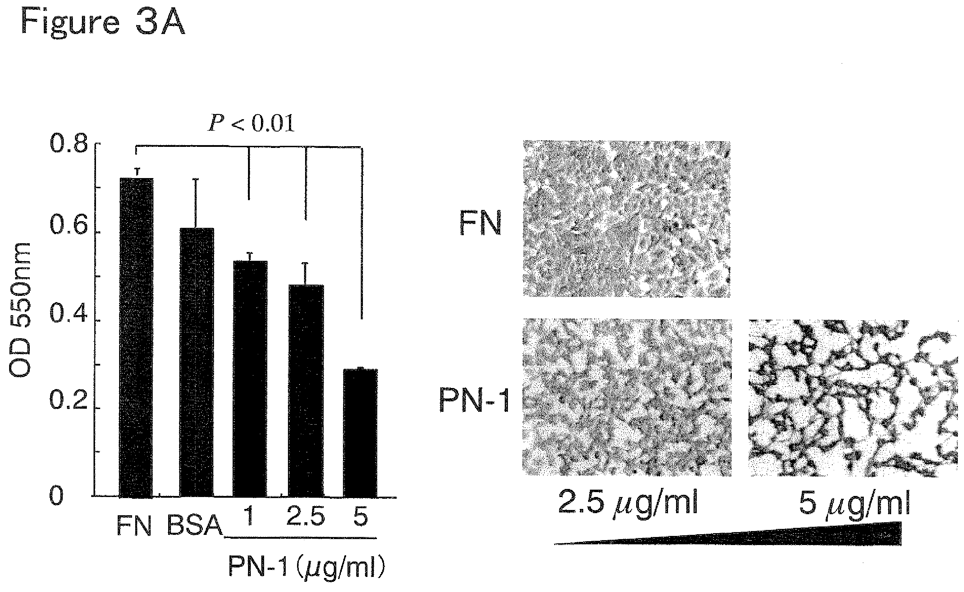

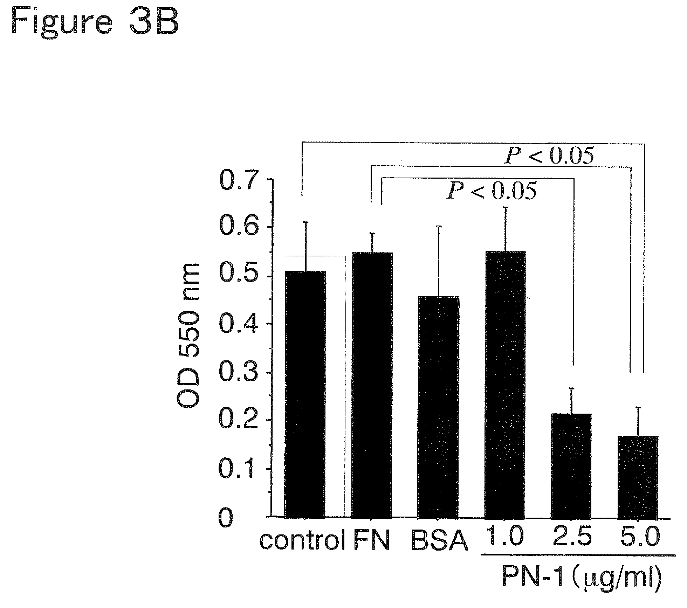

To a 96-well cell culture multiwell plate, mouse melanoma B16-F10 cells or mouse 4T1 breast cancer cells suspended in DMEM (10% BSA, PC/SM) were added at 10⁴ cells/well and cultured overnight in a 37°C incubator. After removal of the culture supernatant, 10 µg/ml fibronectin (SIGMA), 100 µg/ml BSA (SIGMA) or PN-1 protein was added and cultured in a 37°C incubator for 1 to 3 hours. The level of cell adhesion was measured as follows. After removal of the culture supernatant, the cells were fixed in 2.5% glutaraldehyde for 30 minutes, stained with 0.02% crystal violet and then measured for their absorbance at OD 550 nm with a plate reader (BIO-RAD, Model 680 MICRO PLATE READER). Untreated wells were stained as background samples and used to correct the absorbance values for comparison purposes. Data analysis was made by the Fisher's PLSD test (Figures 3A and 3B). The cells were photographed with a Nikon COOLPIX4500 attached to a stereoscopic microscope LEICA MZ16. As a result, the control groups treated with fibronectin and BSA and the untreated group did not show anti-cell adhesive properties because the adhered cells were not detached, in contrast to the group treated with rat PN-1 protein in which the cells were detached, indicating that rat PN-1 protein has a detaching effect on adhered cells, i.e., anti-cell adhesive properties.

### <Example 4> In vitro study of the neutralizing activity of anti-rat Exon-17 polyclonal antibody

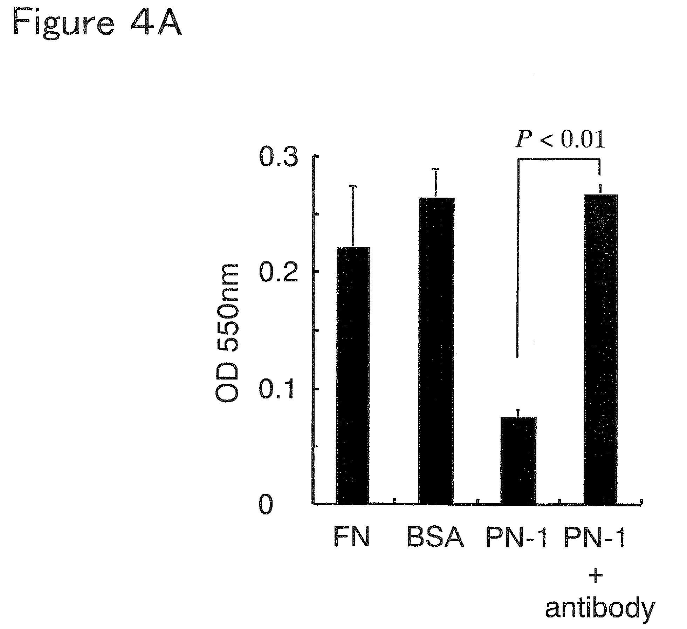

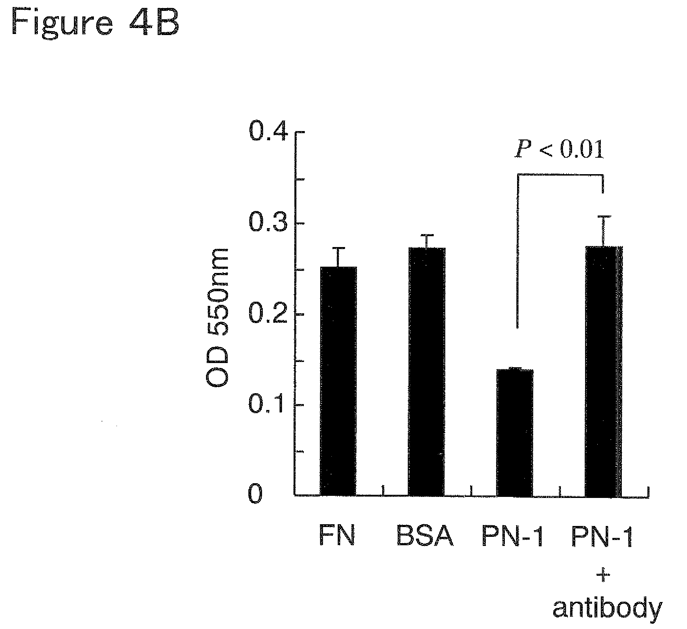

In the same manner as shown in Example 3, to a 96-well cell culture multiwell plate, mouse melanoma B16-F10 cells or mouse 4T1 breast cancer cells suspended in DMEM (10% BSA, PC/SM) were added at 10⁴ cells/well and cultured overnight in a 37°C incubator. After removal of the culture supernatant, the culture was incubated in fresh DMEM medium with 10% FBS containing 10 µg/ml cycloheximide at 37°C for 1 hour. Then, the cells were washed twice with DMEM medium (serum free) prewarmed at 37°C, and rat periostin protein and anti-rat Exon-17 polyclonal antibody were added to DMEM medium (serum free) at final concentrations of 10 µg/ml and 100 µg/ml, respectively. Rat periostin protein alone was used as a positive control and BSA was used as a negative control. After incubation at 37°C for 1 hour, microscopy showed that almost all the cells were detached in the group treated with rat periostin protein alone, and the cells were washed twice with PBS(-) and then fixed in 10% neutral buffered formalin for 30 minutes. Then, the cells were washed three times with PBS(-) and then stained with crystal violet for 30 minutes. Then, the degree of staining was measured using a plate reader at 550 nm (BIO-RAD, Model 680 MICRO PLATE READER) (Figures 4A and 4B). The results showed that the anti-rat Exon-17 polyclonal antibody is an antibody having the activity of inhibiting PN-1 protein-induced detachment of adhered cells, i.e., inhibiting the anti-cell adhesive properties of PN-1 protein, i.e., neutralizing the anti-cell adhesive properties of rat PN-1 protein.

### <Example 5> In vitro study of the effect of anti-rat Exon-17 polyclonal antibody on cell proliferation

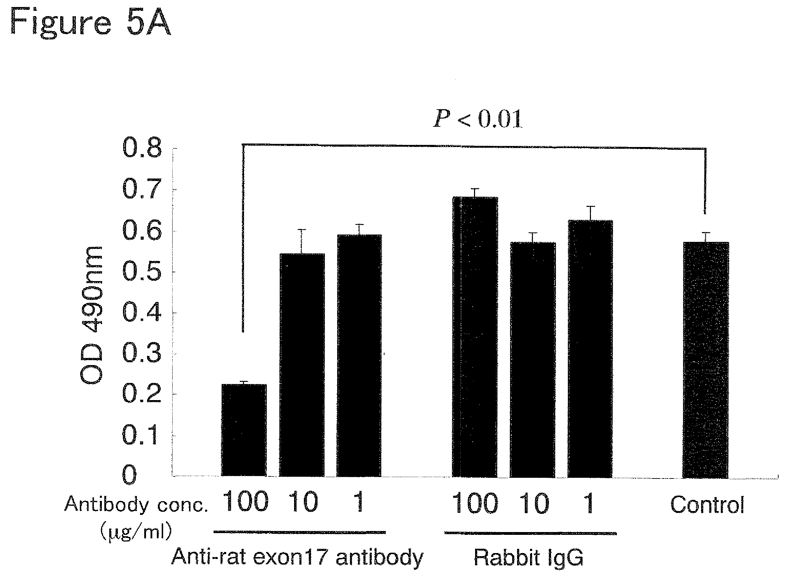

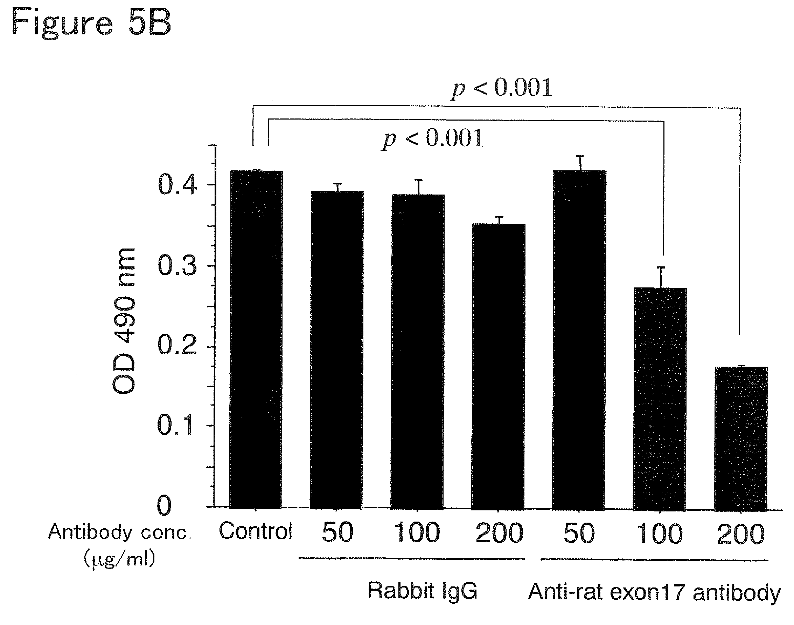

In the same manner as shown in Example 3, to a 96-well cell culture multiwell plate, mouse melanoma B16-F10 cells suspended in DMEM (serum free, PC/SM) were added at 10⁴ cells/well and cultured overnight in a 37°C incubator. After removal of the culture supernatant, DMEM (serum free, PC/SM) medium supplemented with anti-rat Exon-17 polyclonal antibody and rabbit IgG antibody, each at a final concentration of 100 µg/ml, 10 µg/ml or 1 µg/ml, was added to the cells, and overnight culturing was repeated again. On the following day, the medium in each well was replaced by DMEM (10% BSA, PC/SM), and a Cell Titer 96 AQueous One Solution Cell Proliferation Assay kit (Promega) was used to add 20 µl Cell Titer solution per 100 µl medium, followed by incubation at 37°C for 1 hour. Then, the degree of staining was measured using a plate reader at 490 nm (BIO-RAD, Model 680 MICRO PLATE READER) (Figure 5A). The same procedure was also repeated for mouse 4T1 breast cancer cells by addition of anti-rat Exon-17 polyclonal antibody and rabbit IgG antibody, each at a final concentration of 200 µg/ml, 100 µg/ml or 50 µg/ml (Figure 5B). The results showed that the anti-rat Exon-17 polyclonal antibody is an antibody having the activity of inhibiting cell proliferation at high concentration.

### <Example 6> Preparation of monoclonal antibody against human periostin Exon-17 peptide chain

### (1) Antigen preparation

A peptide (antigen peptide; SEQ ID NO: 25) having a Cys residue added to the N-terminus of the amino acid sequence encoded by human periostin Exon-17 (SEQ ID NO: 4) was chemically synthesized by the Fmoc method to obtain the antigen peptide in 10 mg yield at a purity of 90% or more. As a carrier protein, KLH (5 mg, CALBIOCHEM) was coupled to this antigen peptide to give an antigen solution. Namely, KLH was dissolved in PBS (0.01 M) and adjusted to 3.3 mg/mL, to which a 0.2524 mg/mL MBS solution (GE Healthcare Bio-Sciences KK) was then added dropwise and reacted with stirring at room temperature for 60 minutes. Dichloromethane was used to remove free MBS, to thereby obtain KLH-MB. This KLH-MB (5 mg) was mixed with the antigen peptide (5 mg) dissolved in 0.01 M sodium phosphate buffer (pH 7.2) and reacted with stirring at 4°C for 12 hours to obtain the antigen solution.

### (2) Immunization

Three female BALB/c mice at 6 weeks of age were each subcutaneously inoculated in both paws with the whole volume of a mixed emulsion of the antigen solution (50 µl) containing 100 µg KLH-coupled antigen peptide obtained in (1) and FCA (Freund's complete adjuvant, 50 µl). The mice were then inoculated twice in both paws with an *in situ* prepared mixed emulsion of the above antigen solution and FIA (Freund's incomplete adjuvant) at an interval of 2 weeks. The mice were then sacrificed by cervical dislocation and lymph nodes in their paws were aseptically collected. While supplying RPMI medium (Kohjinbio Co., Ltd.), the above lymph nodes were crushed and passed through a mesh of about 10 µm pore size to obtain lymph node cells suspended in RPMI medium. This suspension was centrifuged at 1000 rpm for 10 minutes to obtain lymph node cells as a pellet fraction. After this pellet fraction was hemolyzed to remove red blood cells in a solution (1 ml) prepared by adding 20 mM HEPES buffer (pH 7.4) to a 0.84% ammonium chloride solution, centrifugation was repeated at 1,000 rpm for 5 minutes. The resulting pellet fraction (cell fraction) was washed several times with RPMI medium and then used for cell fusion.

### (3) Preparation of myeloma cells

The mouse myeloma cell line P3X63Ag8U.1 (P3U1), which is resistant to 8-azaguanine and secretes no immunoglobulin, was cultured in RPMI medium containing 20% fetal calf serum (FCS) in a 10% CO₂, 37°C incubator. Cells in the logarithmic growth phase were collected and centrifuged at 1,000 rpm for 5 minutes to obtain the cells alone as a pellet fraction, which were then suspended in RPMI medium.

### (4) Cell fusion

The RPMI medium obtained in (2) containing 10⁸ to 3 x 10⁸ immunized lymph node cells and the RPMI medium obtained in (3) containing 10⁸ myeloma cells were mixed and then centrifuged at 1,000 rpm for 10 minutes. The supernatant was gently removed to obtain the cells as a pellet fraction, followed by addition of 1 ml of 25% (w/v) polyethylene glycol 1500 (PEG 1500, Boehringer). The cells were further diluted to a total volume of 10 ml by slow addition of RPMI medium. To this suspension, 20% FCS-containing RPMI medium (10 ml) was added and allowed to stand for a while, followed by centrifugation at 1,000 rpm for 5 minutes. The resulting pellet fraction (cell fraction) was adjusted to a cell density of 10⁶ cells/ml by addition of 20% FCS-containing RPMI, and this cell suspension was dispensed at 200 µl/well in 96-well culture plates (Corning). After culturing in a 5% CO₂, 37°C incubator for 24 hours, HAT solution (Invitrogen) was added and culturing was continued for an additional 2 weeks.

### (5) Screening by ELISA

Screening was performed to determine positive wells showing a reaction between the culture supernatant and the antigen peptide.

For use as an antigen solution for assay, the antigen peptide (2 mg) obtained in (1) was coupled to ovalbumin (OVA) as a carrier protein to prepare a conjugate.

Each well of a 96-well microtiter plate (Falcon 353912) was coated with the above conjugate (1 µg/ml) by standing overnight at 4°C. After washing this plate, the culture supernatant from (4) (50 µl, containing monoclonal antibodies) was added dropwise to each well and allowed to stand in a 37°C incubator for 2 hours, followed by washing with PBS(-) (phosphate buffered saline). After addition of alkaline phosphatase-conjugated sheep anti-mouse IgG antibody (Zymed), the plate was allowed to stand in a 37°C incubator for 1 hour, washed with PBS(-) and then color developed for 20 minutes by addition of a color development substrate (ALP). The absorbance (antibody titer) at OD 490 nm was measured for each well with a plate reader (BIO-RAD, Model 680 MICRO PLATE READER) to confirm its reactivity with the antigen peptide, to thereby determine positive wells showing a reaction between the culture supernatant and the antigen peptide.

### (6) Cloning of antibody-producing cells

Cells in the positive wells whose reactivity with the antigen peptide was confirmed by ELISA in (5) were provided for cloning of antibody-producing cell lines by limiting dilution. Namely, cells in the positive wells were plated into each well of a 96-well culture plate and cultured in a 5% CO₂, 37°C incubator for 2 weeks. In the same manner as used in (5), reactivity with the antigen peptide was confirmed by ELISA for the culture supernatant in each well, and cloning by limiting dilution was repeated again for each positive well to obtain 30 cells having a high reactivity with the antigen peptide and showing good colony growth. These cells were transferred to 24-well culture plates and cultured in a 5% CO₂, 37°C incubator for 2 weeks. In the same manner as used in (5), reactivity with the antigen peptide (antibody titer) was confirmed again by ELISA for each culture supernatant. Cells in 10 wells showing a high absorbance at OD 490 nm, i.e., 10 hybridoma cell lines were determined to be useful as antibody-producing cells and were selected.

[Table 1]

**Table 1 Hybridoma cell lines**

| No. | OD value |
|---|---|
| 1 | 0.41 |
| 2 | 0.37 |
| 3 | 0.68 |
| 4 | 0.24 |
| 5 | 0.33 |
| 6 | 0.32 |
| 7 | 0.33 |
| 8 | 0.32 |
| 9 | 0.12 |
| 10 | 0.3 |

Since the antibody-producing cells thus obtained always produce antibodies for use in the present invention, i.e., anti-human Exon-17 monoclonal antibodies, the supernatant of the culture in which these antibody-producing cells were cultured can be directly used as the antibody solution of the present invention. It is to be noted that the above antibody-producing cell line (hybridoma) No. 1 (SBM337), which produces anti-human Exon-17 monoclonal antibody, was deposited under FERM BP-10718 on November 1, 2006 with the International Patent Organism Depositary, the National Institute of Advanced Industrial Science and Technology.

### (7) Confirmation of binding capacity to human periostin protein (PN-1)

Antibodies produced by the 10 antibody-producing cell lines obtained in (6) were confirmed for their binding capacity to human periostin protein (PN-1) by dot blotting. Namely, the synthetic protein obtained in Preparation examples 4 to 6 (30 µg/ml) was spotted in 5 µl volumes on a Hybond-ECL nitrocellulose membrane (GE Healthcare Bio-Sciences KK) and washed once with TBS solution (10 mM Tris-HCl (pH 8.0), 150 mM NaCl). Blocking buffer (Block Ace, Snow Brand Milk Products Co., Ltd.) was added and shaken at room temperature for 1 hour. After a 1 µg/ml solution of each monoclonal antibody (primary antibody) obtained in (6) was added to the membrane and shaken for 3 hours, the membrane was washed four times with TBS solution under shaking for 10 minutes. After a 0.4 µg/ml solution of an HRP-labeled anti-mouse IgG antibody (Promega) (secondary antibody) was added to the membrane and shaken at room temperature for 1 hour, the membrane was washed four times with TBS solution under shaking for 10 minutes. Detection reagents (ECL plus western blotting detection system, GE Healthcare Bio-Sciences KK) were added and reacted for 1 minute to detect chemiluminescence. As a result, it was confirmed that all of the 10 antibody-producing cell lines cloned in (6) bind to human periostin PN-1.

### (8) Mass production and purification of monoclonal antibody

BALB/c mice were intraperitoneally administered with pristane [2,6,10,14-tetramethyl pentadecane (0.5 ml, Wako Pure Chemical Industries, Ltd.)] and kept for 2 to 3 weeks. The monoclonal antibody-producing hybridomas No. 1 and No. 3 which had been maintained at the logarithmic growth phase were collected and centrifuged to remove the culture supernatant. To the cells in each pellet fraction, FCS-free RPMI medium was added to prepare a cell suspension at a cell density of 1 x 10⁷ cells/ml. This cell suspension was intraperitoneally inoculated into the BALB/c mice pretreated with pristane and, after about three weeks, the exuded ascites fluid was collected from the abdominal region by a syringe. After each collected ascites fluid was filtered using a filter with a pore size of 0.22 µmφ, the filtrates were purified in a routine manner by affinity chromatography on a Protein G-sepharose column (Millipore, 11511324) to prepare two anti-human Exon-17 monoclonal antibodies.

### <Example 7> Recognition site analysis of anti-human Exon-17 monoclonal antibody in human periostin Exon-17 peptide chain

The resulting two monoclonal antibodies (No. 1 and No. 3) were analyzed for their recognition sites in the human periostin Exon-17 peptide chain (epitope identification). Namely, based on an amino acid sequence consisting of 45 amino acids in total between the -9th phenylalanine from the N-terminus and the 9th isoleucine from the C-terminus of the human periostin Exon-17 peptide chain (SEQ ID NO: 4; the 1st threonine up to the 27th glutamic acid), the following 36 peptides composed of 10 amino acids were synthesized on a cellulose membrane to prepare a membrane-bound peptide array (custom SPOTs service of Sigma-Aldrich Japan K.K.).

### [Formula 1]

- 1: FKEIPVTVYT
- 2: KEIPVTVYTT
- 3: EIPVTVYTTK
- 4: IPVTVYTTKI
- 5: PVTVYTTKII
- 6: VTVYTTKIIT
- 7: TVYTTKIITK
- 8: VYTTKIITKV
- 9: YTTKIITKVV
- 10: TTKIITKVVE
- 11: TKIITKVVEP
- 12: KIITKVVEPK
- 13: IITKVVEPKI
- 14: ITKVVEPKIK
- 15: TKVVEPKIKV
- 16: KVVEPKIKVI
- 17: VVEPKIKVIE
- 18: VEPKIKVIEG
- 19: EPKIKVIEGS
- 20: PKIKVIEGSL
- 21: KIKVIEGSLQ
- 22: IKVIEGSLQP
- 23: KVIEGSLQPI
- 24: VIEGSLQPII
- 25: IEGSLQPIIK
- 26: EGSLQPIIKT
- 27: GSLQPIIKTE
- 28: SLQPIIKTEG
- 29: LQPIIKTEGP
- 30: QPIIKTEGPT
- 31: PIIKTEGPTL
- 32: IIKTEGPTLT
- 33: IKTEGPTLTK
- 34: KTEGPTLTKV
- 35: TEGPTLTKVK
- 36: EGPTLTKVKI

This membrane was allowed to stand in a small volume of methanol for 5 minutes and was then washed three times with TBS solution. Blocking buffer (casein, included in SPOTs) was added and stirred at room temperature for 2 hours. After a 1 µg/ml solution of each monoclonal antibody (primary antibody) obtained in Example 6(8) was added to the membrane and shaken for 3 hours, the membrane was washed three times in TBS solution under shaking for 10 minutes. After a 0.4 µg/ml solution of an HRP-labeled anti-mouse IgG antibody (Promega) (secondary antibody) was added to the membrane and incubated for 2 hours, the membrane was washed three times with TBS solution under shaking for 5 minutes. Detection reagents (SuperSignal West Pico, Pierce) were added and reacted for 1 minute to detect chemiluminescence. As a result, monoclonal antibodies No. 1 and No. 3 were found to react with and bind to only synthetic peptide No. 9 consisting of the amino acid sequence YTTKIITKVV (SEQ ID NO: 26), i.e., a peptide consisting of an amino acid sequence covering from the -1st tyrosine to the 9th valine from the N-terminus of the amino acid sequence of the human periostin Exon-17 peptide chain (SEQ ID NO: 4) or covering from the 669th tyrosine to the 679th valine from the N-terminus of the amino acid sequence of human periostin PN-1 (SEQ ID NO: 2).

### <Example 8> Recognition site analysis of anti-rat Exon-17 polyclonal antibody in human periostin Exon-17 peptide chain

In the same manner as shown in Example 7, the polyclonal antibody prepared in Example 1 was analyzed for its recognition site in the human periostin Exon-17 peptide chain (epitope identification). As a result, as in the case of the monoclonal antibodies in Example 7, the polyclonal antibody was found to react with only synthetic peptide No. 9, indicating that the polyclonal antibody specifically recognizes the same site as the monoclonal antibodies. This suggests that antibodies having the same specificity are obtainable in both cases where a rat periostin Exon-17 peptide is used as an antigen to prepare a polyclonal antibody and where a human periostin Exon-17 peptide is used as an antigen to prepare a monoclonal antibody.

### <Example 9> Conformation of binding capacity to rat periostin protein (PN-1)

The resulting two monoclonal antibodies (No. 1 and No. 3) were confirmed for their binding capacity to rat periostin protein (PN-1) by dot blotting. Namely, the purified protein obtained in Preparation example 6 (30 µg/ml) was spotted in 5 µl volumes on a Hybond-ECL nitrocellulose membrane (GE Healthcare Bio-Sciences KK) and washed once with TBS solution (10 mM Tris-HCl (pH 8.0), 150 mM NaCl). Blocking buffer (Block Ace, Snow Brand Milk Products Co., Ltd.) was added and shaken at room temperature for 1 hour. After a 1 µg/ml solution of each monoclonal antibody (primary antibody) was added to the membrane and shaken for 3 hours, the membrane was washed four times with TBS solution under shaking for 10 minutes. After a 0.4 µg/ml solution of an HRP-labeled anti-mouse IgG antibody (Promega) (secondary antibody) was added to the membrane and shaken at room temperature for 1 hour, the membrane was washed four times with TBS solution under shaking for 10 minutes. Detection reagents (ECL plus western blotting detection system, GE Healthcare Bio-Sciences KK) were added and reacted for 1 minute to detect chemiluminescence. As a result, it was confirmed that the resulting two monoclonal antibodies also bind to rat periostin PN-1.

### <Example 10> Epitope analysis of anti-human Exon-17 monoclonal antibody

The results of Example 7 indicated that the epitope part of each anti-human Exon-17 monoclonal antibody recognizes an amino acid sequence (TTKIITKVV; SEQ ID NO: 22) covering from the N-terminal threonine to the 9th valine of the human periostin Exon-17 peptide chain (SEQ ID NO: 4). Likewise, the results of Example 9 confirmed that each anti-human Exon-17 monoclonal antibody also binds to rat periostin protein (PN-1). These results suggested that the epitope part of each anti-human Exon-17 monoclonal antibody recognizes a region, whose amino acids do not differ between humans and rats, in the amino acid sequence (TTKIITKVV; SEQ ID NO: 22) covering from the N-terminal threonine to the 9th valine of the human periostin Exon-17 peptide chain (SEQ ID NO: 4), i.e., the entire amino acid sequence, or a part thereof, which covers from the N-terminal threonine to the 7th lysine of the human periostin Exon-17 peptide chain (SEQ ID NO: 4) or the rat periostin Exon-17 peptide chain (SEQ ID NO: 3). Thus, for further analysis of the epitope part, alanine scanning was performed.

Based on an amino acid sequence (YTTKIITKVV; SEQ ID NO: 26) covering from the -1st tyrosine to the 9th valine from the N-terminus of the human periostin Exon-17 peptide chain (SEQ ID NO: 4), the following 10 peptides modified to replace some amino acids by alanines were synthesized at a purity of 80% or more.

### [Formula 2]

#1 YTTKIITKVV
#2 ATTKIITKAA
#3 AATKIITKAA
#4 AAAKIITKAA
#5 AAAAIITKAA
#6 AAAAAITKAA
#7 ATTKIITAAA
#8 ATTKIIAAAA
#9 ATTKIAAAAA
#10 ATTKAAAAAA

The synthetic peptides (1 mg each) were solubilized with 50 µl PBS(-), spotted in 1.5 µl volumes on a Hybond-ECL nitrocellulose membrane (GE Healthcare Bio-Sciences KK) and washed once with TBS solution. Blocking buffer (Block Ace, Snow Brand Milk Products Co., Ltd.) was added and shaken at room temperature for 1 hour. After a 1 µg/ml solution of each monoclonal antibody (primary antibody) obtained in Example 6(8) was added to the membrane and shaken for 3 hours, the membrane was washed four times with TBS solution under shaking for 10 minutes. After a 0.4 µg/ml solution of an HRP-labeled anti-mouse IgG antibody (Promega) (secondary antibody) was added to the membrane and shaken at room temperature for 1 hour, the membrane was washed four times with TBS solution under shaking for 10 minutes. Detection reagents (SuperSignal West Pico, Pierce) were added and reacted for 1 minute to detect chemiluminescence.

As a result, the monoclonal antibodies were found to strongly react with synthetic peptide #7 shown above (a peptide comprising alanine substitutions at the 1st and the 8-10th amino acids from the N-terminus of a peptide consisting of an amino acid sequence (YTTKIITKVV; SEQ ID NO: 26) covering from the -1st tyrosine to the 9th valine from the N-terminus of the human periostin Exon-17 peptide chain (SEQ ID NO: 4)), weakly react with synthetic peptides #1 (a peptide consisting of an amino acid sequence (YTTKIITKVV; SEQ ID NO: 26) covering from the -1st tyrosine to the 9th valine from the N-terminus of the human periostin Exon-17 peptide chain (SEQ ID NO: 4)) and #2 (a peptide comprising alanine substitutions at the 1st and the 9-10th amino acids from the N-terminus of a peptide consisting of an amino acid sequence (YTTKIITKVV; SEQ ID NO: 26) covering from the -1st tyrosine to the 9th valine from the N-terminus of the human periostin Exon-17 peptide chain (SEQ ID NO: 4)), and more weakly react with synthetic peptides #3 (a peptide comprising alanine substations at the 1st, the 2nd and the 9-10th amino acids from the N-terminus of a peptide consisting of an amino acid sequence (YTTKIITKVV; SEQ ID NO: 26) covering from the -1st tyrosine to the 9th valine from the N-terminus of the human periostin Exon-17 peptide chain (SEQ ID NO: 4)) and #8 (a peptide comprising alanine substitutions at the 1st and the 7-10th amino acids from the N-terminus of a peptide consisting of an amino acid sequence (YTTKIITKVV; SEQ ID NO: 26) covering from the -1st tyrosine to the 9th valine from the N-terminus of the human periostin Exon-17 peptide chain (SEQ ID NO: 4)).

### <Example 11> Recognition site analysis of anti-rat Exon-17 polyclonal antibody in human periostin Exon-17 peptide chain

In the same manner as shown in Example 10, the polyclonal antibody prepared in Example 1 was analyzed for its recognition site in the human periostin Exon-17 peptide chain (epitope identification). As a result, as in the case of the monoclonal antibodies in Example 10, the polyclonal antibody was found to strongly react with synthetic peptide No. #7, weakly react with synthetic peptides #1 and #2, and still more weakly react with synthetic peptides #3 and #8, indicating that the polyclonal antibody specifically recognizes the same site as the monoclonal antibodies. This suggests that antibodies having the same specificity are obtainable in both cases where a rat periostin Exon-17 peptide is used as an antigen to prepare a polyclonal antibody and where a human periostin Exon-17 peptide is used as an antigen to prepare a monoclonal antibody.

### <Example 12> Expression of periostin isoform having a peptide region encoded by Exon-17 in primary tumor using model mice for lung metastasis of mouse melanoma B16-F10 cells

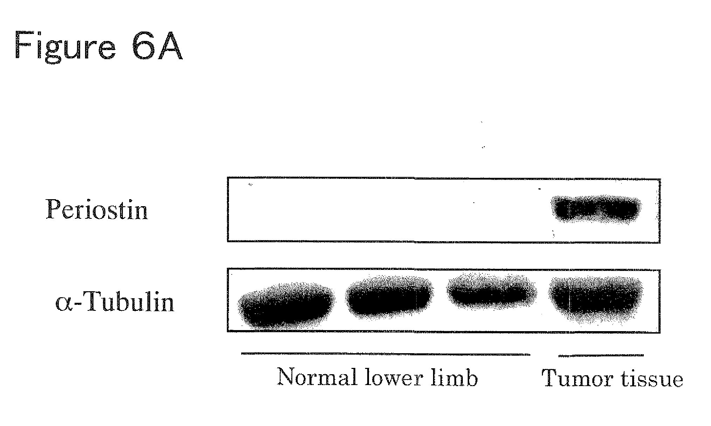

At 2 weeks after inoculation of mouse melanoma B16-F10 cells, mouse primary tumors were collected and the homogenized tissue was allowed to stand on ice for 15 minutes after addition of RIPA buffer (RIPA Lyses Buffer 10×; Upstate), 180 mM Na₃VO₄, protease inhibitor cocktail (Nacalai Tesque, Inc.) and 200 mM NaF. The homogenate was then centrifuged at 15000 rpm, 4°C for 20 minutes to collect the supernatant. The extracted proteins were measured for their concentration using DC protein assay reagents (BIO-RAD). Then, the proteins were mixed into 2× sample buffer (Laemmli Sample Buffer (BIO-RAD), 5% 2-mercaptoethanol) and treated by heating at 98°C for 5 minutes. The prepared protein sample was electrophoresed at 10 mA for 180 minutes using Multigel II mini 7.5 (Daiichi Pure Chemicals Co., Ltd.) and then transferred overnight onto an Immobilon-P Transfer Membrane (MILLIPORE) at 30 V and 4°C. Then, the membrane was soaked in 5% skimmed milk in PBS-T for 1 hour or in Blocking One-P (Nacalai Tesque, Inc.) for 20 minutes and shaken at room temperature for blocking. Then, a primary antibody (anti-human Exon-17 monoclonal antibody (No. 3) at 1:500 dilution; anti-avian α-tubulin monoclonal IgG antibody (Sigma) at 1:5000 dilution as a control) was added and reacted overnight at 4°C, followed by washing three times in PBS-T for 5 minutes. Then, a secondary antibody (Anti mouse IgG HRP (PROMEGA) at 1:10000 dilution) was added and reacted for 1 hour at room temperature. The membrane was washed in PBS-T once for 10 minutes and then twice for 30 minutes, followed by chemiluminescence band detection using an ECL-Plus (Amersiam Biosciences). The results indicated that normal lower limbs showed no expression of a periostin isoform having a peptide region encoded by Exon-17, whereas the tumor tissue showed enhanced expression (Figure 6 A).

### <Example 13> Expression of periostin isoform having a peptide region encoded by Exon-17 in primary tumor using model mice for lung metastasis of mouse 4T1 breast cancer cells

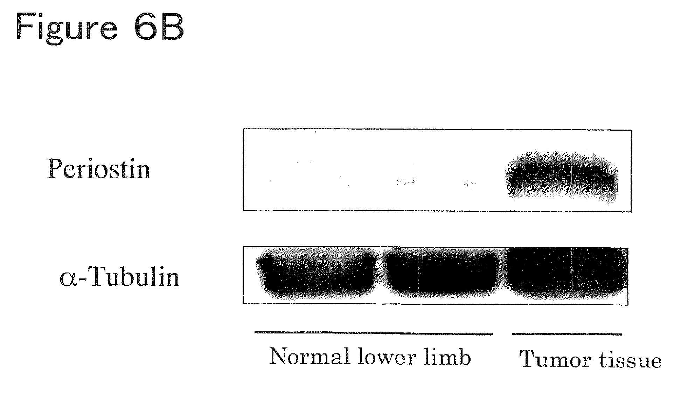

At 2 weeks after inoculation of mouse 4T1 breast cancer cells, mouse primary tumors were collected and the homogenized tissue was allowed to stand on ice for 15 minutes after addition of RIPA buffer (RIPA Lyses Buffer 10×; Upstate), 180 mM Na₃VO₄, protease inhibitor cocktail (Nacalai Tesque, Inc.) and 200 mM NaF. The homogenate was then centrifuged at 15000 rpm, 4°C for 20 minutes to collect the supernatant. The extracted proteins were measured for their concentration using DC protein assay reagents (BIO-RAD). Then, the proteins were mixed into 2x sample buffer (Laemmli Sample Buffer (BIO-RAD), 5% 2-mercaptoethanol) and treated by heating at 98°C for 5 minutes. The prepared protein sample was electrophoresed at 10 mA for 180 minutes using Multigel II mini 7.5 (Daiichi Pure Chemicals Co., Ltd.) and then transferred overnight onto an Immobilon-P Transfer Membrane (MILLIPORE) at 30 V and 4°C. Then, the membrane was soaked in 5% skimmed milk (in PBS-T) for 1 hour or in Blocking One-P (Nacalai Tesque, Inc.) for 20 minutes and shaken at room temperature for blocking. Then, a primary antibody (anti-human Exon-17 monoclonal antibody (No. 3) at 1:500 dilution; anti-avian α-tubulin monoclonal IgG antibody (Sigma) at 1:5000 dilution as a control) was added and reacted overnight at 4°C, followed by washing three times in PBS-T for 5 minutes. Then, a secondary antibody (Anti mouse IgG HRP (PROMEGA) at 1:10000 dilution) was added and reacted for 1 hour at room temperature. The membrane was washed in PBS-T once for 10 minutes and then twice for 30 minutes, followed by chemiluminescence band detection using an ECL-Plus (Amersiam Biosciences). The results indicated that normal lower limbs showed no periostin expression, whereas the tumor tissue showed enhanced expression (Figure 6B).

### <Example 14> Effect of anti-rat Exon-17 polyclonal antibody and anti-human Exon-17 monoclonal antibody using model mice for lung metastasis of mouse melanoma B16-F10 cells

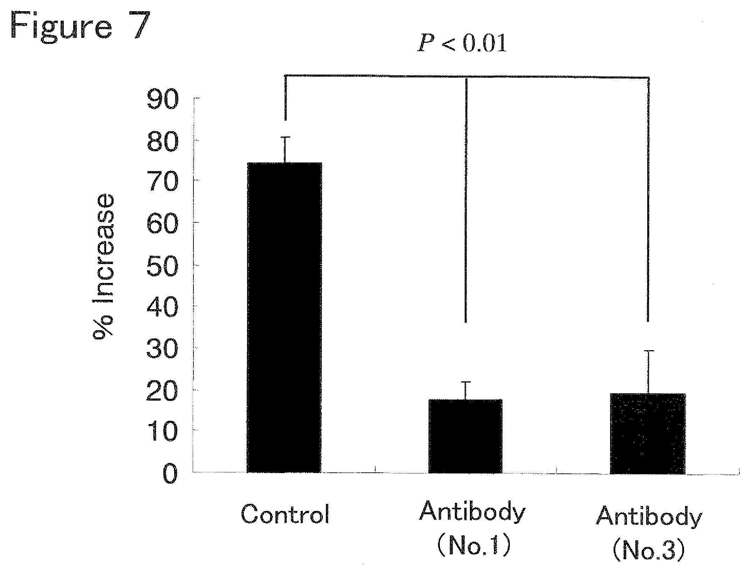

Mouse melanoma B16-F10 cells were cultured in a 37°C incubator, washed with PBS and then treated with trypsin/EDTA to float and collect the cells. Then, the cells collected by centrifugation at 1500 rpm for 3 minutes were counted to give 5 × 10⁵ cells/animal and suspended in 100 µl PBS. The adjusted cells were inoculated into the foot pad of C57BL/6N male mice (8 weeks of age) using an insulin syringe equipped with a 29G Myjector injection needle (TERMO). First of all, to study the effect of the two monoclonal antibodies (No. 1 and No. 3) obtained in Example 6, these antibodies (2 µg/animal) were each administered to the mice via the jugular vein through an insulin syringe equipped with a 29G Myjector injection needle, simultaneously with the inoculation of the cells. As a control, Normal Rabbit IgG (R&D Systems) was used. At one week after administration of the cells and antibodies, the diameter of swelling lesions in the lower limbs was determined with a caliper to evaluate % increase in primary tumor size. As a result, the % increase in primary tumor size was 74.5 ± 6.3% (n=11) in the control group, 17.8 ± 4.4% (n=10) in the antibody (No. 1) group and 19.5 ± 9.9% (n=10) in the antibody (No. 3) group, indicating that both antibodies have the same inhibitory activity on cancer growth (Figure 7).

Next, subsequent to the inoculation of mouse melanoma B16-F10 cells, antibody administration was started after a given period of time to study whether the antibody has an effect under conditions much closer to clinical cases. At one week after cell inoculation for the experiment using anti-rat Exon-17 polyclonal antibody (0.75 mg/ml) and at 3 and 7 days after cell inoculation for the experiment using anti-human Exon-17 monoclonal antibody (No. 3) (2.14 mg/ml), each antibody (20 µg/animal) was administered to the mice via the jugular vein through an insulin syringe equipped with a 29G Myjector injection needle. As a control, Normal Rabbit IgG (R&D Systems) was used. After cell inoculation, the diameter of swelling lesions in the lower limbs was determined weekly with a caliper to evaluate % increase in primary tumor size. To avoid overgrowth of primary tumors which causes direct cancer invasion into the subperitoneal space and leads to death of the mice, lower limbs with primary tumors were excised at 2 weeks after cell inoculation. At 5 weeks after cell inoculation, autopsy was performed to determine the presence or absence of metastasis from primary tumor to lung and the number of lung metastases. The % increase in primary tumor size was expressed as a percentage of increment, assuming that the limb diameter before inoculation of mouse melanoma B16-F10 cells was set to 100, and data analysis was made by the Student's t-test. Likewise, the metastasis rate from primary tumor to lung was analyzed by the χ² test, while the number of lung metastases was analyzed by the Mann-Whitney test.

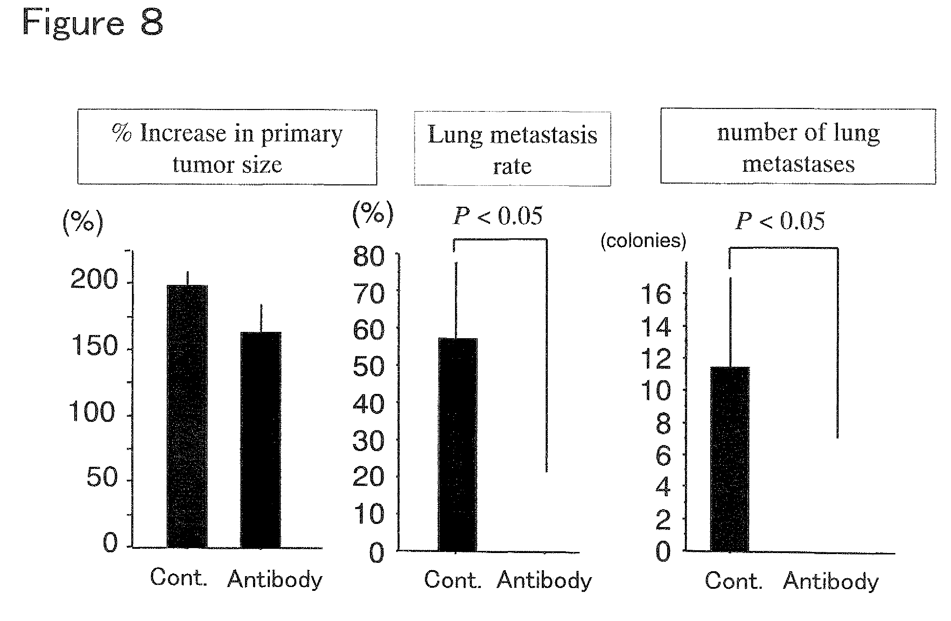

In the experiment where the anti-rat Exon-17 polyclonal antibody was administered, the % increase in primary tumor size at 2 weeks after cell inoculation (in comparison with the limb diameter before cell inoculation) was 198.1 ± 10.428% (n=10) in the control group and 163.2 ± 21.015% (n=11) in the neutralizing antibody group (Figure 8). On the other hand, the metastasis rate from primary tumor to lung was 57.1 % (n=7) in the control group and 0% (n=6) in the neutralizing antibody group, indicating that the metastasis was significantly inhibited in the neutralizing antibody group (Figure 8). The number of lung metastases was 11.43 ± 0.553 in the control group (Figure 8).

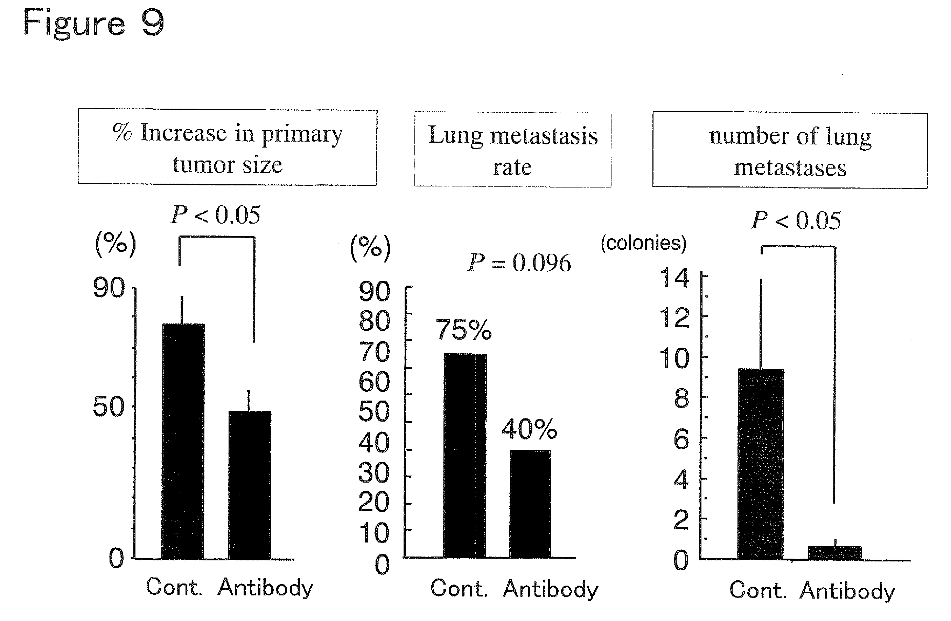

Next, in the experiment where the anti-human Exon-17 monoclonal antibody (No. 3) was administered, the % increase in primary tumor size at 1 week after cell inoculation (in comparison with the limb diameter before cell inoculation) was 77.6 ± 9.484% (n=12) in the control group and 48.9 ± 7.060% (n=11) in the neutralizing antibody group, indicating that primary tumor growth was significantly inhibited in the neutralizing antibody group (P<0.05) (Figure 9). Likewise, the metastasis rate from primary tumor to lung was 75% (n=12) in the control group and 40% in the neutralizing antibody group (n=10), indicating that the metastasis rate tended to decrease in the neutralizing antibody group (P=0.0964) (Figure 9). The number of lung metastases was 9.41 ± 4.38 in the control group and 0.7 ± 0.335 in the neutralizing antibody group, indicating that the number of lung metastases was significantly inhibited in the neutralizing antibody group (P<0.05) (Figure 9). These results showed that the anti-rat Exon-17 polyclonal antibody has an inhibitory effect on lung metastasis of melanoma cells. It was further found that the anti-human Exon-17 monoclonal antibody (No. 3) also has an inhibitory effect on primary tumor growth.

### <Example 15> Effect of anti-rat Exon-17 polyclonal antibody using model mice for lung metastasis of mouse 4T1 breast cancer cells

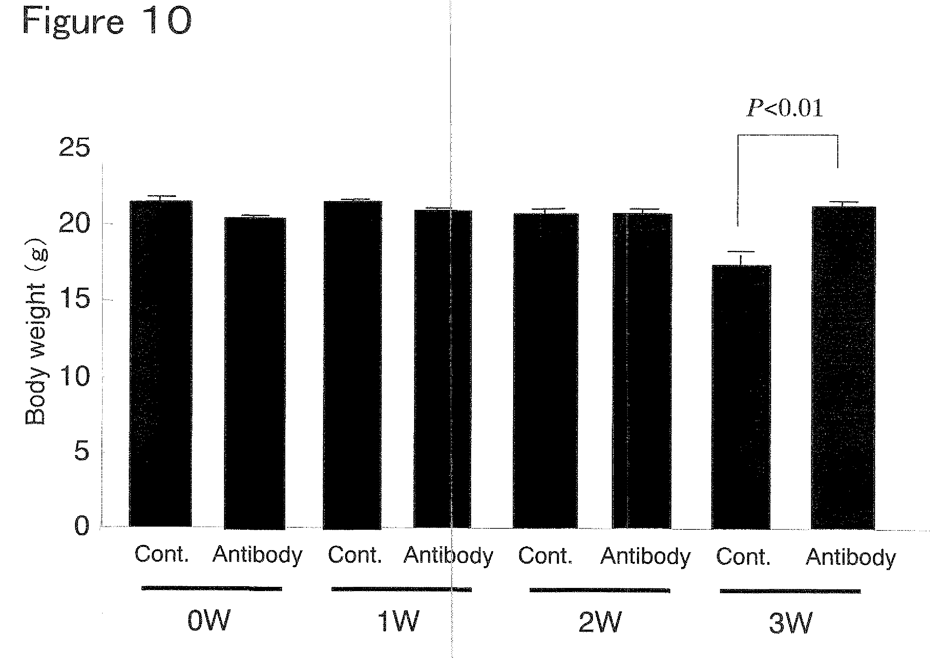

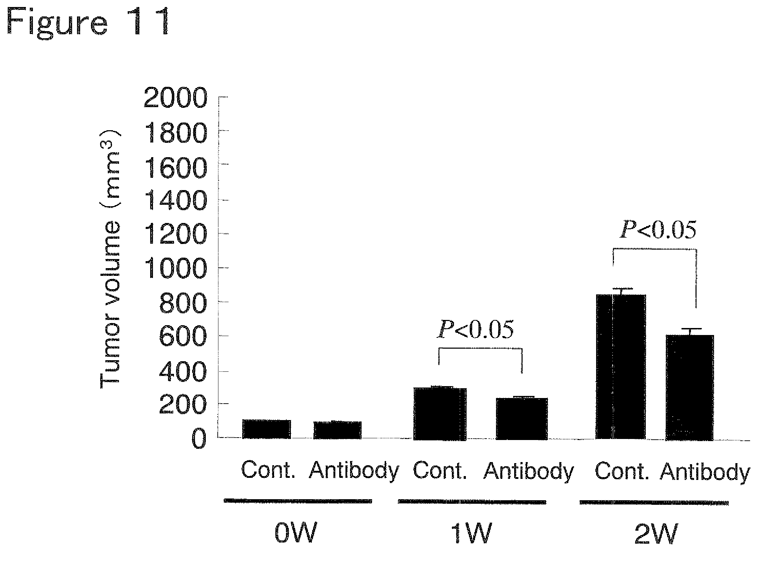

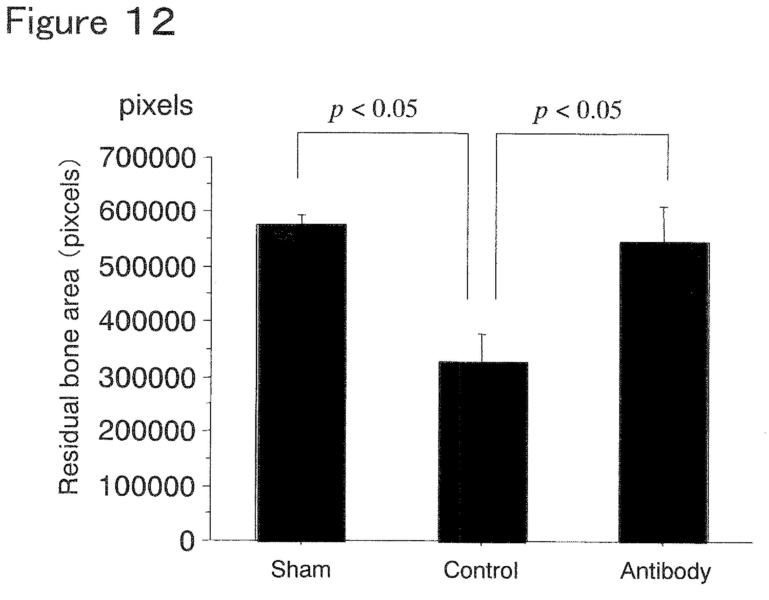

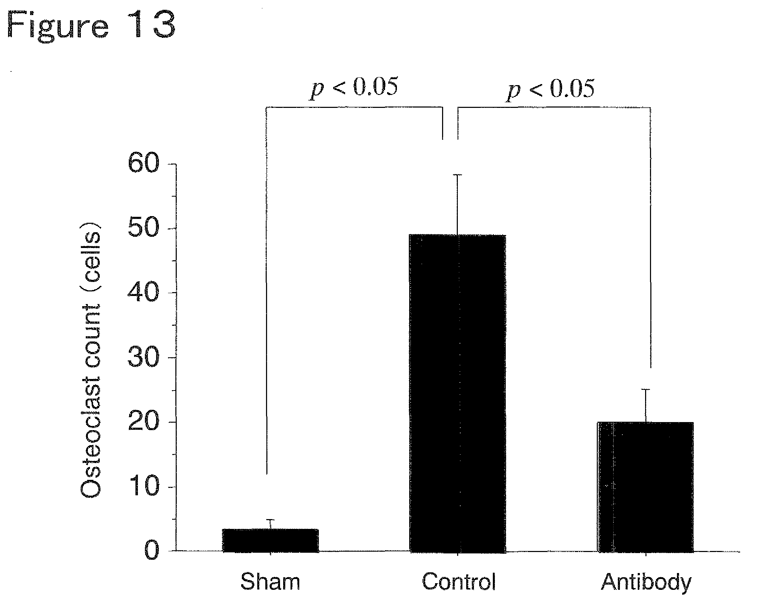

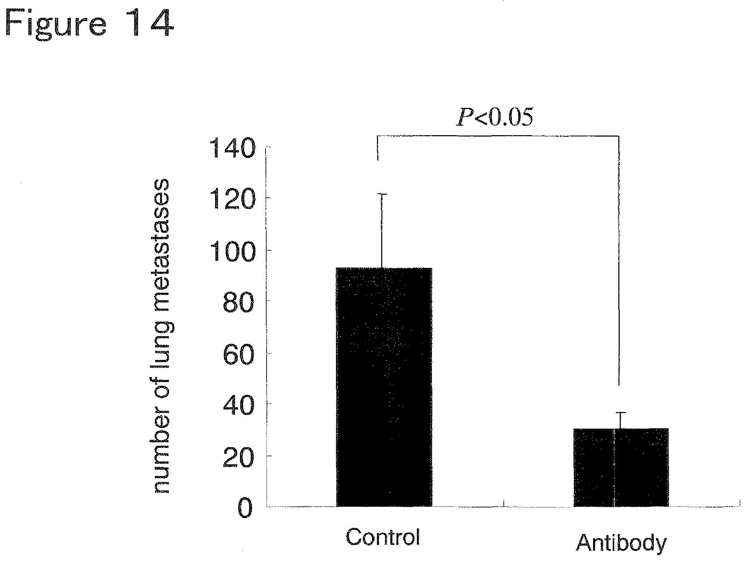

Mouse 4T1 breast cancer cells were seeded in 10 cm Tissue Culture Dishes (Greiner) using RPMI1640 (Gibco) containing 10% bovine serum albumin (FBS) (Bio west) and a Penicillin-streptomycin Mixed solution (Nacalai Tesque, Inc.), and then cultured in a 37°C incubator for 24 hours. After removal of the culture supernatant, the cells were washed with PBS and then allowed to float by treatment with trypsin/EDTA. The cells were collected and centrifuged at 1500 rpm for 3 minutes, 1.5 × 10⁵ of which were then subcultured in a 37°C incubator for 72 hours. Cells in the logarithmic growth phase were counted to give 1 x 10⁶ cells/animal and suspended in 100 µl PBS. The adjusted cells were inoculated into the foot pad of BALB/c female mice (8 weeks of age) using an insulin syringe equipped with a 29G Myjector injection needle (TERMO). An antibody (20 µg/animal) was also administered to the mice via the jugular vein through an insulin syringe equipped with a 29G Myjector injection needle. In the experiment using anti-rat Exon-17 polyclonal antibody (1 mg/ml), the antibody was administered simultaneously with cell inoculation, and further administered at 1 and 2 weeks after cell inoculation. As a control, Normal Rabbit IgG (R&D Systems) was used. After cell inoculation, the mice were weekly measured for their body weight and the diameter of swelling lesions in their lower limbs with a caliper to evaluate primary tumor volume. Evaluation was accomplished as described in Dethlefsen LA. et al., J. Natl. Cancer Inst., 40, 389 (1968) to determine the volume according to the following equation: (length) × (square of width)/2. At 3 weeks after cell inoculation, autopsy was performed to determine body weight, the presence or absence of metastasis from primary tumor to lung, and the number of lung metastases. Data analysis was made by the Student's t-test for each case. As a result, with respect to changes in body weight, there was no significant difference between both groups until 2 weeks after cell inoculation. After 3 weeks, however, the body weight was 17.50 ± 0.703 g (n=6) in the control group and 21.24 ± 0.517 g (n=6) in the neutralizing antibody group, indicating that primary tumor growth was significantly inhibited in the neutralizing antibody group (P<0.05) (Figure 10). Moreover, the tumor volume of primary tumor at 1 week after cell inoculation was 301.3 ± 11.49 mm³ (n=10) in the control group and 235.9 ± 7.842 mm³ (n=10) in the neutralizing antibody group, indicating that primary tumor growth was significantly inhibited in the neutralizing antibody group (P<0.05). Likewise, the tumor volume of primary tumor at 2 weeks after cell inoculation was 842.4 ± 34.71 mm³ (n=10) in the control group and 613.9 ± 45.17 mm³ (n=10) in the neutralizing antibody group, indicating that primary tumor growth was significantly inhibited in the neutralizing antibody group (P<0.05) (Figure 11). At 3 weeks after cell inoculation, the control group showed loss of limbs due to necrosis, whereas the neutralizing antibody group showed no loss of limbs. In addition, for evaluation of bone destruction caused by bone invasion of breast cancer cells from primary tumor, lower ankle bone was analyzed by HE staining and TRAP staining to determine the bone area and the number of osteoblasts. The residual bone area after direct invasion into bone was 326656 ± 53628.7 (n=5) in the control group and 545756.8 ± 65928.8 (n=5) in the neutralizing antibody group, indicating that the neutralizing antibody group showed a significantly greater amount of residual bone (p<0.05) (Figure 12). Further, the number of osteoclasts was inversely correlated with the residual bone area, and was 49 ± 9.576 (n=5) in the control group and 20 ± 5.167 (n=5) in the neutralizing antibody group, indicating that the number of osteoclasts was significantly reduced in the neutralizing antibody group (p<0.05) (Figure 13). With respect to the metastasis rate from primary tumor to lung, both groups showed metastasis, but the number of lung metastases was 89 ± 28.9 in the control group and 30.5 ± 6.30 in the neutralizing antibody group, indicating that the number of lung metastases was significantly inhibited in the neutralizing antibody group (P<0.05) (Figure 14). These results showed that the anti-rat Exon-17 polyclonal antibody not only inhibits primary tumor growth of breast cancer cells, but also has an inhibitory effect on lung metastasis of breast cancer cells.

### <Example 16> Expression study of periostin isoform having the Exon-17 region in human cancer cells and in normal tissues

First of all, primers specific to the Exon-17 region were prepared (sense strand: 5'-TAAAATTATAACCAAAGTTGTGGAACC-3' (SEQ ID NO: 35) and antisense strand: 5'-AGTGTGGGTCCTTCAGTTTTGAT-3' (SEQ ID NO: 36)).

Next, human cancer cell lines 1) to 6) listed below were purchased from ATCC (American Type Culture Collection) and ECACC (European Collection of Cell Cultures) and cultured according to the manufacturers' instructions for these cell lines, followed by extraction of total RNAs using ISOGEN (Nippon Gene) according to the manufacturer's instructions.
1) Breast Ductal Carcinoma (MDA-MB-435s) Cat. No. HTB-129
2) Breast Adenocarcinoma (MDA-MB-231) Cat. No. HTB-26
3) Breast Adenocarcinoma (MCF-7) Cat. No. HTB-22
4) Lung Carcinoma (A-549) Cat. No. CCL-185
5) Melanoma (SK-MEL-5) Cat. No. HTB-70
6) Breast carcinoma (Hs 578T) Cat. No. EC86082104

Likewise, total RNAs derived from human cancer cell lines 1) to 11) listed below were purchased from Ambion.
1) Leukemia (KG-1) Cat. No. AM7830
2) Prostate Adenocarcinoma (PC-3) Cat. No. AM7834
3) Osteogenic Sarcoma (SaOS-2) Cat. No. AM7840
4) Bladder Carcinoma (T-24) Cat. No. AM7844
5) Breast Adenocarcinoma (MCF-7) Cat. No. AM7846
6) Liver Carcinoma (HepG2) Cat. No. AM7848
7) Cervical Adenocarcinoma (HeLa-S3) Cat. No. AM7852
8) Breast Carcinoma (T-470) Cat. No. AM7874
9) Breast Carcinoma (MDA-MB-453) Cat. No. AM7876
10) Breast Carcinoma (DU-4475) Cat. No. AM7878
11) Breast Adenocarcinoma (MDA-MB-361) Cat. No. AM7872

Likewise, total RNAs derived from human cancer tissues 1) to 16) listed below were purchased from Ambion.
1) Lung: Adenocarcinoma Cat. No. AM7225
2) Pancreas: Acinar cell carcinoma Cat. No. AM7229
3) Colon: Adenocarcinoma Cat. No. AM7237
4) Liver: Hepatocellular CA Cat. No. AM7245
5) Bladder: transitional cell cancer Cat. No. AM7249
6) Kidney: Renal cell carcinoma Cat. No. AM7253
7) Stomach: Unknown Cat. No. AM7265
8) Breast: Ductal carcinoma Cat. No. AM7221
9) Uterus: Surgery Cat. No. AM7233
10) Thyroid: Hurthle cell neoplasm Cat. No. AM7241
11) Ovary: Papillary cystadenocarcinoma Cat. No. AM7257
12) Testis: Germ cell tumor Cat. No. AM7261
13) Lymphoma: Large B-cell lymphoma Cat. No. AM7269
14) Cervix: Cervical cancer Cat. No. AM7277
15) Prostate: Acinar adenocarcinoma Cat. No. AM7289
16) Lymph Node: Follicular Lymphoma Cat. No. AM7291

Likewise, total RNAs derived from human cancer tissues 1) to 11) listed below were purchased from Biochain.
1) Adrenal: Pheochromocytoma Cat. No. R1235004-10
2) Bone: Osteosarcoma Cat. No. R1235023-10
3) Breast: Lobular carcinoma Cat. No. R1235086-50
4) Esophagus: Squamous cell carcinoma Cat. No. R1235106-50
5) Lung: Adenocarcinoma Cat. No. R1235152-50
6) Ovary: Adenocarcinoma Cat. No. R1235183-10
7) Rectum: Adenocarcinoma Cat. No. R1235206-50
8) Skin: Skin melanoma Cat. No. R1235218A-10
9) Small Intestine: Malignancy mesothelioma Cat. No. R1235226-10
10) Soft Tissue: Synoviosarcoma Cat. No. R1235257-10
11) Thymus: Thymoma Cat. No. R1235264-10

Likewise, total RNAs derived from human normal tissues listed below were purchased from CLONTECH.
1) Adrenal Gland (Cat. No. 636528)
2) Bladder (Cat. No. 636542)
3) Peripheral Leukocytes (Cat. No. 6:36580)
4) Colon (Cat. No. 636553)
5) Kidney (Cat. No. 636529)
6) Liver (Cat. No. 636531)
7) Lung (Cat. No. 636524)
8) Mammary Gland (Cat. No. 636576)
9) Pancreas (Cat. No. 636577)
10) Prostate (Cat. No. 636550)
11) Small Intestine (Cat. No. 636539)
12) Testis (Cat. No. 636533)
13) Thymus (Cat. No. 636549)
14) Thyroid (Cat. No. 636536)
15) Uterus (Cat. No. 636551)

In addition, the following were purchased from COSMO BIO.
1) Esophagus (Cat. No. CB-R1234106-50)
2) Rectum (Cat. No. CB-R1234206-50)
3) Skin (Cat. No. CB-R1234218-50)
4) Uterus Cervix (Cat. No. CB-R1234275-50)
5) Lymph Node (Cat. No. CB-R1234161-10)
6) Ovary (Cat. No. CB-R1234183-50)
7) Stomach (Cat. No. CB-R1234248-50)

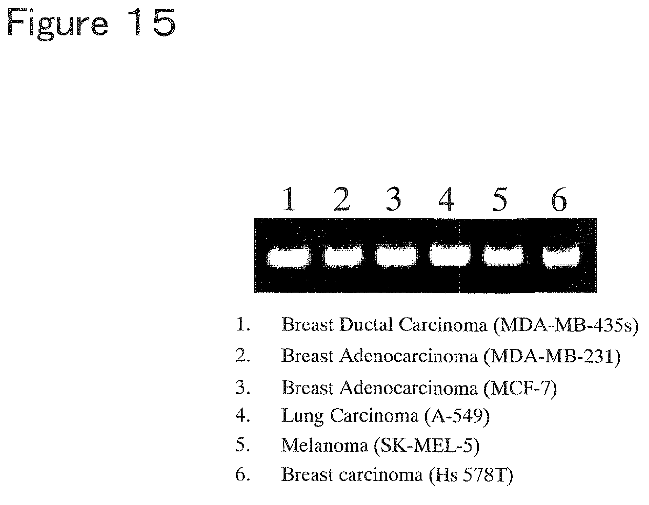

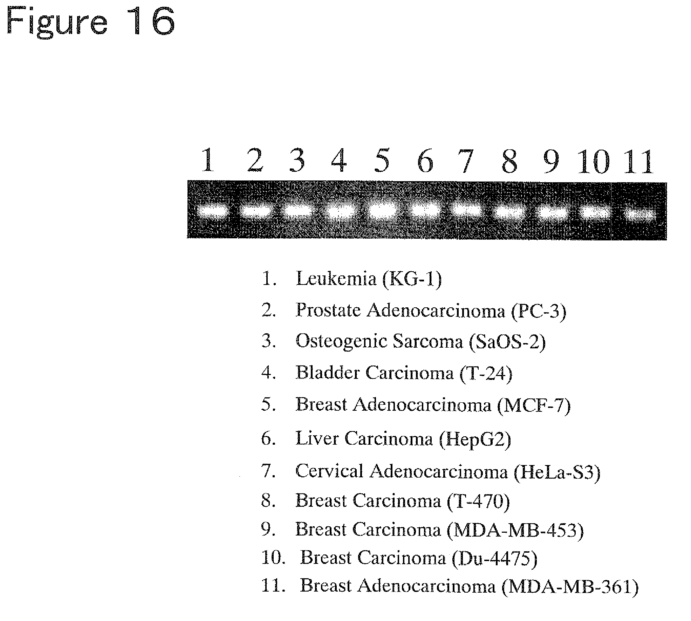

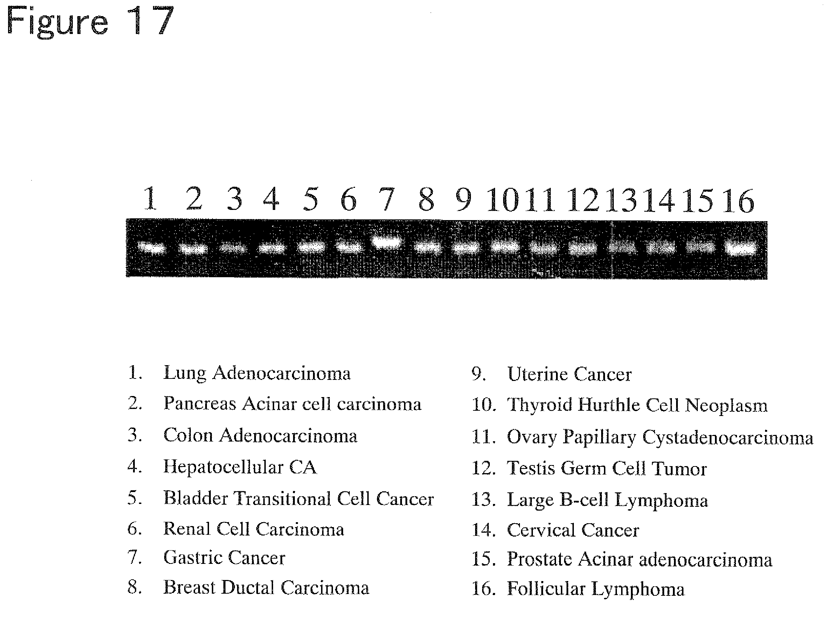

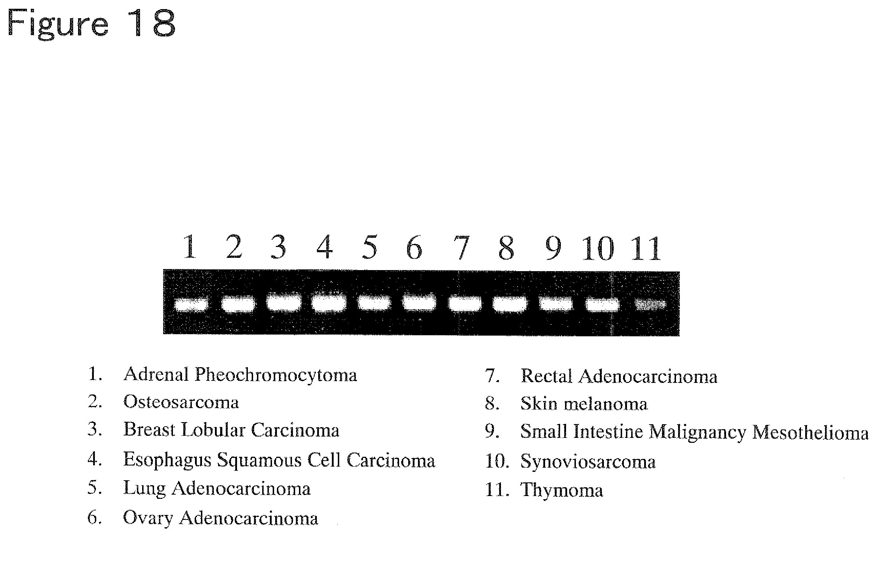

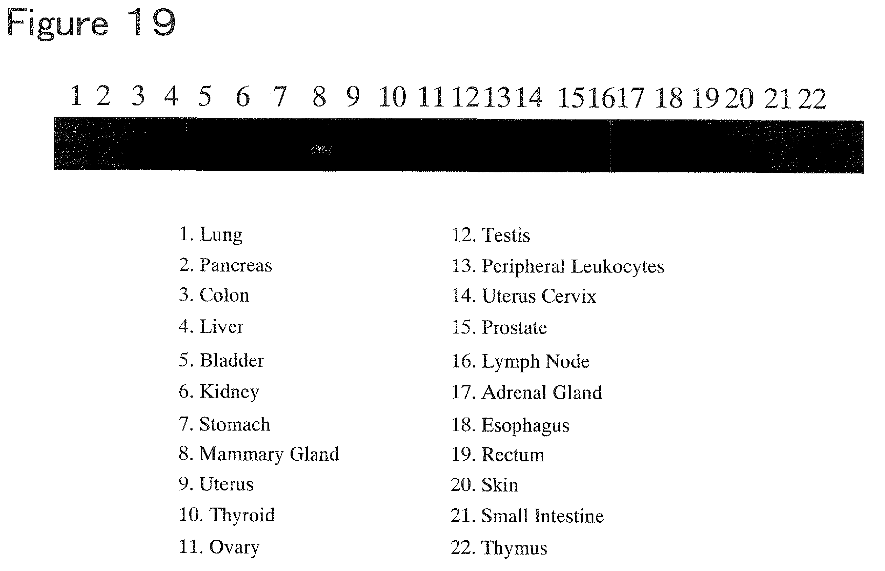

cDNAs were prepared from these total RNAs using a Superscript II First-Strand system (Invitrogen) at 42°C for 50 minutes and then at 70°C for 10 minutes. The cDNAs thus prepared were then subjected to PCR (thermal denaturation at 94°C for 30 seconds, annealing at 56°C for 30 seconds and elongation at 68°C for 30 seconds × 40 cycles) using the primers of SEQ ID NOs: 35 and 36 and KOD plus Taq DNA polymerase (Toyobo Co., Ltd.), followed by electrophoresis on a 3% agarose gel to study the expression of a periostin isoform having the Exon-17 region. As a result, the periostin isoform was confirmed to be expressed in all of the tested cancer types (esophageal cancer, lung cancer, thymic cancer, pancreatic cancer, thyroid cancer, gastric cancer, small intestinal cancer, colon (large bowel) cancer, rectal cancer, liver cancer, bladder cancer, kidney cancer, breast cancer, breast ductal cancer, mammary gland cancer, uterine cancer, uterine cervical cancer, ovarian cancer, testicular cancer, lymphoma, adrenal cancer, prostate cancer, osteogenic sarcoma, malignant melanoma, synovioma, leukemia) (Figures 15, 16, 17 and 18). On the other hand, their corresponding normal tissues were also tested for periostin expression, indicating that mammary glands showed slight expression, which was much weaker than that in the cancer tissues. Moreover, the other normal tissues showed little expression (Figure 19). These results showed that the periostin isoform having the Exon-17 region is expressed specifically in cancer tissues and thus allows cancer diagnosis by measuring its expression level. The results also showed that antibodies against a peptide encoded by the Exon-17 site of periostin may be targeted not only for melanoma or breast cancer, but also for the above cancers.

### INDUSTRIAL APPLICABILITY

The use of antibodies against a peptide region encoded by Exon-17 of periostin enables cancer treatment, more specifically the inhibition of primary tumor growth and metastasis. It is also possible to know the presence or absence of cancers and the degree of their progress by measuring the amount of the periostin isoform in patient samples using the above antibodies or primers specific to the Exon-17 region.

## Claims

1. A pharmaceutical composition for use in a method of treatment of cancer, which comprises an antibody against a peptide consisting of any one amino acid sequence selected from the group consisting of SEQ ID NO: 3, SEQ ID NO: 4 and SEQ ID NO: 21.

2. The pharmaceutical composition for use according to claim 1, wherein the antibody specifically binds to a site consisting of an amino acid sequence of SEQ ID NO: 26.

3. The pharmaceutical composition for use according to claim 1 or 2, wherein the antibody specifically recognizes a site consisting of an amino acid sequence of SEQ ID NO: 34.

4. The pharmaceutical composition for use according to any one of claims 1 to 3, wherein the cancer treatment is to inhibit cancer metastasis.

5. The pharmaceutical composition for use according to any one of claims 1 to 3, wherein the cancer treatment is to inhibit the growth of primary tumor.

6. The pharmaceutical composition for use according to any one of claims 1 to 3, wherein the cancer treatment is to inhibit cancer bone invasion and/or bone destruction caused thereby.

7. The pharmaceutical composition for use according to any one of claims 1 to 6, wherein the cancer is esophageal cancer, lung cancer, thymic cancer, pancreatic cancer, thyroid cancer, gastric cancer, small intestinal cancer, colon (large bowel) cancer, rectal cancer, liver cancer, bladder cancer, kidney cancer, breast cancer, breast ductal cancer, mammary gland cancer, uterine cancer, uterine cervical cancer, ovarian cancer, testicular cancer, lymphoma, adrenal cancer, prostate cancer, osteogenic sarcoma, malignant melanoma, synovioma, or leukemia.

8. The pharmaceutical composition for use according to any one of claims 1 to 6, wherein the cancer is malignant melanoma or breast cancer.

9. An *ex vivo* method for cancer diagnosis, which comprises the step of measuring the amount of a periostin isoform having a peptide region encoded by Exon-17 in a biological sample by using an antibody against a peptide consisting of any one amino acid sequence selected from the group consisting of SEQ ID NO: 3, SEQ ID NO: 4 and SEQ ID NO: 21.

10. A reagent for use in an *in vivo* method of diagnosis of cancer, which comprises an antibody against a peptide consisting of any one amino acid sequence selected from the group consisting of SEQ ID NO: 3, SEQ ID NO: 4 and SEQ ID NO: 21.

11. An *ex vivo* method for cancer diagnosis, which comprises the step of measuring the amount of a periostin isoform having a peptide region encoded by Exon-17 in a biological sample by using PCR primers consisting of a nucleotide having sequence of SEQ ID NO: 35 and a nucleotide having sequence of SEQ ID NO: 36.

12. A reagent for use in an *in vivo* method of diagnosis of cancer, which comprises PCR primers consisting of a nucleotide having sequence of SEQ ID NO: 35 and a nucleotide having sequence of SEQ ID NO: 36.

## Patentansprüche

1. Pharmazeutische Zusammensetzung zur Verwendung in einem Verfahren zur Behandlung von Krebs, die einen Antikörper gegen ein Peptid, bestehend aus irgendeiner Aminosäuresequenz, ausgewählt aus der Gruppe, bestehend aus SEQ ID NO: 3, SEQ ID NO: 4 und SEQ ID NO: 21, umfasst.

2. Pharmazeutische Zusammensetzung zur Verwendung nach Anspruch 1, wobei der Antikörper speziell an eine Stelle, bestehend aus einer Aminosäuresequenz SEQ ID NO: 26, bindet.

3. Pharmazeutische Zusammensetzung zur Verwendung nach Anspruch 1 oder 2, wobei der Antikörper speziell eine Stelle, bestehend aus einer Aminosäuresequenz SEQ ID NO: 34, erkennt.

4. Pharmazeutische Zusammensetzung zur Verwendung nach irgendeinem der Ansprüche 1 bis 3, wobei die Krebsbehandlung zur Hemmung von Krebsmetastasierung bestimmt ist.

5. Pharmazeutische Zusammensetzung zur Verwendung nach irgendeinem der Ansprüche 1 bis 3, wobei die Krebsbehandlung zur Hemmung des Primärtumorwachstums bestimmt ist.

6. Pharmazeutische Zusammensetzung zur Verwendung nach irgendeinem der Ansprüche 1 bis 3, wobei die Krebsbehandlung zur Hemmung von Krebsknocheninvasion und/oder dadurch verursachter Knochenzerstörung bestimmt ist.

7. Pharmazeutische Zusammensetzung zur Verwendung nach irgendeinem der Ansprüche 1 bis 6, wobei der Krebs Speiseröhrenkrebs, Lungenkrebs, Thymuskrebs, Bauchspeicheldrüsenkrebs, Schilddrüsenkrebs, Magenkrebs, Dünndarmkrebs, Darm (Dickdarm)-Krebs, Mastdarmkrebs, Leberkrebs, Blasenkrebs, Nierenkrebs, Brustkrebs, duktaler Brustkrebs, Brustdrüsenkrebs, Gebärmutterkrebs, Gebärmutterhalskrebs, Eierstockkrebs, Hodenkrebs, Lymphom, Nebennierenkrebs, Prostatakrebs, osteogenes Sarkom, malignes Melanom, Synovialsarkom oder Leukämie ist.

8. Pharmazeutische Zusammensetzung zur Verwendung nach irgendeinem der Ansprüche 1 bis 6, wobei der Krebs ein malignes Melanom oder Brustkrebs ist.

9. Ex vivo Verfahren zur Krebsdiagnose, das den Schritt der Messung der Menge einer Periostin-Isoform mit einer durch Exon-17 kodierten Peptidregion in einer biologischen Probe durch Verwendung eines Antikörpers gegen ein Peptid, bestehend aus irgendeiner Aminosäuresequenz, ausgewählt aus der Gruppe bestehend aus SEQ ID NO: 3, SEQ ID NO: 4 und SEQ ID NO: 21, umfasst.

10. Reagenz zur Verwendung in einem in vivo Verfahren zur Krebsdiagnose, das einen Antikörper gegen ein Peptid, bestehend aus irgendeiner Aminosäuresequenz, ausgewählt aus der Gruppe, bestehend aus SEQ ID NO: 3, SEQ ID NO: 4 und SEQ ID NO: 21, umfasst.

11. Ex vivo Verfahren zur Krebsdiagnose, das den Schritt der Messung der Menge einer Periostin-Isoform mit einer durch Exon-17 kodierten Peptidregion in einer biologischen Probe durch Verwendung von PCR-Primern, bestehend aus einem Nukleotid mit der Sequenz SEQ ID NO: 35 und einem Nukleotid mit der Sequenz SEQ ID NO: 36, umfasst.

12. Reagenz zur Verwendung in einem in vivo Verfahren zur Krebsdiagnose, das PCR Primer, bestehend aus einem Nukleotid mit der Sequenz SEQ ID NO: 35 und einem Nukleotid mit der Sequenz SEQ ID NO: 36, umfasst.

## Revendications

1. Composition pharmaceutique destinée à être utilisée dans un procédé de traitement du cancer, qui comprend un anticorps dirigé contre un peptide consistant en une quelconque séquence d'acides aminés sélectionnée dans le groupe consistant en SEQ ID NO: 3, SEQ ID NO: 4 et SEQ ID NO: 21.

2. Composition pharmaceutique destinée à être utilisée selon la revendication 1, dans laquelle l'anticorps se lie spécifiquement à un site consistant en une séquence d'acides aminés de SEQ ID NO: 26.

3. Composition pharmaceutique destinée à être utilisée selon la revendication 1 ou 2, dans laquelle l'anticorps reconnaît spécifiquement un site consistant en une séquence d'acides aminés de SEQ ID NO: 34.

4. Composition pharmaceutique destinée à être utilisée selon l'une quelconque des revendications 1 à 3, dans laquelle le traitement du cancer est l'inhibition des métastases cancéreuses.

5. Composition pharmaceutique destinée à être utilisée selon l'une quelconque des revendications 1 à 3, dans laquelle le traitement du cancer est l'inhibition de la croissance d'une tumeur primaire.

6. Composition pharmaceutique destinée à être utilisée selon l'une quelconque des revendications 1 à 3, dans laquelle le traitement du cancer est l'inhibition de l'invasion osseuse du cancer et/ou de la destruction osseuse provoquée par celle-ci.

7. Composition pharmaceutique destinée à être utilisée selon l'une quelconque des revendications 1 à 6, dans laquelle le cancer est le cancer de l'oesophage, le cancer du poumon, le cancer du thymus, le cancer du pancréas, le cancer de la thyroïde, le cancer de l'estomac, le cancer de l'intestin grêle, le cancer du côlon (gros intestin), le cancer du rectum, le cancer du foie, le cancer de la vessie, le cancer du rein, le cancer du sein, le cancer canalaire du sein, le cancer de la glande mammaire, le cancer de l'utérus, le cancer du col de l'utérus, le cancer de l'ovaire, le cancer du testicule, le lymphome, le cancer de la glande surrénale, le cancer de la prostate, le sarcome ostéogénique, le mélanome malin, le synoviome, ou la leucémie.

8. Composition pharmaceutique destinée à être utilisée selon l'une quelconque des revendications 1 à 6, dans laquelle le cancer est le mélanome malin ou le cancer du sein.

9. Procédé *ex vivo* de diagnostic du cancer, qui comprend l'étape de mesure de la quantité d'une isoforme de la périostine possédant une région peptidique codée par l'exon-17 dans un échantillon biologique en utilisant un anticorps dirigé contre un peptide consistant en une quelconque séquence d'acides aminés sélectionnée dans le groupe consistant en SEQ ID NO: 3, SEQ ID NO: 4 et SEQ ID NO: 21.

10. Réactif destiné à être utilisé dans un procédé *in vivo* de diagnostic du cancer, qui comprend un anticorps dirigé contre un peptide consistant en une quelconque séquence d'acides aminés sélectionnée dans le groupe consistant en SEQ ID NO: 3, SEQ ID NO: 4 et SEQ ID NO: 21.

11. Procédé *ex vivo* de diagnostic du cancer, qui comprend l'étape de mesure de la quantité d'une isoforme de la périostine possédant une région peptidique codée par l'exon-17 dans un échantillon biologique en utilisant des amorces de PCR consistant en un nucléotide ayant une séquence de SEQ ID NO: 35 et un nucléotide ayant une séquence de SEQ ID NO: 36.

12. Réactif destiné à être utilisé dans un procédé *in vivo* de diagnostic du cancer, qui comprend des amorces de PCR consistant en un nucléotide ayant une séquence de SEQ ID NO: 35 et un nucléotide ayant une séquence de SEQ ID NO: 36.
